# EUROPEAN PATENT SPECIFICATION

(11) **EP 0 436 334 B1**
(45) Date of publication and mention of the grant of the patent: **07.12.1994**
(21) Application number: 90313680.2
(22) Date of filing: 14.12.1990
(51) Int. Cl.: C07D 207/14, C07D 211/56, C07D 223/12, C07D 221/20, A61K 31/40, A61K 31/445, A61K 31/55

(54) **3-Aminopiperidine derivatives and related nitrogen containing heterocycles**
3-Aminopiperidinderivate und verwandte Nitrogen-enthaltende Heterozyklen
Dérivés de 3-Aminopipéridine et N-hétérocycles apparentés

(30) Priority: 04.01.1990 WO PCT/US90/00116
(43) Date of publication of application: 10.07.1991
(60) Divisional application: 93201034.1
(73) Proprietor: PFIZER INC., New York, N.Y. 10017 (US)
(72) Inventor: Desai, Manoj C., Mystic, State of Connecticut (US); Rosen, Terry J., East Lyme, State of Connecticut (US)
(74) Representative: Moore, James William, Dr.

(56) References cited:
- WO-A-90/05729
- US-A- 3 560 510
- US-A- 3 992 389

## Description

The present invention relates to novel 3-amino-piperidine derivatives and related compounds, pharmaceutical compositions comprising such compounds and the use of such compounds in the treatment and prevention of inflammatory and central nervous system disorders, as well as several other disorders. The pharmaceutically active compounds of this invention are substance P antagonists. This invention also relates to novel intermediates used in the synthesis of such substance P antagonists.

Substance P is a naturally occurring undecapeptide belonging to the tachykinin family of peptides, the latter being named because of their prompt stimulatory action on smooth muscle tissue. More specifically, substance P is a pharmacologically active neuropeptide that is produced in mammals (having originally been isolated from gut) and possesses a characteristic amino acid sequence that is illustrated by D. F. Veber et al. in U.S. Patent No. 4,680,283. The wide involvement of substance P and other tachykinins in the pathophysiology of numerous diseases has been amply demonstrated in the art. For instance, substance P has recently been shown to be involved in the transmission of pain or migraine (see B.E.B. Sandberg et al., Journal of Medicinal Chemistry, 25, 1009 (1982)), as well as in central nervous system disorders such as anxiety and schizophrenia, in respiratory and inflammatory diseases such as asthma and rheumatoid arthritis, respectively, in rheumatic diseases such as fibrositis, and in gastrointestinal disorders and diseases of the GI tract such as ulcerative colitis and Crohn's disease, etc. (see D. Regoli in "Trends in Cluster Headache," edited by F. Sicuteri et al., Elsevier Scientific Publishers, Amsterdam, pp. 85-95 (1987)).

In the recent past, some attempts have been made to provide antagonists for substance P and other tachykinin peptides in order to more effectively treat the various disorders and diseases listed above. The few such antagonists thus far described are generally peptide-like in nature and are therefore too labile from a metabolic point of view to serve as practical therapeutic agents in the treatment of disease. The non-peptidic antagonists of the present invention, on the other hand, do not possess this drawback, being far more stable from a metabolic point of view than the agents referred to above.

Certain quinuclidine substance P antagonists are disclosed in WO 90/05729 while US 3560510 discloses certain 3-benzhydrylquinuclidines as diuretic agents. US 3992389 describes various heterocyclic compounds including 1-N-substituted-benzamidopiperidines; the compounds exhibit various pharmacological effects including hypotensive, depressant and antihistamine activity.

The present invention relates to compounds of the formula 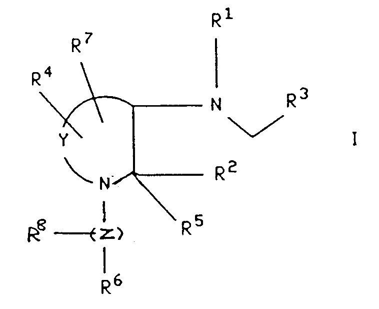
wherein Y is (CH₂)ₙ wherein n is an integer from 1 to 4, and wherein any one of the carbon-carbon single bonds in said (CH₂)ₙ may optionally be replaced by a carbon-carbon double bond, and wherein any one of the carbon atoms of said (CH₂)ₙ may optionally be substituted with R⁴, and wherein any one of the carbon atoms of said (CH₂)ₙ may optionally be substituted with R⁷;
Z is (CH₂)ₘ wherein m is an integer from 0 to 6, and wherein any one of the carbon-carbon single bonds of (CH₂)ₘ may optionally be replaced by a carbon-carbon double bond or a carbon-carbon triple bond, and any one of the carbon atoms of said (CH₂)ₘ may optionally be substituted with R⁸;
R¹ is hydrogen or (C₁-C₈) alkyl optionally substituted with hydroxy, (C₁-C₄)alkoxy or fluoro;
R² is a radical selected from hydrogen, (C₁-C₆) straight or branched alkyl, (C₃-C₇) cycloalkyl wherein one of the CH₂ groups in said cycloalkyl may optionally be replaced by NH, oxygen or sulfur; aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; phenyl-(C₂-C₆)- alkyl, benzhydryl and benzyl, wherein each of said aryl and heteroaryl groups and the phenyl moieties of said benzyl, phenyl-(C₂-C₆)-alkyl and benzhydryl may optionally be substituted with one or more substituents independently selected from halo, nitro, (C₁-C₆) alkyl, (C₁-C₆) alkoxy, trifluoromethyl, amino, (C₁-C₆)-alkylamino, 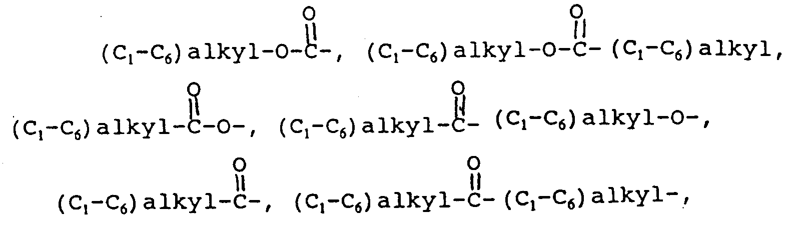
di-(C₁-C₆)alkylamino, 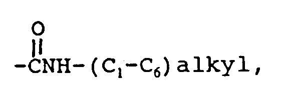 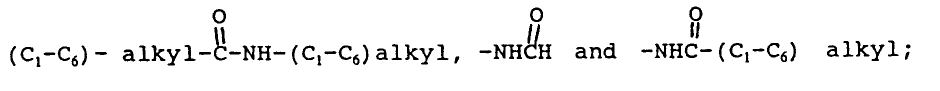
and wherein one of the phenyl moieties of said benzhydryl may optionally be replaced by naphthyl, thienyl, furyl or pyridyl;
R⁵ is hydrogen, phenyl or (C₁-C₆)alkyl;
or R² and R⁵, together with the carbon to which they are attached, form a saturated ring having from 3 to 7 carbon atoms wherein one of the CH₂ groups in said ring may optionally be replaced by oxygen, NH or sulfur;
R³ is aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; and cycloalkyl having 3 to 7 carbon atoms wherein one of the (CH₂) groups in said cycloalkyl may optionally be replaced by NH, oxygen or sulfur; wherein each of said aryl and heteroaryl groups may optionally be substituted with one or more substituents, and said (C₃-C₇) cycloalkyl may optionally be substituted with one or two substituents, each of said substituents being independently selected from halo, nitro, (C₁-C₆) alkyl, (C₁-C₆) alkoxy, trifluoromethyl,
amino, (C₁-C₆) alkylamino, 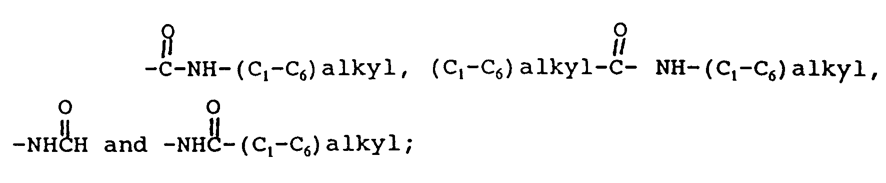
and
R⁴ and R⁷ are each independently selected from hydroxy, hydrogen halo, amino, oxo, cyano, methylene, hydroxymethyl, halomethyl, (C₁-C₆)alkylamino, di-(C₁-C₆)alkylamino, (C₁-C₆)alkoxy, 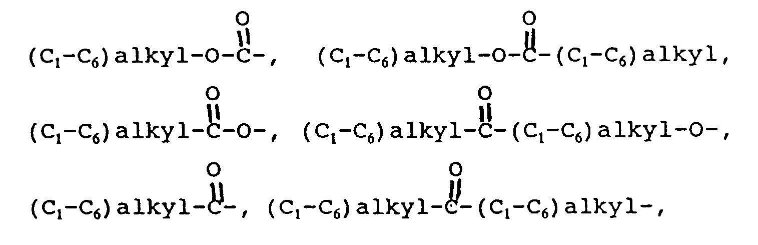
and the radicals set forth in the definition of R²,
R⁶ is 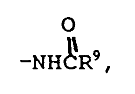
-NHCH₂R⁹, SO₂R⁹ or one of the radicals set forth in any of the definitions of R², R⁴ and R⁷;
R⁸ is oximino (=NOH) or one of the radicals set forth in any of the definitions of R², R⁴ and R⁷;
R⁹ is (C₁-C₆)alkyl, hydrogen, phenyl or phenyl (C₁-C₆)alkyl;
with the proviso that (a) when m is 0, R⁸ is absent, (b) when R⁴, R⁶, R⁷ or R⁸ is as defined in R², it cannot form, together with the carbon to which it is attached, a ring with R⁵, (c) when R⁴ and R⁷ are attached to the same carbon atom, then either each of R⁴ and R⁷ is independently selected from hydrogen, fluoro and (C₁-C₆) alkyl, or R⁴ and R⁷, together with the carbon to which they are attached, form a (C₃-C₆) saturated carbocyclic ring that forms a spiro compound with the nitrogen-containing ring to which they are attached, and (d) one of R² and R⁵ must be other than hydrogen or unsubstituted (C₁-C₆)alkyl.

The present invention also relates to the pharmaceutically acceptable acid addition salts of compounds of the formula I. The acids which are used to prepare the pharmaceutically acceptable acid addition salts of the aforementioned base compounds of this invention are those which form non-toxic acid addition salts, i.e., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [i.e., 1,1'-methylene-bis-(2-hydroxy-3- naphthoate)]salts.

The present invention also relates to compounds of the formula 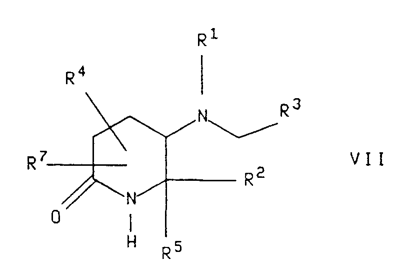
wherein R¹, R², R³, R⁴, R⁵ and R⁷ are as defined for compounds of the formula I. The compounds of the formula VII are novel intermediates used in the synthesis of compounds of the formula I.

The present invention also relates to the compound 3-amino-2-phenylpiperidine.

The term "halo", as used herein, unless otherwise indicated, includes chloro, fluoro, bromo and iodo.

The term "alkyl", as used herein, unless otherwise indicated, includes saturated monovalent hydrocarbon radicals having straight, branched or cyclic moieties or combinations thereof.

The term "one or more substituents," as used herein, includes from one to the maximum number of substituents possible based on the number of available bonding sites.

Preferred compounds of the formula I are those wherein R¹, R⁴, R⁵ R⁶ and R⁷ are hydrogen, R² is phenyl, R³ is 2-methoxyphenyl, wherein the phenyl moiety may optionally be substituted with chlorine, fluorine, methyl, (C₁-C₆)alkoxy or trifluoromethane, m is 0 and n is 3 or 4.

Specific preferred compounds of the formula I are:
cis-3-(2-chlorobenzylamino)-2-phenylpiperidine;
cis-3-(2-trifluoromethylbenzylamino)-2-phenylpiperidine;
cis-3-(2-methoxybenzylamino)-2-(2-fluorophenyl)piperidine;
cis-3-(2-methoxybenzylamino)-2-(2-chlorophenyl)piperidine;
cis-3-(2-methoxybenzylamino)-2-(2-methylphenyl)piperidine;
cis-3-(2-methoxybenzylamino)-2-(3-methoxyphenyl)piperidine;
cis-3-(2-methoxybenzylamino)-2-(3-fluorophenyl)piperidine;
cis-3-(2-methoxybenzylamino)-2-(3-chlorophenyl)piperidine;
cis-3-(2-methoxybenzylamino)-2-phenylpiperidine;
cis-3-(2-methoxybenzylamino)-2-(3-methylphenyl)piperidine;
cis-3-(2-methoxybenzylamino)-2-(4-fluorophenyl)piperidine;
cis-3-(2-methoxybenzylamino)-2-(3-thienyl)- piperidine;
cis-3-(2-methoxybenzylamino)-2-phenylazacycloheptane;
3-(2-methoxybenzylamino)-4-methyl-2-phenyl- piperidine;
3-(2-methoxybenzylamino)-5-methyl-2-phenyl- piperidine;
3-(2-methoxybenzylamino)-6-methyl-2-phenyl- piperidine;
(2S,3S)-3-(2-methoxybenzylamino)-2-phenylpiperidine;
(2S,3S)-1-(5-carboethoxypent-1-yl)-3-(2-methoxybenzylamino)-2-phenylpiperidine;
(2S,3S)-1-(6-hydroxy-hex-1-yl)-3-(2-methoxybenzylamino)-2-phenylpiperidine;
(2S,3S)-1-(4-hydroxy-4-phenylbut-1-yl)-3-(2-methoxybenzylamino)-2-phenylpiperidine;
(2S,3S)-1-(4-oxo-4-phenylbut-1-yl)-3-(2-methoxybenzylamino)-2-phenylpiperidine;
(2S,3S)-1-(5,6-dihydroxyhex-1-yl)-3-(2-methoxybenzylamino)-2-phenylpiperidine;
cis-3-(5-fluoro-2-methoxybenzylamino)-2-phenylpiperidine;
(2S,3S)-1-[4-(4-fluorophenyl)-4-oxobut-1-yl]-3-(2-methoxybenzylamino)-2-phenylpiperidine;
(2S,3S)-1-[4-[4-fluorophenyl)-4-hydroxybut-1-yl]-3-(2-methoxybenzylamino)-2-phenylpiperidine;
cis-3-(2-methoxy-5-methylbenzylamino)-2-phenylpiperidine;
(2S,3S)-1-(4-benzamidobut-1-yl)-3-(2-methoxybenzylamino)-2-phenylpiperidine;
cis-3-(2-methoxynaphth-1-ylmethylamino)-2-phenylpiperidine;
(2S,3S)-3-(2-methoxybenzylamino)-1-(5-N-methylcarboxamidopent-1-yl)-2-phenylpiperidine;
(2S,3S)-1-(4-cyanobut-1-yl)-3-(2-methoxybenzylamino)-2-phenylpiperidine;
(2S,3S)-1-[4-(2-naphthamido)but-1-yl]-3-(2-methoxybenzylamino)-2-phenylpiperidine;
(2S,3S)-1-(5-benzamidopent-1-yl)-3-(2-methoxybenzylamino)-2-phenylpiperidine;
(2S,3S)-1-(5-aminopent-1-yl)-3-(2-methoxybenzylamino)-2-phenylpiperidine;
(2S,3S)-3-(5-chloro-2-methoxybenzylamino)-2-phenylpiperidine;
(2S,3S)-3-(2,5-dimethoxybenzylamino)-2-phenylpiperidine;
cis-3-(3,5-difluoro-2-methoxybenzylamino)-2-phenylpiperidine;
cis-3-(4,5-difluoro-2-methoxybenzylamino)-2-phenylpiperidine;
cis-3-(2,5-dimethoxybenzylamino)-1-[4-(4-fluorophenyl)-4-oxobut-1-yl]-2-phenylpiperidine;
cis-3-(5-chloro-2-methoxybenzylamino)-1-(5,6-dihydroxyhex-1-yl)-2-phenylpiperidine;
cis-1-(5,6-dihydroxyhex-1-yl)-3-(2,5-dimethoxybenzylamino)-2-phenylpiperidine;
cis-2-phenyl-3-[2-(prop-2-yloxy)benzylamino]piperidine;
cis-3-(2,5-dimethoxybenzyl)amino-2-(3-methoxyphenyl)piperidine hydrochloride;
cis-3-(5-chloro-2-methoxybenzyl)amino-2-(3-methoxyphenyl)piperidine dihydrochloride;
cis-3-(5-chloro-2-methoxybenzyl)amino-2-(3-chlorophenyl)piperidine dihydrochloride;
3-(2-methoxybenzylamino)-2,4-diphenylpiperidine; and
cis-3-(2-methoxybenzylamino)-2-phenylpyrrolidine.

Other compounds of the formula I are:
3-(2-methoxybenzylamino)-5-methylene-2-phenylpiperidine;
5-hydroxy-3-(2-methoxybenzylamino)-2-phenylpiperidine;
4-fluoro-3-(2-methoxybenzylamino)-2-phenyl- piperidine;
5-hydroxymethyl-3-(2-methoxybenzylamino)-2-phenylpiperidine;
5-fluoromethyl-3-(2-methoxybenzylamino)-2-phenylpiperidine;
3-(2-methoxybenzylamino)-2-phenyl-1,2,3,5-tetrahydropyridine;
6-aza-4-(2-methoxybenzylamino)-5-phenyl-spiro-[2,5]-octane;
3β-(2-methoxybenzylamino)-5α-methyl-2β-phenylpiperidine;
5,5-dimethyl-3-(2-methoxybenzylamino)-2-phenylpiperidine;
5,6-dimethyl-3-(2-methoxybenzylamino)-2-phenylpiperidine;
2,5-diphenyl-3-(2-methoxybenzylamino)-2-phenylpiperidine;
4-hydroxy-3-(2-methoxybenzylamino)-4-methyl-2-phenylpiperidine;
2,6-diphenyl-3-(2-methoxybenzylamino)piperidine;
1-(5-cyclohexylpent-1-yl)-3-(2-methoxybenzylamino)-2-phenylpiperidine;
2-benzhydryl-3-(2-methoxybenzylamino)piperidine;
cis-3-(5-fluoro-2-methoxybenzyl)amino-2-(3-fluorophenyl)piperidine;
cis-3-(2,5-dimethoxybenzyl)amino-2-(3-fluorophenyl)piperidine;
cis-3-(2,4-dimethoxylbenzyl)amino-2-(3-fluorophenyl)piperidine;
cis-3-(2-methoxy-5-methylbenzyl)amino-2-(3-fluorophenyl)piperidine;
cis-3-(5-chloro-2-methoxybenzyl)amino-2-(2-fluorophenyl)piperidine;
cis-3-(2,5-dimethoxybenzyl)amino-2-(2-fluorophenyl)piperidine;
cis-3-(5-fluoro-2-methoxybenzyl)amino)-2-(2-fluorophenyl)piperidine;
cis-2-(2-fluorophenyl)-3-(2-methoxy-5-methylbenzyl)aminopiperidine;
cis-3-(5-chloro-2-methoxybenzyl)amino-2-(3-methoxyphenyl)piperidine;
cis-3-(5-fluoro-2-methoxybenzyl)amino-2-(3-methoxyphenyl)piperidine;
cis-3-(2,5-dimethoxybenzyl)amino-2-(3-methoxyphenyl)piperidine;
cis-2-(3-methoxyphenyl)-3-(2-methoxy-5-methylphenyl)piperidine;
2-benzhydryl-3-(2-methoxybenzylamino)pyrrolidine;
cis-3-(5-chloro-2-methoxybenzyl)amino-2-(3-chlorophenyl)piperidine;
cis-3-(5-fluoro-2-methoxybenzyl)amino-2-(3-chlorophenyl)piperidine;
cis-2-(3-chlorophenyl)-3-(2,5-dimethoxybenzyl amino)piperidine;
cis-2-(3-chlorophenyl)-3-(2-methoxy-5-methylbenzyl amino)piperidine;
cis-3-(2,6-dichloro-4-methoxybenzylamino)-2-phenylpiperidine;
cis-3-(2,4-dichloro-6-methoxybenzylamino)-2-phenylpiperidine;
cis-3-(2,4-dimethoxybenzylamino)-2-phenylpiperidine;
cis-3-(2,3-dimethoxybenzylamino)-2-phenylpiperidine;
cis-3-(2-methoxy-3-methylbenzylamino)-2-phenylpiperidine;
cis-3-[2-(tert-butoxy)benzylamino]-2-phenylpiperidine;
cis-3-(2-cyclopentyloxybenzylamino)-2-phenylpiperidine;
cis-3-[3-(tert-butyl)-2-methoxybenzylamino)-2-phenylpiperidine;
cis-1-(2-cyanoeth-1-yl)-3-(2-methoxybenzylamino)-2-phenylpiperidine;
cis-1-(2-aminoeth-1-yl)-3-(2-methoxybenzylamino)-2-phenylpiperidine;
cis-1-(2-benzamidoeth-1-yl)-3-(2-methoxybenzylamino)-2-phenylpiperidine;
cis-1-[4-(tert-butyramido)but-1-yl)-3-(2-methoxybenzylamino)-2-phenylpiperidine;
cis-1-(4-N-methylcarboxamidobut-1-yl)-3-(2-methoxybenzylamino)-2-phenylpiperidine;
cis-1-(3,5-dihydroxypent-1-yl)-3-(2-methoxybenzylamino)-2-phenylpiperidine;
2,2-diphenyl-3-(2-methoxybenzylamino)-2-phenylpiperidine;
3-(2-methoxybenzylamino)-2-methyl-3-phenylpiperidine;
cis-3-(2,5-dimethoxybenzylamino)-2-diphenylmethylpiperidine;
cis-3-(5-chloro-2-methoxybenzylamino)-2-diphenylmethylpiperidine;
cis-3-(2-methoxybenzylamino)-2-(pyrid-3-yl)piperidine; and
3-(2-methoxybenzylamino)-4-phenylpiperidine.

The present invention also relates to a pharmaceutical composition comprising a compound of the formula I, or a pharmaceutically acceptable salt thereof, together with a pharmaceutically acceptable diluent or carrier.

The present invention also relates to the use of a compound of formula I for treating or preventing a condition selected from the group consisting of inflammatory diseases (e.g., arthritis, psoriasis, asthma and inflammatory bowel disease), anxiety, depression or dysthymic disorders, colitis, psychosis, pain, allergies such as eczema and rhinitis, chronic obstructive airways disease, hypersensitivity disorders such as poison ivy, vasospastic diseases such as angina, migraine and Reynaud's disease, fibrosing and collagen diseases such as scleroderma and eosinophilic fascioliasis, reflex sympathetic dystrophy such as shoulder/hand syndrome, addiction disorders such as alcoholism, stress related somatic disorders, peripheral neuropathy, neuralgia, neuropathological disorders such as Alzheimer's disease, AIDS related dementia, diabetic neuropathy and multiple sclerosis, disorders related to immune enhancement or suppression such as systemic lupus erythematosus, and rheumatic diseases such as fibrositis in a mammal, including a human.

The compounds of the present invention are also of value in a method of antagonizing the effects of substance P in a mammal, including a human, which comprises administering to said mammal a substance P antagonizing amount of compound of the formula I, or a pharmaceutically acceptable salt thereof.

The compounds of the present invention are also of value in a method of treating or preventing a disorder in a mammal, including a human, resulting from an excess of substance P, which comprises administering to said mammal a substance P antagonizing amount of a compound of the formula I, or a pharmaceutically acceptable salt thereof.

The compounds of the present invention are also of value in a method of treating or preventing a condition selected from the group consisting of inflammatory diseases (e.g., arthritis, psoriasis, asthma and inflammatory bowel disease), anxiety, depression or dysthymic disorders, colitis, psychosis, pain, allergies such as eczema and rhinitis, chronic obstructive airways disease, hypersensitivity disorders such as poison ivy, vasospastic diseases such as angina, migraine and Reynaud's disease, fibrosing and collagen diseases such as scleroderma and eosinophilic fascioliasis, reflex sympathetic dystrophy such as shoulder/hand syndrome, addiction disorders such as alcoholism, stress related somatic disorders, peripheral neuropathy, neuralgia, neuropathological disorders such as Alzheimer's disease, AIDS related dementia, diabetic neuropathy and multiple sclerosis, disorders related to immune enhancement or suppression such as systemic lupus erythematosus, and rheumatic diseases such as fibrositis in a mammal, including a human, which comprises administering to said mammal an amount of a compound of the formula I, or a pharmaceutically acceptable salt thereof, effective in antagonizing the effect of substance P at its receptor site.

The compounds of the present invention are also of value in a method of treating or preventing a disorder in mammal, including a human, the treatment or prevention of which is effected or facilitated by a decrease in substance P mediated neurotransmission which comprises administering to said mammal an amount of a compound of the formula I, or a pharmaceutically acceptable salt thereof, effective in antagonizing the effect of substance P at its receptor site.

The compounds of the present invention are also of value in a method of treating or preventing a disorder in mammal, including a human, the treatment or prevention of which is effected or facilitated by a decrease in substance P mediated neurotransmission which comprises administering to said mammal an amount of a compound of the formula I, or a pharmaceutically acceptable salt thereof, effective in treating or preventing such disorder.

According to further preferred aspects, the invention includes compounds of the formula: 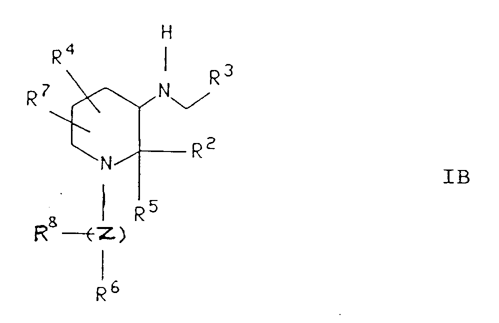
wherein R² to R⁸ and Z are as previously defined; compounds of the formula: 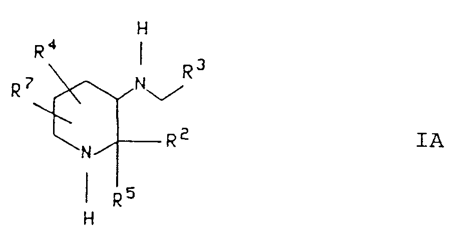
wherein R² to R⁷ are as previously defined; compounds of the formula: 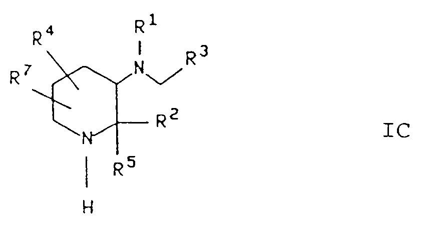
wherein R¹ to R⁵ and R⁷ are as previously defined; and the 2S, 3S enantiomers of compounds of the formula I of the formula: 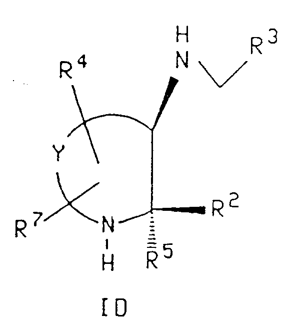
wherein R² to R⁵ and R⁷ are as previously defined.

The compounds of the formula I have chiral centers and therefore exist in different enantiomeric forms. This invention relates to all optical isomers and all stereoisomers of compounds of the formula I, and mixtures thereof.

Formulae I and VII above include compounds identical to those depicted but for the fact that one or more hydrogen or carbon atoms are replaced by radioactive isotopes thereof. Such radiolabelled compounds are useful as research and diagnostic tools in metabolism pharmokinetic studies and in binding assays. Specific applications in research include radioligand binding assays, autoradiography studies and in vivo binding studies, while specific applications in the diagnostic area include studies of the substance P receptor in the human brain in in vivo binding in the relevant tissues for inflammation, e.g. immune-type cells or cells that are directly involved in inflammatory bowel disorders and the like. Included among the radiolabelled forms of compounds of the formulae I and VII are the tritium and C¹⁴ isotopes thereof.

The compounds of the formula I may be prepared as described in the following reaction schemes and discussion. Each of the formulae designated IA, IB, IC, and ID represents a different group of compounds having the general formula I. Unless otherwise indicated, R¹, R², R³, R⁴, R⁵, R⁶, R⁷, R⁸, Y,Z,n and m in the reaction schemes and discussion that follow are defined as above.

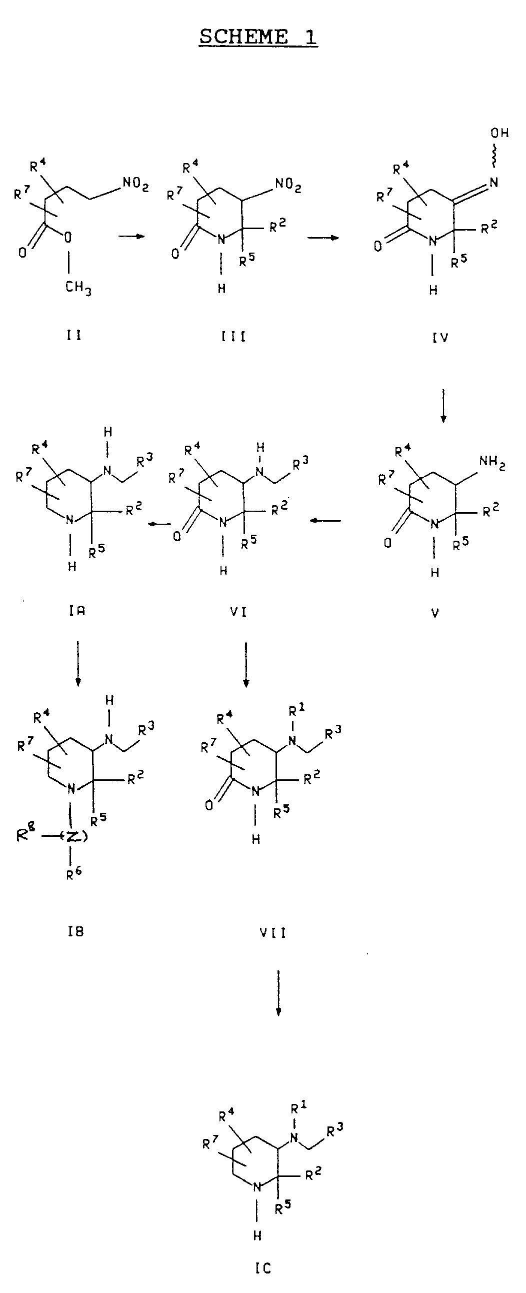

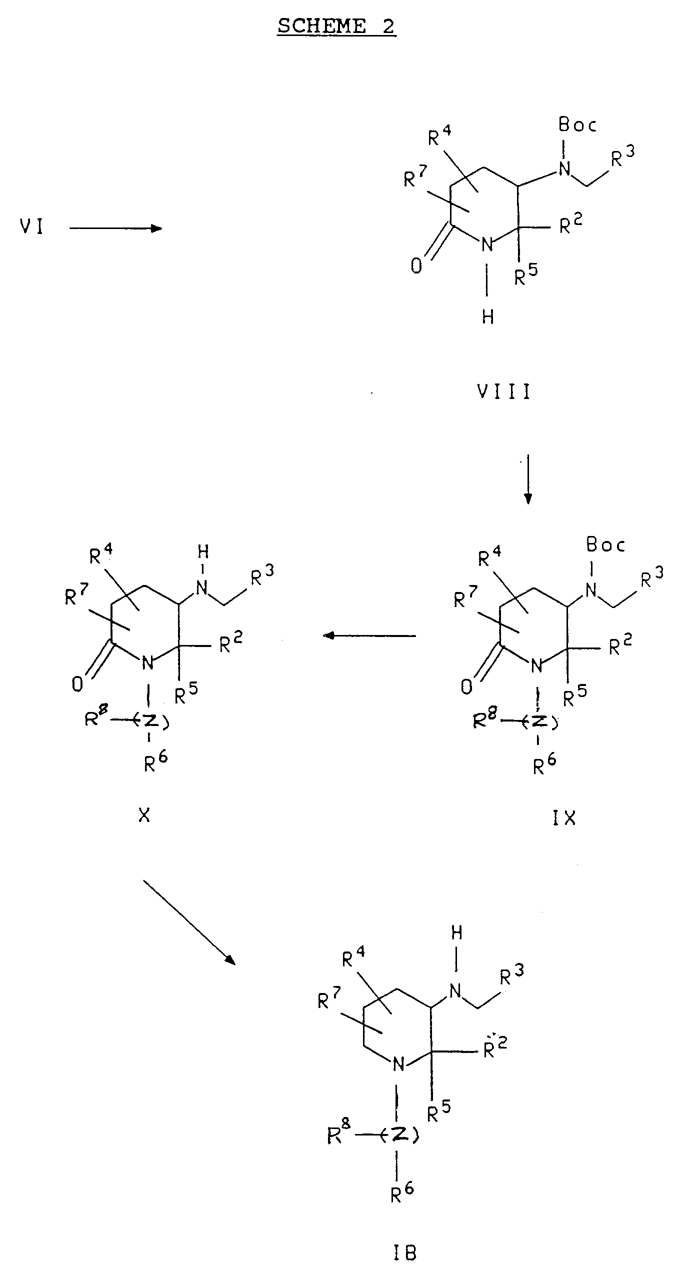

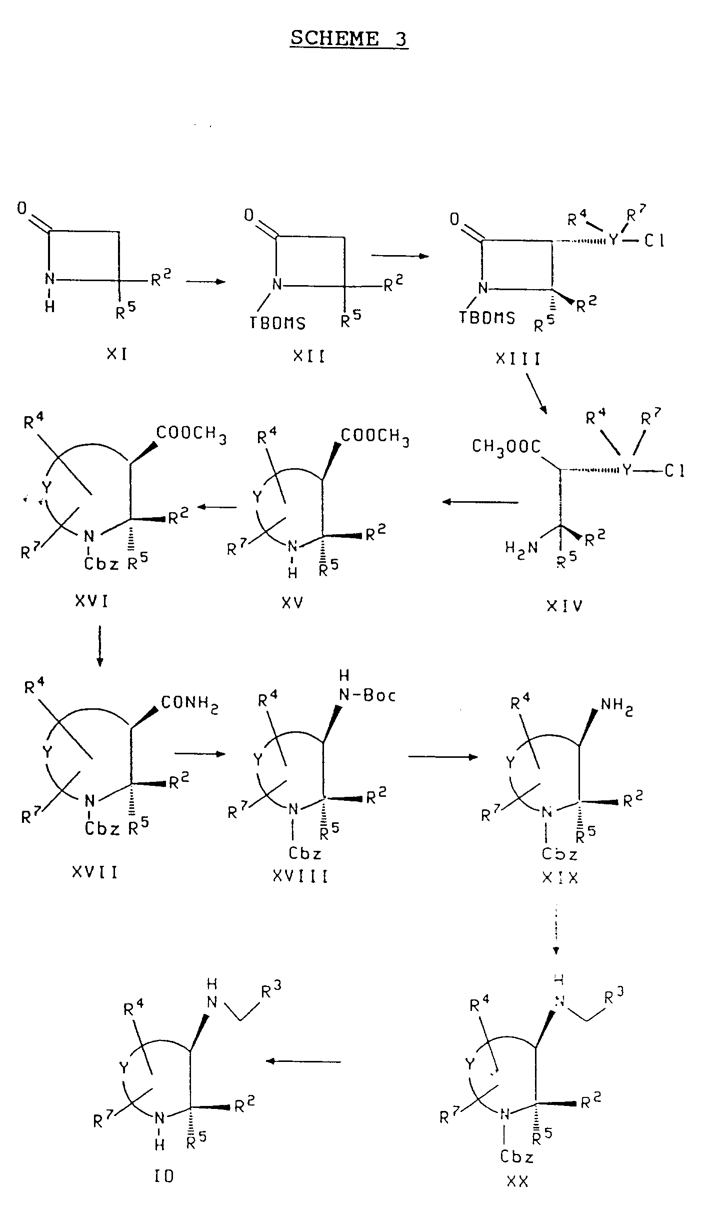

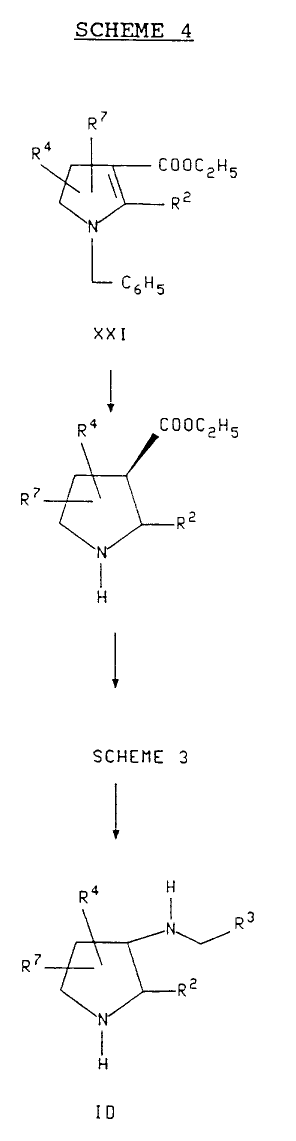

Scheme 1 illustrates the preparation of compounds of the formulae IA, IB and IC. Formula IA represents compounds of the formula I wherein each of R¹ and R⁶ is hydrogen, m is 0 and n is 3, with the proviso that R² is not benzhydryl and neither R⁴ nor R⁷ is attached to the "6" position of the piperidine ring. Formula IB represents compounds of the formula I wherein R¹ is hydrogen and n is 3, with the proviso that R² is not benzhydryl and neither R⁴ nor R⁷ is attached to the "6" position of the piperidine ring. Formula IC represents compounds of the formula I wherein R⁶ is hydrogen, m is 0 and n is 3, with the proviso that R² is not benzhydryl and neither R⁴ nor R⁷ is attached to the "6" position of the piperidine ring.

Referring to scheme 1, a compound of the formula II is reacted with a compound of the formula 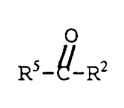
in the presence of ammonium acetate, in a polar solvent such as ethanol, acetic acid or dimethyl sulfoxide. Ethanol is the preferred solvent. Temperatures from about room temperature to about 150°C are suitable, with the reflux temperature of the solvent being preferred. This reaction yields, by intramolecular condensation, a compound of the formula III (Von M. Muhlstadt and B. Schulze, J. Prak. Chem, 317, 919 (1975)).

The condensation product of formula III is then converted, via a Nef reaction, to an oxime of the formula IV. This reaction may be carried out using reagents such as agueous Ti(III) chloride, potassium permanganate, pyridine/hexamethylphosphoramide complex of molybdenum pentoxide, tributylphosphinediphenyl disulphide or ozone in the presence of a base. Suitable temperatures range from about -100°C to about 0°C. Preferably, the reaction is performed by bubbling ozone through the reaction mixture in the presence of potassium t-butoxide at about -78°C, and then quenching the reaction mixture with hydroxylamine hydrochloride at ambient temperature.

The oxime of formula IV is then reduced to yield both the cis and trans isomers of a compound of the formula V. Suitable reducing agents include Raney nickel/hydrogen, 10% palladium on charcoal/hydrogen, and aluminum amalgam. Preferably, the reduction is carried out using Raney nickel in ethanol under a hydrogen gas pressure of about 3 atm and at a temperature of about 25°C. Temperatures from about 10°C to about 60°C and pressures from about 1 to about 10 atmospheres are also suitable.

Reductive amination of the mixture of cis and trans isomers of the compound of the formula V from the above step with sodium cyanoborohydride or sodium triacetoxyborohydride and a compound of the formula R³CHO yields a mixture of the cis and trans isomers of a compound of the formula VI. This reaction is typically carried out in a polar solvent such as acetic acid or a lower alkanol, at a temperature from about 0°C to about 50°C. Methanol is the preferred solvent and about 25°C is the preferred temperature. It is also preferable that the pH of the reaction mixture be about 4 to about 5. The cis and trans isomers of the compound of the formula VI so formed can be easily separated by using silica-gel flash chromatography, eluting with 3% methanol in methylene chloride.

Reduction of either the cis or trans isomer of the compound of formula VI, or a mixture thereof, yields a compound of the formula IA having the same stereochemistry. Suitable reducing agents include borane dimethylsulfide in tetrahydrofuran ("THF"), lithium aluminum hydride, borane in THF and sodium borohydride-titanium (IV) chloride. Best results are obtained by using borane dimethylsulfide in THF. The reaction may be carried out at temperatures from about room temperature to about 150°C, and is preferably carried out at the reflux temperature of the solvent.

The compound of formula IA so formed may be converted to a compound of the formula IB having the same stereochemistry, as illustrated in scheme 1, by reacting it with a compound of the formula R⁶-( Z ) -X, wherein X is halo, Z is (CH₂)ₘ and wherein one of the carbon-carbon single bond of said (CH₂)ₘ may optionally be replaced by a carbon-carbon double bond, and wherein one of the carbons of said (CH₂)ₘ may optionally be substituted with R⁸. This reaction is typically carried out in the presence of a base such as triethylamine or potassium t-butoxide, in a polar solvent such as methylene chloride or dichloroethane, and at a temperature from about room temperature to about 150°C. Preferably, the reaction is carried out at the reflux temperature in methylene chloride in the presence of triethylamine.

Compounds of the formula IC may be prepared as illustrated in scheme 1 and described below. A compound of the formula VI is reacted with a compound of the formula R¹X, wherein X is halo, to yield a compound of the formula VII having the same stereochemistry (e.g. cis, trans or a mixture thereof). This reaction is typically carried out in the presence of a base such as triethylamine or potassium t-butoxide in a polar solvent such as methylene chloride or dichloroethane, at a temperature from about room temperature to about 150°C. Preferably, the reaction is carried out at about the reflux temperature in methylene choride in the presence of triethylamine.

Reduction of the compound of formula VII so formed yields a compound of the formula IC having the same stereochemistry. Examples of suitable reducing agents are lithium aluminum-hydride, borane dimethylsulfide in THF, borane in THF and sodium borohydride-titanium (IV) chloride. Best results are obtained using borane dimethylsulfide in THF. The reaction may be carried out at temperatures from about room temperature to about 150°C, and is preferably carried out at the reflux temperature of the solvent.

Scheme 2 illustrates an alternate method of preparing compounds of the formula IB. The starting material for this method is a compound of the formula VI, which is illustrated in scheme 1. In the first step of this method, the basic nitrogen of the starting material is protected with a group such as t-butoxycarbonyl (Boc), trifluoroacetyl, carbobenzyloxy or carboethoxy, by reacting it, respectively, with di-t-butyl dicarbonate, trifluoroacetic anhydride, benzyl chloroformate or ethylchloroformate. The preferred protecting group, t-butoxycarbonyl, is illustrated in scheme 2. The reaction of the starting material with di-t-butyl dicarbonate is typically carried out in a polar solvent such as THF, dichloromethane or chloroform, at a temperature from about 0°C to about 100°C. The preferred solvent is dichloromethane and the preferred temperature is room temperature. The reaction is generally carried out for about 0.5 to 72 hours. This reaction yields a compound of the formula VIII having the same stereo- chemistry as the starting material.

The compound of formula VIII so formed is then reacted with a compound of the formula X-( Z ) -R⁶ wherein X is halo, or CH₃SO₂O-( Z ) -R⁶, to form a compound of the formula IX having the same stereochemistry. In each of X-( Z ) -R⁶ and CH₃SO₂O-( Z ) -R⁶, Z is (CH₂)ₘ and one of the carbons of said (CH₂)ₘ may optionally be substituted with R⁸ and one of the carbon-carbon single bonds of said (CH₂)ₘ may optionally be replaced with a carbon-carbon double bond or a carbon-carbon triple bond. This reaction is generally carried out in the presence of a base such as potassium hydroxide, potassium t-butoxide, lithium diisopropylamine or sodium methoxide, in a polar solvent such as t-butanol or DMF, for about 0.5 to about 24 hours. The preferred base is potassium t-butoxide and the preferred solvent is t-butanol. Reaction temperatures will generally range from about -25°C to about 150°C. The preferred temperature is generally the reflux temperature of the solvent.

The protecting group is then removed from the compound of formula IX by reacting it with an acid such as hydrochloric acid, trifluoroacetic acid or perchloric acid, to yield a compound of the formula X having the same stereochemistry. Appropriate solvents for this reaction include polar solvents such as methylene chloride, dioxane, ether or THF, preferably dioxane. The reaction is typically run at a temperature from about -10°C to about 50°C, preferably about 25°C, for about 0.5 to about 24 hours.

Reduction of the compound of formula X so formed yields a compound of the formula IB having the same stereochemistry. This reaction is carried out in the same manner as described above in the discussion of scheme 1 for preparing compounds of the formula IA from compounds of the formula VI, and for preparing compounds of the formula IC from compounds of the formula VII.

Scheme 3 illustrates a method of preparing compounds of the formula ID. Formula ID represents compounds of the formula I wherein each of R¹ and R⁶ are hydrogen, m is 0 and n is 2, 3 or 4. This group of compounds includes those of the formula IA. The method of scheme 3 can be used to prepare the pure 2S,3S enantiomer, the pure 2R,3R enantiomer, or a racemic mixture of a compound of the formula ID, depending on whether the starting material is, respectively, the R-enantiomer, the S-enantiomer, or a racemic mixture of the starting material of formula XI. Also, because formula ID includes compounds of the formula IA, the method of scheme 3 can be used to prepare compounds of the formula IA wherein R⁴ is attached to the "6" position of the nitrogen containing ring. The method of scheme 3 can also be used to prepare compounds of the formula ID wherein R² is benzhydryl.

Referring to scheme 3, compounds of the formula ID may be prepared as follows. The pure R-enantiomer, S-enantiomer or a racemic mixture of a compound of the formula XI is reacted with a nitrogen-protecting reagent such as t-butyldimethylsilyl chloride (TBDMS-Cl), t-butyldimethylsilyl triflate (TBDMS-OTf) or benzyl bromide/t-butoxide, preferably TBDMS-Cl, to form a compound of the formula XII. This reaction is typically carried out in a polar solvent such as DMF or triethyl- amine, preferably triethylamine, at a temperature of from about 0 to about 140°C. Room temperature is preferred.

The above reaction is followed by a stereospecific alkylation of the compound of formula XII to form the trans stereoisomer of a compound of the formula XIII. First, the compound of formula XII is reacted with lithium diethylamide in a polar solvent such as ether or THF, preferably THF, at a temperature from about -100°C to about room temperature, preferably about -78°C. Then, a compound of the formula 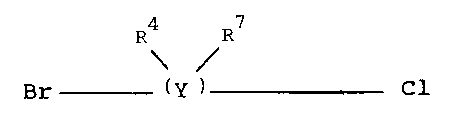
is added to the reaction mixture to produce the trans isomer of a compound of the formula XIII. Simultaneous removal of the TBDMS group and cleavage of the β-lactam using concentrated sulfuric or perchloric acid, preferably sulfuric acid, in a polar solent such as methanol or ethanol, preferably methanol, yields a compound of the formula XIV. This reaction is typically carried out at a temperature from about room temperature to about 150°C, preferably at about the reflux temperature of the solvent, for about 0.5 to about 16 hours.

The cyclization of the compound of formula XIV to produce a compound of the formula XV is accomplished by heating the crude product of formula XIV from the foregoing reaction at a temperature from about 80°C to about 140°C, preferably at about 100°C, for about 5 minutes to about 2 days, preferably for about 15 minutes, in a high boiling solvent such as DMF or toluene, preferably in DMF. Generally, this reaction is conducted in the presence of sodium iodide and sodium bicarbonate. In the compound of formula XV produced by this reaction, R² and -COOCH₃ are cis to each other.

The compound of formula XV is then treated with benzylchloroformate in a polar solvent such as water, water/acetone, chloroform, dichloroethane or ethyl acetate, in the presence of a base such as triethylamine or sodium bicarbonate, to yield the N-carbobenzyloxy piperidine (N-Cbz piperidine) of formula XVI having the same stereochemistry (i.e., wherein R² and -COOCH₃ are in the cis configuration). This reaction may be carried out at temperatures from about 0°C to about 100°C, preferably about 25°C, for about 5 minutes to 18 hours. Treatment of the compound of formula XVI so formed with about 5 equivalents each of trimethyl aluminum and ammonium chloride in a nonpolar solvent such as benzene or toluene for about 0.5 to about 16 hours yields a compound of the formula XVII having the same stereochemistry. Reaction temperatures may range from about room temperature to about 100°C, with about 50°C being preferred.

The conversion of the carboxamide group of the compound of formula XVII to form a compound of the formula XVIII having the same stereochemistry may be accomplished by a Hoffmann degradation using reagents such as bromine/sodium methoxide in methanol, lead tetraacetate in t-butyl alcohol, tin (IV) chloride, iodobenzene bis(trifluoroacetate) in aqueous acetonitrile, sodium bromide or benzyltrimethyl ammonium tribromide. Preferably, the compound of formula XVII is treated with lead tetraacetate in t-butanol. This reaction is typically carried out at a temperature from about room temperature to the reflux temperature of the solvent, preferably at the reflux temperature, for about 15 minutes to about 10 hours, preferably for about 3 to about 5 hours. Reaction of the compound of formula XVIII with an acid such as hydrochloric acid, trifluroacetic acid or perchloric acid yields a compound of the formula XIX having the same stereochemistry. The solvent is typically a polar solvent such as methylene chloride, dioxane, ether or THF, preferably dioxane. This reaction is typically carried out at a temperature from about -10 to about 50°C, preferably at about 25°C, for about 0.5 to 24 hours.

Reductive amination of the compound of the formula XIX from the above step with sodium cyanoborohydride or sodium triacetoxyborohydride and a compound of the formula R³CHO yields a compound of the formula XX having the same stereochemistry. This reaction is generally carried out in a polar solvent such as acetic acid or a lower alkanol, at a temperature from about 0 to about 50°C. Methanol is the preferred solvent and about 25°C is the preferred temperature. It is also preferred that the pH of the reaction mixture be about 4 to about 5.

The compound of formula XX is converted into a compound of the formula ID wherein R² and the amino group are cis to each other by reacting it with ammonium formate in the presence of palladium on charcoal (e.g. 10% palladium on charcoal). Typically, a polar solvent such as ethyl acetate or a lower alkanol is used, and the reaction is run at a temperature from about room temperature to about 150°C for about 0.5 to about 24 hours. Preferably, the reaction is conducted in ethanol at room temperature for about 3 to about 24 hours.

The trans isomer of a compound of the formula ID (i.e., one wherein the amino group and R² are trans to each other) may be prepared by the same procedure described above for obtaining the cis isomer, with the following modification. To prepare the trans isomer, either the compound of formula XV or the compound of formula XVI, after its formation as described above, is treated with potassium t-butoxide or a lithium dialkylamide. The solvent for this reaction is generally a polar solvent such as THF or ether, and the reaction is generally conducted at a temperature from about -78°C to room temperature, preferably at about 0°C, for about 5 minutes to about 10 hours.

An alternate method of preparing compounds of the formula ID wherein R² is benzhydryl is described in Examples 21-26.

Scheme 4 illustrates a preferred method of preparing compounds of the formula ID wherein n is 2. According to this method, a compound of the formula XXI is treated with hydrogen gas in the presence of a metal catalyst such a palladium on charcoal, platinum on charcoal or platinum dioxide, preferably palladium on charcoal, and in the presence of an acid such as trifluroacetic acid or hydrochloric acid, to produce a compound of the formula XXII. A polar inert solvent is generally used. The preferred solvent is ethanol. This reaction is typically carried out at a pressure of about 1.5 atm to about 5 atm, preferably at about 3.0 atm, at a temperature from about 0°C-60°C, preferably at about 25°C. The compound of formula XXII so formed is then converted to a compound of the formula ID by the procedure illustrated in scheme 3 and described above.

Enantiomerically pure compounds of the formula IC (i.e., compounds of the formula ID wherein R¹ is (C₁-C₆) alkyl rather than hydrogen) may be prepared as follows. A compound of the formula XX, prepared as described above, is alkylated by reacting it with a compound of the formula R¹X, wherein X is halo. This reaction is usually conducted in the presence of a base such as triethylamine or potassium t-butoxide, in a polar solvent such as methylene chloride or dichloroethane, and at a temperature from about room temperature to about 200°C. Preferably, the reaction is conducted at the reflux temperature in methylene chloride in the presence of triethylamine. The alkylated product, which has the same stereochemistry as the starting material of formula XX, is then converted to a compound of the formula IC having the same stereochemistry, by reacting it with ammonium formate in the presence of palladium on charcoal (e.g. 10% palladium on charcoal). Typically, a polar solvent such as ethyl acetate or a lower alkanol is used, and the reaction is run at a temperature from about room temperature to about 80°C for about 3 to about 24 hours. The reaction is preferably conducted in ethanol at room temperature for about 0.5 to about 24 hours.

Enantiomerically pure compounds of the formula IB may be prepared by reacting the analogous compound of the formula ID, having the same stereochemistry, with a compound of the formula R⁶-( Z ) -X, wherein X is halo or mesylate. In each of X-( Z ) -R⁶ and CH₃SO₂O-( Z ) -R⁶, Z is (CH₂)ₘ and one of the carbons of said (CH₂)ₘ may optionally be substituted with R⁸ and one of the carbon-carbon single bonds of said (CH₂)ₘ may optionally be replaced with a carbon-carbon double bond. The reaction is performed in the same manner as described above for converting compounds of the formula IA into compounds of the formula IB.

Compounds having the formula IA wherein R⁴, R⁵ and R⁷ are each hydrogen and R² is phenyl may be prepared, alternatively, by reductive amination of 3-amino-2-phenylpiperidine, using the appropriate aldehyde of the formula R³CHO, as described above for converting compounds of the formula V to the corresponding compounds of the formula VI. The starting material for this reaction, 3-amino-2-phenylpiperidine, may be prepared by hydrogenolysis of 3-(2-methoxybenzylamino)-2-phenylpiperidine. The hydrogenolysis reaction is usually carried out using a catalyst such as palladium on carbon or palladium hydroxide, in an inert solvent such as acetic acid or an alcoholic solvent, at a temperature from about 0°C to about 50°C. It is preferably carrier out at about room temperature in a methanol/ethanol solvent. It is also preferable to conduct this reaction in the presence of a mineral acid such as hydrochloric or sulfuric acid.

The above two step process for preparing compounds of the formula IA wherein R⁴, R⁵ and R⁷ are each hydrogen and R² is phenyl from 3-(2-methoxybenzylamino)-2-phenylpiperidine preserves the stereochemistry at the "2" and "3" positions of the piperidine ring. It therefore may be used to produce either pure enantiomer or a racemic mixture of the product of formula IA from a sample of 3-(2-methoxybenzylamino)-2-phenylpiperidine having the same stereochemistry. Similarly, the first step of the above process may be used to produce either pure enantiomer or a racemic mixture of 3-amino-2-phenylpiperidine.

An alternative method of preparing racemic 3-amino-2-phenylpiperidine is by reducing 3-amino-2-phenylpyridine. This reduction is generally accomplished using either sodium in alcohol, lithium aluminum hydride/aluminum trichloride, electrolytic reduction or hydrogen in the presence of a metal catalyst. The reduction with sodium is generally conducted in a boiling alcohol, preferably butanol, at a temperature from about 20°C to about the reflux temperature of the solvent, preferably at about 120°C. The reduction with lithium aluminum hydride/aluminum trichloride is usually carried out in ether, THF or dimethoxyethane, preferably ether, at a temperature from about 25°C to about 100°C, preferably at about room temperature. The electrolytic reduction is conducted, preferably, at room temperature, but temperatures from about 10°C to about 60°C are also suitable.

Hydrogenation in the presence of a metal catalyst is the preferred method of reduction. Suitable hydrogenation catalysts include palladium, platinum, nickel and rhodium. The preferred catalyst for hydrogenation is platinum oxide. The reaction temperature may range from about 10°C to about 50°C, with about 25°C being preferred. The hydrogenation is generally carried out at a pressure from about 1.5 to about 4 atmospheres, preferably at about 3.0 atmospheres.

Compounds of the formula IA wherein R⁴, R⁵ and R⁷ are each hydrogen and R² is phenyl may also be prepared by the following method. According to this method, 3-amino-2-phenylpyridine is first converted into the pyridine analog of the desired piperidine of the formula IA by reacting it with the appropriate compound of the formula R³CHO or R³CH₂X wherein X is a leaving group (e.g. chloro, bromo, iodo, mesylate or tosylate).

The reaction of 3-amino-2-phenylpyridine with a compound of the formula R³CHO to produce the pyridine analog of the piperidine of formula IA is typically carried out in the presence of a reducing agent such as sodium cyanoborohydride, sodium triacetoxyborohydride, sodium borohydride, hydrogen and a metal catalyst, zinc and hydrochloric acid, or formic acid at a temperature from about -60°C to about 50°C. Suitable reaction inert solvents for this reaction include lower alcohols (e.g., methanol, ethanol and isopropanol), acetic acid and THF. Preferably, the solvent is methanol, the temperature is about 25°C, and the reducing agent is sodium cyanoborohydride.

Alternatively, the reaction of 3-amino-2-phenylpyridine with a compound of the formula R³CHO may be carried out in the presence of a drying agent or using an apparatus designed to remove azeotropically the water generated, to produce an imine of the formula 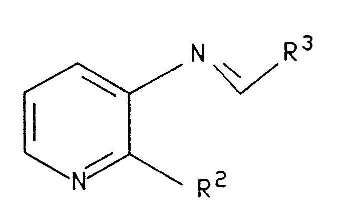
which is then reacted with a reducing agent as described above, preferably with sodium triacetoxyborohydride at about room temperature. The preparation of the imine is generally carried out in a reaction inert solvent such as benzene, xylene or toluene, preferably toluene, at a temperature from about 25°C to about 110°C, preferably at about the reflux temperature of the solvent. Suitable drying agents/solvent systems include titanium tetrachloride/dichloromethane and molecular sieves/THF. Titanium tetrachloride/dichloromethane is preferred.

The reaction of 3-amino-2-phenylpyridine with a compound of the formula R³CH₂X is typically carried out in a reaction inert solvent such as dichloromethane or THF, preferably dichloromethane, at a temperature from about 0°C to about 60°C, preferably at about 25°C.

The pyridine so formed is then reduced to form the desired piperidine of formula IA by the procedure described above for reducing 3-amino-2-phenylpyridine.

Compounds of the formula IB may be prepared, in addition to the method illustrated in scheme 1 and described above, from other compounds of the formula IB by modifying the R⁶ and R⁸ containing side chain. The appropriate modifications may be accomplished using methods well known to those skilled in the art. Some of these modifications are described in Examples 93-104.

The preparation of other compounds of the formula I not specifically described in the foregoing experimental section can be accomplished using combinations of the reactions described above that will be apparent to those skilled in the art.

In each of the reactions discussed or illustrated in schemes 1 to 4 above, pressure is not critical unless otherwise indicated. Pressures from about 0.5 atmospheres to about 5 atmospheres are generally acceptable, and ambient pressure, i.e. about 1 atmosphere, is preferred as a matter of convenience.

The novel compounds of the formula I and the pharmaceutically acceptable salts thereof are useful as substance P antagonists, i.e., they possess the ability to antagonize the effects of substance P at its receptor site in mammals, and therefore they are able to function as therapeutic agents in the treatment of the aforementioned disorders and diseases in an afflicted mammal.

The compounds of the formula I which are basic in nature are capable of forming a wide variety of different salts with various inorganic and organic acids. Although such salts must be pharmaceutically acceptable for administration to animals, it is often desirable in practice to initially isolate a compound of the Formula I from the reaction mixture as a pharmaceutically unacceptable salt and then simply convert the latter back to the free base compound by treatment with an alkaline reagent and subsequently convert the latter free base to a pharmaceutically acceptable acid addition salt. The acid addition salts of the base compounds of this invention are readily prepared by treating the base compound with a substantially equivalent amount of the chosen mineral or organic acid in an aqueous solvent medium or in a suitable organic solvent, such as methanol or ethanol. Upon careful evaporation of the solvent, the desired solid salt is readily obtained.

The compounds of Formula I and their pharmaceutically acceptable salts exhibit substance P receptor-binding activity and therefore are of value in the treatment and prevention of a wide variety of clinical conditions the treatment or prevention of which are effected or facilitated by a decrease in substance P mediated neurotransmission. Such conditions include inflammatory diseases (e.g., arthritis, psoriasis, asthma and inflammatory bowel disease), anxiety, depression or dysthymic disorders, colitis, psychosis, pain, allergies such as eczema and rhinitis, chronic obstructive airways disease, hypersensitivity disorders such as poison ivy, vasospastic diseases such as angina, migraine and Reynaud's disease, fibrosing and collagen diseases such as scleroderma and eosinophilic fascioliasis, reflex sympathetic dystrophy such as shoulder/hand syndrome, addiction disorders such as alcoholism, stress related somatic disorders, peripheral neuropathy, neuralgia, neuropathological disorders such as Alzheimer's disease, AIDS related dementia, diabetic neuropathy and multiple sclerosis, disorders related to immune enhancement or suppression such as systemic lupus erythematosus, and rheumatic diseases such as fibrositis. Hence, these compounds are readily adapted to therapeutic use as substance P antagonists for the control and/or treatment of any of the aforesaid clinical conditions in mammals, including humans.

The compounds of the formula I and the pharmaceutically acceptable salts thereof can be administered via either the oral, parenteral or topical routes. In general, these compounds are most desirably administered in dosages ranging from about 5.0 mg up to about 1500 mg per day, although variations will necessarily occur depending upon the weight and condition of the subject being treated and the particular route of administration chosen. However, a dosage level that is in the range of about 0.07 mg to about 21 mg per kg of body weight per day is most desirably employed. Variations may nevertheless occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effect, provided that such larger doses are first divided into several small doses for administration throughout the day.

The compounds of the invention may be administered alone or in combination with pharmaceutically acceptable carriers or diluents by either of the three routes previously indicated, and such administration may be carried out in single or multiple doses. More particularly, the novel therapeutic agents of this invention can be administered in a wide variety of different dosage forms, i.e., they may be combined with various pharmaceutically acceptable inert carriers in the form of tablets, capsules, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the therapeutically-effective compounds of this invention are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a therapeutic compound of the present invention in either sesame or peanut oil or in aqueous propylene glycol may be employed. The aqueous solutions should be suitably buffered (preferably pH greater than 8) if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

Additionally, it is also possible to administer the compounds of the present invention topically when treating inflammatory conditions of the skin and this may preferably be done by way of creams, jellies, gels, pastes, ointments and the like, in accordance with standard pharmaceutical practice.

The activity of the compounds of the present invention as substance P antagonists is determined by their ability to inhibit the binding of substance P at its receptor sites in bovine caudate tissue, employing radioactive ligands to visualize the tachykinin receptors by means of autoradiography. The substance P antagonizing activity of the herein described compounds may be evaluated by using the standard assay procedure described by M. A. Cascieri et al., as reported in the Journal of Biological Chemistry, Vol. 258, p. 5158 (1983). This method essentially involves determining the concentration of the individual compound required to reduce by 50% the amount of radiolabelled substance P ligands at their receptor sites in said isolated cow tissues, thereby affording characteristic IC₅₀ values for each compound tested.

In this procedure, bovine caudate tissue is removed from a -70°C freezer and homogenized in 50 volumes (w./v.) of an ice-cold 50 mM Tris (i.e., trimethamine which is 2-amino-2-hydroxymethyl-1,3-propanediol) hydrochloride buffer having a pH of 7.7. The homogenate is centrifuged at 30,000 x G for a period of 20 minutes. The pellet is resuspended in 50 volumes of Tris buffer, rehomogenized and then recentrifuged at 30,000 x G for another twenty- minute period. The pellet is then resuspended in 40 volumes of ice-cold 50 mM Tris buffer (pH 7.7) containing 2 mM of calcium chloride, 2 mM of magnesium chloride, 40 g/ml of bacitracin, 4µg/ml of leupeptin, 2µg of chymostatin and 200 g/ml of bovine serum albumin. This step completes the production of the tissue preparation.

The radioligand binding procedure is then carried out in the following manner, viz., by initiating the reaction via the addition of 100 µl of the test compound made up to a concentration of 1 µM, followed by the addition of 100 µl of radioactive ligand made up to a final concentration 0.5 mM and then finally by the addition of 800 µl of the tissue preparation produced as described above. The final volume is thus 1.0 ml, and the reaction mixture is next vortexed and incubated at room temperature (ca. 20°C) for a period of 20 minutes. The tubes are then filtered using a cell harvester, and the glass fiber filters (Whatman GF/B) are washed four times with 50 mM of Tris buffer (pH 7.7), with the filters having previously been presoaked for a period of two hours prior to the filtering procedure. Radioactivity is then determined in a Beta counter at 53% counting efficiency, and the IC₅₀ values are calculated by using standard statistical methods.

The anti-psychotic activity of the compounds of the present invention as neuroleptic agents for the control of various psychotic disorders is determined primarily by a study of their ability to suppress substance P-induced or substance P agonist induced hypermotility in guinea pigs. This study is carried out by first dosing the guinea pigs with a control compound or with an appropriate test compound of the present invention, then injecting the guinea pigs with substance P or a substance P agonist by intracerebral administration via canula and thereafter measuring their individual locomotor response to said stimulus.

The present invention is illustrated by the following examples. It will be understood, however, that the invention is not limited to the specific details of these examples.

### EXAMPLE 1

### Cis-3-(2-methoxybenzylamino)-2-phenylpiperidine

### A. 2-Oxo-5-oximino-6-phenylpiperidine

To a stirred solution of trans-5-nitro-2-oxo-6-phenylpiperidine (27.0 gms, 122.6 mmole) in 1:1 methylene chloride:methanol was added potassium tert. butoxide (135 mmole, 15.1 gms) at 25°C. This reaction mixture was cooled to -78°C and ozone gas was bubbled until ( 3 hrs) TLC (10% methanol in methylene chloride) indicated no starting material. The reaction mixture was then purged with nitrogen to remove excess ozone, and was then treated with dimethyl sulfide (60 ml) at -78°C. After warming to room temperature in 30 min., it was treated with an aqueous solution of hydroxylamine (85.2 gms, 1.22 mole) and sodium acetate (50.3 gms, 613 mmole) in water (220 ml). After stirring for 16 hrs, the volatile material was removed using a rotary evaporator. The residue was poured into 1.2 liters of cold water and stirred for 30 min. The precipitated solid was filtered to give 2-oxo-3-oxamino-6-phenylpiperidine (14.0 gms, 56.0%). M.p. 178°C.
¹H NMR (DMSO-d₆, 300 MHz, δ): 2.04-2.22 (2H, m); 2.4-2.42 (1H, m), 2.71 (1H, dt, J = 8, 16 Hz); 5.02 (1H, d, J = 4 Hz), 7.28-7.41 (5H, m); 8.35 (1H, d, J = 4 Hz); 10.99 (1H, s).
TLC: (90:10 - methylene chloride:methanol) R_{f}=0.54.

### B. Cis-5-(2-methoxybenzylamino)-2-oxo-6-phenylpiperidine:

2-Oxo-5-oximino-6-phenylpiperidine (28.2 gms, 138 mmole) was dissolved (heating on steam bath is necessary to achieve a clear solution) in ethanol (500 ml) containing methanol (50ml). Neutral Raney Ni (80 gms) was added and the mixture was shaken on a Parr shaker under hydrogen (40 psi). After 18 hrs, the reaction mixture was filtered through diatomaceous earth (Celite (Trademark)) which was thoroughly washed with methanol. The organic solvents were removed using a rotary evaporator to afford an oil which solidified on standing (26.2 gms, 100%). ¹H-NMR indicated it to be a 3:1 mixture of cis-5-amino-2-oxo-6-phenylpiperidine and trans-5-amino-2-oxo-6-phenylpiperidine, respectively. This mixture was dissolved in methanol (345 ml) and the pH was adjusted to 5 with saturated methanolic hydrochloric acid. Four Å sieves (55 gms), sodium cyanoborohydride (138 mmole) and o-methoxy-benzaldehyde (22.5 gms, 165 mmole) were added to the system. Stirring was continued ( 4 hours) until the reaction was complete as indicated by TLC. The reaction mixture was filtered through diatomaceous earth (Celite (trademark)) and the filtrate was concentrated using a rotary evaporator. The residue was suspended in water and the pH made basic. The aqueous phase was extracted with methylene chloride (4 x 200 ml) washed with water, brine, and then dried (anhyd. magnesium sulfate) and concentrated to give an oil (47.0 gms) which was flash chromatographed. Elution with 3% methanol in methylene chloride afforded a white solid (19.6 gms, m.p. 122°C).
¹H NMR (CDCl₃) δ1.81-1.96 (1H, m); 2.0-2.18 (1H, m); 2.4 (1H, dt, J = 4.5, 16 Hz); 2.75 (1H, ddd, J = 6.5, 10.5 16 Hz); 3.48 (3H, s); 3.54 (1H, dd, J = 13.8 Hz); 3.76 (1H, dd, J = 13.8 Hz); 4.72 (1H, d, J = 4Hz); 5.72 (1H, bs); 6.71 (1H, d, J = 8 Hz); 6.8 (1H, t, J = 6.8 Hz); 7.04 (1H, dd, J = 1.8, 7.2 Hz); 7.17 (1H, dt, J = 1.6, 8.2 Hz); 7.2-7.44 (5H, m).
HRMS: Calculated for C₁₉H₂₂N₂O₂: 310.1682;
Found 310.1649.
TLC: (90:10 - methylene chloride:methanol) R_{f} = 0.47.

### C. Cis-3-(2-methoxybenzylamino)-2-phenylpiperidine

Borane dimethylsulfide in tetrahydrofuran (2M, 158 ml, 315 mmole) was added to a solution of cis-5-(2-methoxybenzylamino)-2-oxo-6-phenylpiperidine (19.6 g, 63.0 mmole) in tetrahydrofuran (500 ml) under nitrogen and the reaction mixture was heated at reflux for 18 hours. At the end of this period, the reaction mixture was cooled and the excess borane dimethylsulfide was cautiously decomposed by dropwise addition of methanol. The contents of the reaction mixture were then concentrated under vacuum. Ethanol (500 ml) and powdered potassium carbonate (17.5 g, 126 mmole) were added to the residue and the reaction mixture was heated at reflux (18 hours). Then the reaction mixture was concentrated under vacuum and the residue was extracted with methylene chloride (4 x 250ml) and dried (anhydrous magnesium sulfate). The organic solvents were removed under vacuum to afford a residue which was dissolved in a minimum amount of methylene chloride. To this solution was added excess hydrochloric acid/ether, thus precipitating the dihydrochloride salt of cis-3-(2-methoxybenzylamino)-2-phenylpiperidine, which was isolated by filtration. This was heated at reflux in chloroform (400 ml) for 3 hours and filtered to give the essentially pure hydrochloride salt of the title compound (22.4 gms, m.p. 245°C, 96%), which was crystallized from a mixture of hot methanol-ethanol to afford a white crystalline solid (19.2 gms, 83%).
M.p. 255°C (HCl salt). ¹H-NMR (CDCl₃, free base) δ 7.1-7.3 (6H, m); 6.97 (1H, dd, J = 1.7, 7.4 Hz); 6.79 (1H, bt, J = 7.4 Hz); 6.66 (1H, d, J = 8.2 Hz); 3.87 (1H, d, J = 2.3 Hz); 3.67 (1H, d, J = 11.4 Hz): 3.44 (3H, s); 3.4 (1H, d, J = 14 Hz); 3.22-3.3 (1H, bd, J = 12.2 Hz); 2.72-2.86 (2H, m); 2.09-2.19 (1H, bd, J = 13.7 Hz); 1.84-2.01 (1H, dt, J = 4.0, 13.0 Hz); 1.53-1.7 (1H, dt, J = 3.5, 13.4 Hz); 1.33-1.45 (1H, bd, J = 12.5 Hz). ¹³C-NMR (CDCl₃, free base) δ 157.6, 142.5, 129.6, 128.3, 128.2, 127.8, 126.5, 126.3, 120.0, 109.8, 64.0, 54.8, 54.7, 47.8, 46.7, 28.2, 20.4. HRMS Calcd. for C₁₉H₂₄N₂O: 296.1886. Found: 296.1904.
TLC : (90:10 - methylene chloride:methanol) R_{f} = 0.39.

### EXAMPLE 2

### Cis-1-allyl-3-(2-methoxybenzylamino)-2-phenylpiperidine

Under a nitrogen atmosphere, in a round-bottom flask, were placed 60 mg (0.2 mmol) of the title compound of Example 1 and 0.2 ml of methylene chloride. To this system were added 28 µl (0.2 mmol) of triethylamine and 17.5 µl (0.2 mmol) of allyl bromide, and the reaction mixture was stirred at room temperature overnight. The mixture was partitioned between methylene chloride and saturated aqueous sodium bicarbonate, the layers were separated, and the aqueous phase was extracted with three portions of methylene chloride. The combined organic fractions were dried (sodium sulfate) and concentrated with a rotary evaporator. The crude material was purified by flash column chromatography to obtain 26 mg of the title compound.
¹H NMR (CDCl₃) δ 7.20 (m, 5H), 7.03 (t, 1H, J = 6 Hz), 6.79 (d, 1H, J = 6 Hz), 6.88 (t, 1H, J = 6 Hz), 6.57 (d, 1H, J = 6 Hz), 5.78 (m, 1H), 4.94 (m, 2H), 3.62 (d, 1H, J = 12 Hz), 3.40 (s, 3H), 3.32 (d, 1H, J = 12 Hz), 3.26 (d, 1H, J = 2 Hz), 3.18 (m, 1H), 2.56 (m, 1H), 2.36 (m, 1H), 1.98 (m, 3H), 1.68 (m, 1H), 1.38 (m, 2H). HRMS: Calcd for C₂₂H₂₈N₂O: 336.2202. Found: 336.2216.

### EXAMPLE 3

### Cis-1-ethyl-3-(2-methoxybenzylamino)-2-phenylpiperidine

### A. Cis-5-(N-tert-butoxycarbonyl-2-methoxybenzylamino)-2-oxo-6-phenylpiperidine

Under a nitrogen atmosphere in a round-bottom flask were placed 2.0 g (6.4 mmol) of cis-5-(2-methoxybenzylamino)-2-oxo-6-phenylpiperidine, 7 mL of methylene chloride and 14.1 g (64.5 mmol) of di-tert-butyldicarbonate. The reaction mixture was stirred at room temperature for 4 days, poured into saturated aqueous sodium bicarbonate and extracted with two portions of methylene chloride. The combined organic fractions were washed with H₂O, dried (sodium sulfate) and concentrated with a rotary evaporator to obtain 16 g of oil. The crude material was purified by flash column chromatography to obtain 2.4 g (91% yield) of cis-5-(N-tert-butoxycarbonyl-2-methoxybenzylamino)-2-oxo-6-phenylpiperidine as a white solid.
¹H NMR (CDCl₃) δ 7.34 (m, 3H), 7.14 (m, 2H), 7.04 (m, 1H), 6.92 (d, 1H, J = 7 Hz), 6.79 (t, 1H, J = 7 Hz), 6.62 (d, 1H, J = 7 Hz), 5.00, 4.86 (2m, 1H), 4.68, 4.46 (2m, 1H), 4.00, 3.78 (2d, 1H, J = 18 Hz), 3.58 (s, 3H), 2.82 (d, 1H, J = 18 Hz), 2.20 (m, 2H), 1.80 (m, 1H), 1.44 (m, 1H), 1.53, 1.36 (2s, 3H).

### B. Cis-N-ethyl-5-(2-methoxybenzylamino)-2-oxo-6-phenylpiperidine

Under a nitrogen atmosphere in a round-bottom flask were placed 50 mg (0.12 mmol) of cis-5-(N-tert-butoxycarbonyl-2-methoxybenzylamino)-2-oxo-6-phenylpiperidine and 0.2 mL of THF. To the system were added 13.5 mg (0.12 mmol) of potassium tert-butoxide and 20 µL (0.24 mmol) of ethyl iodide. The reaction mixture was stirred at room temperature for 3 hours (during this period, additional potassium tert-butoxide (13.5 mg) and ethyl iodide (20 µL) were added to the system). The mixture was partitioned between methylene chloride and aqueous sodium bicarbonate, the layers were separated and the aqueous phase was extracted with three portions of methylene chloride. The combined organic fractions were dried (sodium sulfate) and concentrated with a rotary evaporator. The crude material was purified by flash column chromatography using 3:97 methanol/chloroform as the eluant to obtain 42 mg of cis-N-ethyl-5-(2-methoxybenzylamino)- 2-oxo-6-phenylpiperidine.
¹H NMR (CDCl₃) δ 7.36 (m, 3H), 7.10 (m, 3H), 6.92 (d, 1H, J = 6Hz), 6.80 (t, 1H, J = 6 Hz), 6.63 (d, 1H, J = Hz), 4.97, 4.82 (2m, 1H), 4.60, 4.40 (2m, 1H), 4.00 (m, 1H), 3.80 (m, 1H, J = 18 Hz), 3.58 (s, 3H), 2.80 (d, 1H, J = 18 Hz), 2.50 (m, 3H), 1.80 (m, 1H), 1.56, 1.38 (2s, 9H), 1.06 (t, 3H, J = 7Hz). Mass spectrum m/e 438 (parent).

### C. Cis-1-ethyl-3-(2-methoxybenzylamino)-2-oxo-6-phenylpiperidine.

In a round bottom flask were placed 173 mg (0.39 mmol) of cis-N-ethyl-5-(N-tert-butoxycarbonyl-2-methoxybenzylamino)-2-oxo-6-phenylpiperidine and 0.5 mL of dioxane. To this system were added 5 mL of dioxane saturated with hydrogen chloride. The reaction mixture was stirred at room temperature for 2.5 hours and concentrated with a rotary evaporator. The residue was partitioned between saturated aqueous sodium bicarbonate and chloroform and extracted with three portions of chloroform.

The combined organic fractions were dried (sodium sulfate) and concentrated to obtain 84 mg of cis-1-ethyl-3-(2-methoxybenzylamino)-2-oxo-6-phenylpiperidine, which was used immediately without further purifiction;
¹H NMR (CDCl₃) δ 7.28 (m, 7H), 6.90 (t, 1H, J = 6 Hz), 6.81 (d, 1H, J = 6 Hz), 4.68 (d, 1H, J = 2 Hz), 3.88 (m, 3H), 3.74 (s, 3H), 3.14 (m, 1H), 2.56 (m, 3H), 1.76 (m, 1H), 1.54 (m, 1H), 1.04 (t, 3H, J = 6 Hz).

### D. Cis-1-Ethyl-3-(2-methoxybenzylamino)-2-phenylpiperidine

Under a nitrogen atmosphere, in a round-bottom flask were placed 80 mg (0.24 mmol) of the amine prepared above and 5 mL of THF. To this system was added 0.59 mL (1.18 mmol) of 2.0 M borane methylsulfide in THF, and the reaction mixture was heated overnight at 60°C. The mixture was cooled, ca. 2 mL of methanol was added carefully to the system, and the mixture was stirred for 1 hour and concentrated with a rotary evaporator. Sixty-six mg (0.48 mmol) of potassium carbonate in 2 mL of ethanol was added to the system, and the mixture was heated at reflux for 2.5 hours, cooled and concentrated. The residue was partitioned between water and methylene chloride, the layers were separated and the aqueous phase was extracted with three portions of dichloromethane. The combined organic fractions were dried (sodium sulfate) and concentrated to obtain 64 mg of a yellow oil. This oil was dissolved in methylene chloride, and then ether saturated with hydrogen chloride was added to the solution. The resulting yellow solid was collected, affording 60 mg of the hydrochloride salt of the title compound.
¹H NMR (free base, CDCl₃) δ 7.22 (m, 5H), 7.03 (t, 1H, J = 6 Hz), 6.78 (d, 1H, J = 6 Hz), 6.68 (t, 1H, J = 6 Hz), 6.56 ( d, 1H, J = 6 Hz), 3.62 (d, 1H, J = 12 Hz), 3.39 (s, 3H), 3.31 (d, 1H, J = 12 Hz), 3.25 (d, 1H, J = 2Hz), 3.16 (m, 1H), 2.55 (m, 2H), 1.99 (m, 2H), 1.86 (m, 2H), 1.40 (m, 2H), 0.90 (t, 3H, J = 6 Hz). HRMS Calc'd. for C₂₁H₂₈N₂O: 324.2201. Found: 324.2193.

The title compounds of Examples 4-14 were prepared by a procedure similar to that described in Example 2.

### EXAMPLE 4

### Cis-3-(2-methoxybenzylamino)-2-phenyl-1-(prop-1-yl)piperidine

M.p. 223-225°C. ¹H NMR (CDCl₃) δ 7.28 (m, 5H), 7.10 (t, 1H, J = 6 Hz), 6.87 (d, 1H, J = 6 Hz), 6.74 (t, 1H, J = 6 Hz), 6.60 (d, 1H, J = 6 Hz), 3.86 (d, 1 H, J = 12 Hz), 3.46 (d, 1H, J = 12 Hz), 3.40 (s, 3H), 3.29 (m, 1H), 2.64 (m, 1H), 2.50 (m, 1H), 2.02 (m, 4H), 1.46 (m, 4H), 0.72 (t, 3H, J = 7 Hz). Mass spectrum m/e 338 (parent).

### EXAMPLE 5

### Cis-1-butyl-3-(2-methoxybenzylamino)-2-phenylpiperidine

M.p. 139-140°C (HCl salt). ¹H NMR (CDCl₃) δ 7.20 (m, 5H), 7.02 (t, 1H, J = 6 Hz), 6.77 (d, 1 H, J = 6 Hz), 6.66 (t, 1H, J = 6 Hz), 6.55 (d, 1H, J = 6 Hz), 3.60 (d, 1H, J = 14 Hz), 3.37 (s, 1H), 3.30 (d, 1H, J = 14 Hz), 3.22 (d, 1H, J = 2Hz), 3.16 (m, 1H), 2.48 (m, 2H), 1.98 (m, 3H), 1.36 (m, 3H), 1.08 (m, 3H), 0.71 (t, 3H, J = 9 Hz). Mass spectrum m/e 352 (parent).

### EXAMPLE 6

### Cis-3-(2-methoxybenzylamino)-2-phenyl-1-(2-phenyleth-1-yl)piperidine

¹H NMR (CDCl₃) δ 7.18 (m, 10H), 6.92 (d, 1H, J = 6 Hz), 6.82 (d, 1H, J = 6 Hz), 6.71 (t, 1H, J = 6Hz), 6.00 (d, 1H, J = 6 Hz), 3.66 (d, 1H, J = 15 Hz), 3.44 (s, 3H), 3.35 (m, 2H), 2.72 (m, 3H), 2.60 (m, 1H), 2.12 (m, 4H), 1.68 (m, 1H), 1.44 (m, 2H). HRMS Calc'd. for C₂₇H₃₂N₂O: 400.2515. Found: 400.2521.

### EXAMPLE 7

### Cis-3-(2-methoxybenzylamino)-2-phenyl-1-propargylpiperidine

M.p. 147-149°C (HCl salt, dec). ¹H NMR (CDCl₃) δ 7.22 (m, 5H), 7.02 (t, 1H, J = 7 Hz), 6.82 (d, 1H, J = 7Hz), 6.68 (t, 1H, J = 7 Hz), 6.56 (d, 1H, J = 7 Hz), 3.62 (d, 1H, J = 12 Hz), 3.47 (d, 1H, J = 2Hz), 3.38 (m, 4H), 3.30 (d, 1H, J = 12 (Hz), 3.21 (d, 1H, J = 2 Hz), 3.15 (d, 1H, J = 2 Hz), 2.94 (m, 1 H), 2.55 (m, 2H), 2.06 (m, 3H), 1.40 (m, 1H). Mass spectrum m/e 334 (parent). Calc'd. for C₂₂H₂₆OH₂ 2HCl·2.75 H₂O: C, 57.83; H, 7.39; N, 6.13. Found: C, 57.81; H, 7.58; N, 5.91.

### EXAMPLE 8

### Cis-3-(2-Methoxybenzylamino)-2-phenyl-1-(3-phenylprop-1-yl)piperidine

M.p. 120-125°C (HCl salt, dec). ¹H NMR (CDCl₃) δ 7.14 (m, 1H), 6.80 (d, 1H, J = 6 Hz), 6.68 (t, 1H, J = 6Hz), 6.58 (d, 1H, J = 8 Hz), 3.62 (d, 1H, J = 14 Hz), 3.40 (s, 3H), 3.32 (d, 1H, J = 14 Hz), 3.26 (d, 1H, J = 2 Hz), 3.18 (m, 1H), 2.52 (m, 2H), 2.35 (m, 1H), 2.00 (m, 3H), 1.76 (m, 4H), 1.42 (m, 2H). Mass spectrum m/e 414 (parent). Calc'd. for C₂₈H₃₄ON₂ 2HCl·2.75H₂O: C, 62.62; H, 7.79; N, 5.22. Found: C, 62.63; H, 7.82; N, 5.08.

### EXAMPLE 9

### Cis-1-(carboxamidomethyl)-3-(2-methoxybenzylamino)-2-phenylpiperidine

M.p. 235°C (HCl salt). ¹H NMR (CDCl₃) δ 7.20 (m, 5H), 7.05 (t, 1H, J = 7 Hz), 6.82 (d, 1H, J = 7 Hz), 6.68 (t, 1H, J = 7 Hz), 6.56 (d, 1H, J = 7 Hz), 3.64 (d, 1H, J = 16 Hz), 3.39 (d, 1H, J = 2 Hz), 3.30 (s, 3H), 3.29 (d, 1H, J = 16 Hz), 3.20 (d, 1H, J = 18 Hz), 3.06 (m, 1H), 2.57 (m, 1H), 2.36 (d, 1H, J = 18 Hz), 2.06 (m, 3H), 1.41 (m, 2H). Mass spectrum m/e 353 (parent).

### EXAMPLE 10

### Cis-1-Carboxymethyl-3-(2-methoxybenzylamino)-2-phenylpiperidine

M.p. 58°C (HCl salt, very hygroscopic). ¹H NMR (CD₃OD) δ 7.72 (m, 2H), 7.62 (m, 3H), 7.36 (t, 1H, J = 7 Hz), 7.28 (d, 1H, J = 7 Hz), 6.96 (m, 2H), 5.14 (m, 1H), 4.18 (m, 2H), 4.00 (m, 1H), 3.66 (m, 3H), 3.40 (m, 1H), 2.34 (m, 5H), 2.07 (m, 1H). Mass spectrum m/e 354 (parent).

### EXAMPLE 11

### Cis-3-(2-Methoxybenzylamino)-2-phenyl-1-(5-phenylpent-1-yl)piperidine

M.p. 109°C (HCl salt, dec). ¹H NMR (CDCl₃) δ 7.14 (m, 11H), 6.78 (d, 1H, J = 6 Hz), 6.68 (t, 1H, J = 6 Hz), 6.56 (d, 1H, J = 6 Hz), 3.62 (d, 1H, J = 14 Hz), 3.40 (s, 3H), 3.32 (d, 1H, J = 14 Hz), 3.24 (d, 1H, J = 2 Hz), 3.16 (m, 1H), 2.50 (m, 4H), 2.00 (m, 4H), 1.76 (m, 1H), 1.42 (m, 5H), 1.14 (m, 2H). Mass spectrum m/e 442 (parent).

### EXAMPLE 12

### Cis(2-Methoxybenzylamino)-2-phenyl-1-(4-phenylbut-1-yl)piperidine

M.p. 65-70°C (HCl salt). ¹H NMR (CDCl₃) δ 7.20 (m, 11H), 6.84 (d, 1H, J = 7 Hz), 6.73 (t, 1H, J = 7 Hz), 6.62 (d, 1H, J = 7 Hz), 3.68 (d, 1H, J = 12 Hz), 3.44 (s, 3H), 3.38 (d, 1H, J = 12 Hz), 3.30 (d, 1H, J = 3 Hz), 3.18 (m, 1H), 2.34 (m, 4H), 2.02 (m, 3H), 1.80 (m, 1H), 1.47 (m, 6H). Mass spectrum m/e 428 (parent).

### EXAMPLE 13

### Cis-3-(2-Methoxybenzylamino)-2-phenyl-1-(3-phenyl-prop-2-ene-1-yl)piperidine

M.p. 54-58°C (HCl salt, dec). ¹H NMR (CDCl₃) δ 7.20 (m, 11H), 6.84 (d, 1H, J = 6 Hz), 6.72 (t, 1H, J = 6 Hz), 6.60 (d, 1H, J = 6 Hz), 6.28 (m, 2H), 3.76 (d, 1H, J = 12 Hz), 3.40 (m, 5H), 3.20 (m, 1H), 2.56 (m, 2H), 2.04 (m, 4H), 1.44 (m, 1H). Mass spectrum m/e 412 (parent).

### EXAMPLE 14

### Cis-3-(2-Methoxybenzylamino)-1-(2-phenoxyeth-1-yl)-2-phenylpiperidine

¹H NMR (CDCl₃) δ 7.26 (m, 7H), 7.08 (t, 1H, J = 6 Hz), 6.80 (m, 5H), 6.61 (d, 1H, J = 6 Hz), 4.04 (m, 1H), 3.68 (d, 1H, J = 14 Hz), 3.42 (s, 3H), 3.37 (d, 1H, J = 14 Hz), 2.97 (m, 1H), 2.60 (m, 1H), 2.28 (m, 2H), 2.06 (m, 3H), 1.47 (m, 1H), 1.26 (m, 3H). Mass spectrum m/e 323 (parent).

The title compounds of Examples 15-17 were prepared by a procedure similar to that described in Example 3.

### EXAMPLE 15

### Cis-3-(2-Methoxybenzylamino)-1-methyl-2-phenylpiperidine

M.P. 58°C (HCl salt, very hygroscopic, dec). ¹H NMR (CDCl₃) δ 7.22 (m, 5H), 7.04 (t, 1H, J = 6 Hz), 6.82 (d, 1H, J = 6 Hz), 6.78 (t, 1H, J = 6 Hz), 6.58 (d, 1H, J = 6 Hz), 3.62 (d, 1H, J = 12 Hz), 3.42 (s, 3H), 3.32 (d, 1H, J = 12 Hz), 3.02 (m, 2H), 2.56 (m, 1H), 2.04 (m, 3H), 2.02 (s, 3H), 2.38 (m, 2H). Mass spectrum m/e 310 (parent).

### EXAMPLE 16

### Cis-1-Benzyl-3-(2-Methoxybenzylamino)-2-phenylpiperidine

M.p. 68-70°C (HCl salt, dec). ¹H NMR (CDCl₃) δ 7.28 (m, 11H), 6.83 (d, 1H, J = 6 Hz), 6.70 (t, 1H, J = 6 Hz), 6.61 (d, 1H, J = 6 Hz), 3.85 (d, 1H, J = 14 Hz), 3.64 (d, 1H, J = 14 Hz), 3.47 (s, 3H), 3.35 (m, 2H), 2.96 (m, 1H), 2.79 (d, 1H, J = 14 Hz), 2.62 (m, 1H), 1.96 (m, 3H), 1.38 (m, 2H). Mass spectrum m/e 386 (parent).

### EXAMPLE 17

### Cis-1-(2-Hydroxyeth-1-yl)-3-(2-methoxybenzylamino)-2-phenylpiperidine

M.p. 148-149°C (HCl salt, dec). ¹H NMR (CDCl₃) δ 7.28 (m, 5H), 7.12 (t, 1H, J = 7 Hz), 6.88 (d, 1H, J = 7 Hz), 6.75 (t, 1H, J = 7 Hz), 6.63 (d, 1H, J = 7 Hz), 3.70 (m, 3H), 3.44 (m, 5H), 3.26 (m, 1H), 2.85 (m, 1H), 2.64 (m, 1H), 2.06 (m, 3H), 1.88 (m, 1H), 1.30 (m, 2H). HRMS Calc'd. for C₂₁H₂₈N₂O₃: 340.2150. Found: 340.2142. Calc'd. for C₂₁H₂₈O₂N₂ 2HCl·2.6H₂O: C, 54.81; H, 7.71; N, 6.08. Found; C, 54.81; H, 8.02; N, 5.82.

### EXAMPLE 18

### Cis-3-(2-Methoxybenzylamino)-2-phenylpyrrolidine

1-Benzyl-3-carboethoxy-2-phenyl-2,3-didehydropyrrolidine, made according to the procedure described by Celerier et al., Tetrahedron Lett., 28, 6597 (1987), (2.0 g, 6.5 mmol) was dissolved in 70 mL of ethanol. To this solution was added 1 mL of concentrated aqueous hydrogen chloride and 2.0 g of 5% palladium on carbon. The mixture was placed on a Parr apparatus (40 p.s.i. hydrogen) for 1 hour. The mixture was filtered through diatomaceous earth (Celite (trademark)) and the filtrate was concentrated with a rotary evaporator. Saturated aqueous sodium bicarbonate was added to the residue until the liquid was basic (pH 8), and the material was extracted with three portions of methylene chloride. The combined organic fractions were dried (sodium sulfate) and concentrated (rotary evaporator) to obtain 1.1 g of an oil. This material was suspended in 10 mL of 10% aqueous sodium bicarbonate, and the system was cooled in an ice bath. To the system was added 0.65 mL (4.6 mmol) of benzylchloroformate, the cold bath was removed and the mixture was stirred for 30 min. Ether was then added, the layers were separated, and the ether phase was washed with water, dried (sodium sulfate) and concentrated with a rotary evaporator. The crude material was purified by flash column chromatogrpahy (80 g of silica gel) using 1:3 ethyl acetate/hexanes as the eluant to obtain 940 mg of pure 1-benzyl-3-carboethoxy-2-phenylpyrrolidine. ¹H NMR (CDCl₃) 7.16 (m, 9H), 6.76 (m, 1H), 5.02 (m, 3H), 3.78 (m, 3H), 3.54 (m, 1H), 3.34 (m, 1H), 2.40 (m, 1H), 2.02 (m, 1H), 1.94 (t, 3H, J = 6 Hz). Mass spectrum m/e 353 (parent).

This material was converted to the title compound by a procedure similar to that described in Example 63 E-G. ¹H NMR (CDCl₃) δ 7.26 (m, 5H), 7.12 (t, 1H, J = 7 Hz), 6.98 (d, 1H, J = 7 Hz), 6.80 (t, 1H, J = 7 Hz), 6.70 (d, 1H, J = 6 Hz), 4.11 (d, 1H, J = 4 Hz), 3.86 (d, 1H, J = 12 Hz), 3.52 (s, 3H), 3.42 (d, 1H, J = 12 Hz), 3.34 (m, 1H), 3.25 (m, 1H), 2.98 (m, 1H), 1.9 (m, 2H).

### EXAMPLE 19

### Cis-3-(N,N-Methyl-2-methoxybenzylamino)-2-phenylpiperidine

Under a nitrogen atmosphere in a round-bottom flask were placed 75 mg (0.24 mmol) of the lactam 5-(2-methoxybenzylamino)-2-oxo-6-phenylpiperidine, 0.036 mL (0.48 mmol) of methyl iodide, 0.066 mL (0.48 mmol) of triethylamine and 0.2 mL of THF. The reaction mixture was stirred at room temperature for 5 hours and poured into saturated aqueous sodium bicarbonate. This mixture was extracted with three portions of methylene chloride. The methylene chloride extracts were dried (sodium sulfate) and concentrated with a rotary evaporator. The residue was resubjected to the above conditions, employing the following quantities of reagents: 0.11 mL (1.4 mmol) of methyl iodide and 0.066 mL (0.48 mmol) of triethylamine. The mixture was stirred at room temperature for 7.5 hours, and during this period additional methyl iodide (0.11 mL) was added to the system. The reaction mixture was treated as described above to obtain 70 mg of a clear colorless oil. The crude material was purified by flash column chromatography (7 g of silica gel) using 3:97 methanol/chlorform as the eluant to obtain 44 mg of cis-3-(N,N-methyl-(2-methoxybenzylamino) -2-phenylpiperidin-6-one.
¹H NMR (CDCl₃) δ 1.86 (m, 5H), 2.52 (m, 1H), 2.70 (m, 1H), 3.34 (m, 1H), 3.52 (d, 1H, J = 14), 3.74 (d, 1H, J = 14), 3.84 (s, 3H), 4.68 (m, 1H), 6.90, (m, 2H), 7.80 (m, 7H), HRMS: Calcd. for C₂₀H₂₄N₂O₂: 324.1838. Found: 324.1884.

Under a nitrogen atmosphere in a round-bottom flask were placed 54 mg (0.17 mmol) of cis-3-(N,N-methyl-(2-methoxy)benzylamino)-2-phenylpiperidin-6-one and 2.5 mL of THF. To the system was added slowly 0.43 mL (0.86 mmol) of 2.0 M borane-methylsulfide complex in THF, and the reaction mixture was heated at 60°C overnight. The reaction mixture was cooled to room temperature, methanol was added slowly to the system and the mixture was stirred at room temperature for 30 min and concentrated with a rotary evaporator. Two milliliters of ethanol and 48 mg (0.35 mmol) of potassium carbonate were then added, and the reaction mixture was heated at reflux for 4 hours and cooled to room temprature. The solvent was removed with a rotary evaporator. The residue was partitioned between chloroform and water, the layers were separated, and the aqueous phase was extracted with chloroform. The combined organic fractions were dried (sodium sulfate) and concentrated to obtain 75 mg of an oil. This oil was dissolved in a minimum volume of methylene chloride and ether saturated with hydrogen chloride was added to the solution. Water was added to the system, and the mixture was washed with two portions of methylene chloride. The aqueous phase was basified with aqueous sodium hydroxide and extracted with four portions of methylene chloride. These combined fractions were dried and concentrated to obtain 20 mg of the title compound as an oil.
¹H NMR (CDCl₃) δ 7.62 (d, 2H, J = 6 Hz), 7.22 (m, H), 7.06 (t, 1H, J = 7 Hz), 6.82 (d, 1H, J = 6 Hz), 6.70 (m, 2H), 4.06 (d, 1H, J = 2 Hz), 3.71 (s, 3H), 3.62 (d, 1H, J = 12 Hz), 3.44 (d, 1H, J = 12 Hz), 3.11 (m, 1H), 2.81 (m, 2H), 2.19 (s, 3H), 1.73 (m, 4H). Mass spectrum m/e 310 (parent).

### EXAMPLE 20

### Cis-2,4-Diphenyl-3-(2-methoxybenzylamino)piperidine

Under a nitrogen atmosphere in a round-bottom flask equipped with a reflux condenser were placed 21.1 g (89 mmol) of ethyl 4-nitro-3-phenylbutyrate (McMurray, J.E. et. al., Syn.Comm., 8, 53(1978)) and 90 mL of ethanol. To the system was added 9.04 mL (89 mmol) of benzaldehyde and 13.7 g (180 mmol) of ammonium acetate, and the reaction mixture was heated at 70°C overnight. The reaction mixture was cooled, a small volume of ethanol was added and the suspension was filtered. The collected solid was rinsed with a small volume of ethanol followed by ether to afford 22.7 g of 4,6-diphenyl-5-nitro-2-oxopiperidine.
¹H NMR (DMSO) δ 2.53 (dd, 1H, J = 6, 18), 2.82 (m, 1H), 3.88 (m, 1H), 4.80 (d, 1H, J = 8), 5.47 (t, 1H, J = 8), 7.3 (m, 10H). Mass spectrum m/e 296 (parent).

In a round-bottom flask were placed 15 g (50.6 mmol) of the nitro lactam 4,6-diphenyl-5-nitro-2-oxopiperidine and 85 mL of methylene chloride. Potassium tert-butoxide (5.72 g, 50.6 mmol) was added and the mixture was stirred for 15 min. To this system was added 85 mL of methanol. The mixture was stirred for 15 min and the system was cooled to -78°C. Ozone was bubbled thorugh the reaction mixture for 4 hours, nitrogen was bubbled through the mixture, 10 mL of dimethyl sulfide was added and nitrogen was bubbled through the mixture overnight. A mixture of water and methylene chloride was added to the system and the resulting solid (8.8 g of a mixture of the nitro lactam 4,6-diphenyl-5-nitro-2-oxopiperidine and 2,5-dioxo-4,6-diphenyl-piperidine was collected by suction filtration. The filtrate was concentrated with a rotary evaporator and the residue was partitioned between methylene chloride and water. The layers were separated, and the aqueous phase was extracted with two portions of methylene chloride. The combined organic fractions were dried (sodium sulfate) and concentrated to afford 5.14 g of crude 2,5-dioxo-4,6-diphenylpiperidine which was used immediately without further purification. Under a nitrogen atmosphere in a round-bottom flask were placed 2,5-dioxo-4,6-diphenylpiperidine (5.14 g, 19 mmol) and 75 mL of ethanol. A solution of 3.96 g (57 mmol) of hydroxylamine hydrocloride and 7.74 g (95 mmol) of sodium acetate in 25 mL of water were added and the reaction mixture was stirred at room temperature. The reaction mixture was concentrated to ca. 1/2 its initial volume, and the resulting precipitate was collected by suction filtration. This precipitate (1.5 g) was washed with saturated aqueous sodium bicarbonate, water and ether to afford 722 mg of 4,6-diphenyl-5-oximino-2-oxopiperidine as a white solid.
¹H NMR (DMSO) δ 2.52 (m, 2H), 2.76 (m, 1H), 4.12 (m, 1H), 5.80 (m, 1H), 7.30 (m, 10H), 8.24 (m, 1H). Mass spectrum m/z = 280 (parent).

To a solution of 4,6-diphenyl-5-oximino-2-oxopiperidine (700 mg, 2.5 mmol) was added ca. 2 g of wet Raney nickel which had been washed with water (until washings had a neutral pH) followed by ethanol, and the mixture was placed under an atmosphere of hydrogen (40 psi, Parr apparatus) overnight. The mixture was filtered through a pad of diatomaceous earth (Celite (trademark)), and the filter cake was rinsed well with ethanol. The filtrate was concentrated to afford 500 mg of 5-amino-4,6-diphenyl-2-oxopiperidine as a foam.
¹H NMR (CDCl₃) δ 2.96 (m, 4H), 4.12, 4.5 (m, 1H), 7.2 (m, 10H). Mass spectrum: m/z 266 (parent).

Under a nitrogen atmosphere in a round bottom flask were placed 500 mg (1.9 mmol) of 5-amino-4,6-diphenyl-2-oxopiperidine and 5 mL of methanol. To the system was added 1 g of 3 Å molecular sieves, and the pH of the mixture was adjusted to 4.5 using methanol saturated with hydrogen chloride. To this system was added 284 mg (2.1 mmol) of 2-methoxybenzaldehyde, and the mixture was stirred at room temperature overnight. The mixture was filtered through diatomaceous earth (Celite (trademark)), the filter cake was rinsed well with methanol and the filtrate was concentrated with a rotary evaporator. The residue was partitioned between saturated aqueous sodium bicarbonate and chloroform, the layers were separated and the aqueous phase was extracted with three portions of chloroform. The combined chloroform extracts were dried (sodium sulfate) and concentrated, and the residue was subjected to flash column chromatography (30 g of silica gel) using 3:97 methanol/chloroform as the eluant to obtain 115 mg of 4,6-diphenyl-5-(2-methoxybenxylamino)-2-oxopiperidine.
¹H NMR (CDCl₃) δ 2.36 (dd, 1H, J = 6, 18), 2.99 (m, 2H), 3.30 (m, 1H), 3.35 (s, 3H), 3.62 (d, 1H, J = 16), 3.74 (d, 1H, J = 16), 4.22 (m, 1H), 6.62 (d, 1H, J = 6), 6.80 (t, 1H, J = 6), 6.96 (m, 3H), 7.18 (m, 10H). Mass spectrum: m/z 386 (parent).

Under a nitrogen atmosphere in a round-bottom flask were placed 115 mg (0.3 mmol) of the amine 4,6-diphenyl-5-(2-methoxybenzylamino)-2-oxopiperidine and 5 mL of THF. To the system was added 0.74 mL (1.5 mmol) of 2.0 M borane-methyl sulfide complex in THF, and the reaction mixture was heated at 60°C overnight. The mixture was cooled to room temperature, and methanol was added carefully to the system. The mixture was stirred for 2 hours and concentrated with a rotary evaporator. To this system were added 83 mg (0.6 mmol) of potassium carbonate and ca. 3 mL of ethanol, and the mixture was heated at 85°C for 3 hours. The mixture was cooled to room temperature, concentrated, partitioned between methylene chloride and saturated aqueous sodium bicarbonate and extracted with three portions of methylene chloride. The combined methylene chloride fractions were dried (sodium sulfate) and concentrated to obtain 109 mg of an oil. This crude material was subjected to flash column chromatography (5 g of silica gel) using 1:19 methanol/chlorform as the eluant to afford 56 mg of the title compound. The hydrochloride salt of this material was prepared by treating a methylene chloride solution of the product with ether saturated with hydrogen chloride, concentrating, triturating with ether, scratching and repeating the concentration from ether. M.p. 176-178°C (HCl salt, dec).
¹H NMR (CDCl₃) δ 7.18 (m, 11H), 6.92 9 (d, 1H) = 6 Hz), 6.76 (t, 1H, J = 6 Hz), 6.61 (d, 1H, J = 6 Hz), 4.01 (d, 1H, J = 2 Hz), 3.66 (d, 1H, J = 12 Hz), 3.53 (d, 1H, J = 12 Hz), 3.38 (s, 3H), 3.30 (m, 1H), 3.12 (m, 3H), 2.12 (m, 2H). HRMS calc'd. for C₂₅H₂₈N₂O: 3.72.2202. Found: 372.2193.

The title compounds of Examples 21-26 have the following general formula 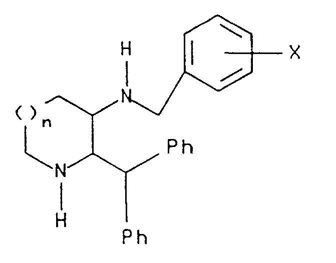
and were prepared by the following procedure.

### A. Methyl 4-hydroxy-5-nitro-6,6-diphenyl hexanoate

A solution of 2,2-diphenyl-nitroethane (42.6 gm, 187 mmole) and potassium tert. butoxide (3.15 gm, 28 mmole) was stirred into a mixture of tetrahydrofuran and tert. butanol (1.5:1, 320 mL) at -78°C, and methyl 3-formyl-propionate (24.0 gm, 206 mmole) was added. The reaction mixture was then allowed to warm to 10°C over a period of 1 hour, after which it was quenched with acetic acid (1.8 ml). The mixture was concentrated under vacuum, diluted with pH 7 buffer (400 ml), and extracted with methylene chloride (3 x 400 ml). The combined extracts were dried (magnesium sulfate), filtered and concentrated to afford an orange oil which on trituration with ether afforded methyl 4-hydroxy-5-nitro-6,6-diphenyl hexanoate (29.94 gm). The filtrate was concentrated and flash chromatographed. Elution with 10% ethyl acetate in hexane afforded an additional 20.66 gm of methyl 4-hydroxy-5-nitro-6,6-diphenyl hexanoate. Total yield (79%).
¹H NMR (CDCl₃) δ 7.2-7.4 (10H, m), 5.3 (1H, dd, J = 2.5, 12 Hz), 4.9 (1H, d, J = 12 Hz), 3.6 (3H, s), 2.6 (1H, m), 2.45 (2H, t, J = 7 Hz), 1.7-2.0 (1H, m), 1.6-1.7 (1H, m).

### B. 2-Oxo-5-hydroxy-6-benzhydrylpiperidine

To a stirred solution of methyl 4-hydroxy-5-nitro-6,6-diphenyl hexanoate (50.5 gm, 147 mmol) in ethanol (200 ml) at 25°C was added neutral Raney nickel (50 gms). The reaction mixture was shaken on a Parr shaker under hydrogen (30 psi). After 18 hours, the reaction mixture was filtered through diatomaceous earth (Celite (trademark)) which was thoroughly washed with ethanol (400 ml) and methylene chloride (600 ml). The organic phases were combined and concentrated under vacuum to a yellow oil (40.25 gms), which on trituration with cold ether afforded 2-oxo-5-hydroxy-6-benzhydryl piperidine (18.5 gm, m.p. 208°C, 45%). Evaporation of the mother liquor afforded an oily residue upon treatment with potassium tert. butoxide in tetrahydrofuran at room temperature for 6 hours. Extraction with methylene chloride and trituration with ether afforded an additional 2.55 gms of 2-oxo-5-hydroxy-6-benzhydryl-piperidine (overall yield 51%).
IR (neat max 3380, 1640 cm⁻¹)
¹H NMR (CDCl₃) δ 7.17-7.4 (10H, m), 5.49 (1H, bs), 4.18 (2H, s), 3.86 (1H, bs), 2.54-2.7 (1H, m), 2.3-2.42 (1H, m), 1.8-2.08 (2H, m).
HRMS Calc'd for C₁₈H₂₀N₂O: 282.1495. Found: 282.1495.

### C. 2,5-Dioxo-6-benzhydrylpiperidine

To a stirred solution of 2-oxo-5-hydroxy-6-benzhydryl piperidine (18.15 gm, 64.5 mmole) in acetone (150 ml) at -5°C was added Jones reagent (2.67 M, 94 mmole), and the reaction mixture was further stirred for 4 hrs. At the end of this period, the excess reactant was decomposed with 2-propanol and the solution concentrated under vacuum to half of its volume. The contents of the flask were then diluted with water (1000 ml) and extracted with methylene chloride (3 x 1000 ml). The combined organic phases were dried (anhyd. magnesium sulfate) and the methylene chloride was removed under vacuum to afford 2,5-dioxo-6-benzhydrylpiperidine (15.35 gm, 85%).
¹H NMR (CDCl₃) δ 7.18-7.4 (10H, m), 4.8 (1H, d, J = 4Hz), 4.7 (1H, dd, J = 4, 1.6 Hz), 2.38-2.6 (2H, m), 2.16-2.3 (2H, m), 1.9-2.01 (1H, m).

### D. 2-Oxo-5-oximino-6-benzhydrylpiperidine

To a stirred solution of 2,5-dioxo-6-benzhydrylpiperidine (15.35 gm, 55 mmole) in pyridine (150 ml) was added hydroxylamine hydrochloride (10.63 gm, 165 mmole) and the reaction mixture was stirred for 15 min. The reaction mixture was concentrated under vacuum, and the contents were poured into 1N HCl (250 ml). The aqueous phase was extracted with methylene chloride (2 x 300 ml) and dried (anhyd. magnesium sulfate). The methylene chloride was removed under vacuum to afford 2-oxo-5-oximino-6-benzhydrylpiperidine (10.62 gms, 65%).
¹H NMR (CDCl₃) δ 7.18-7.4 (10H, m), 5.96 (1H, bd), 5.59 (1H, bs), 4.8 (1H, m), 3.8 (1H, d, J = 10 Hz), 2.98-3.09 (1H, m), 2.05-2.42 (3H, m).

The title compounds of examples 21-26 were prepared from the title compound of "D" above by a procedure similar to that described in Examples 1(B) and 1(C).

### EXAMPLE 21

Cis-3-benzylamino-2-benzhydrylpiperidine (X=H, n = 1) M.p. 117°C. ¹H NMR (CDCl₃) δ 7.0-7.4 (15H, m), 4.39 (1H, d, J = 10 Hz), 3.76 (1H, d, J = 12 Hz), 3.4 (1H, d, J = 12 Hz), 3.28 (1H, d, J = 10 Hz), 2.94 (1H, m), 2.54 (1H, m), 2.54 (1H, m), 2.0 (2H, m), 1.7 (1H, m), 1.22 (1H, m). HRMS Calc'd for C₂₅H₂₈N₂: 356.2253. Found: 356.2256.

### EXAMPLE 22

Trans-3-benzylamino-2-benzhydrylpiperidine (X=H, n = 1) M.p. 186° (HCl salt). ¹H NMR (CDCL₃) δ 7.1-7.6 (15H, m), 4.57 (1H, d, J = 10 Hz), 3.82 (1H, d, J = 14 Hz), 3.65 (1H, d, J = 14 Hz), 3.46 (1H, bt), 2.9 (1H, m), 2.5 (3H, m), 2.05 (1H, m), 1.72 (1H, m), 1.45 (1H, m). HRMS Calcd for C₂₅H₂₈N₂: 356.2253. Found: 356.2269.

### EXAMPLE 23

Cis-3-(2-methoxybenzylamino)-2-benzhydrylpiperidine (x = 2-OMe, n = 1) M.p. 258°C (dec., HCl salt). ¹H NMR (CDCl₃) δ 6.7-7.4 (14H, m), 4.4 (1H, d, J = 10 Hz), 3.8 (3H, s), 3.75 (2H, dd, J = 12 Hz), 3.45 (1H, bd), 3.39 (1H, d, J = 10 Hz), 3.0 (1H, bd), 2.62 (2H, m), 2.08 (1H, m), 1.7 (1H, m), 1.4 (1H, m), 1.2 (1H, m). HRMS Calc'd for C₂₆H₃₀N₂O: 386.2358. Found: 386.2358.

### EXAMPLE 24

Trans-3-(2-methoxybenzylamino)-2-benzhydryl- piperidine (X = 2-OMe, n = 1) Oil. ¹H NMR (CDCl₃) δ 6.7-7.4 (14H, m), 4.55 (1H, d, J = 10 Hz), 3.8 (3H, s), 3.81 (1H, d, J = 14 Hz), 3.6 (1H, d, J = 14 Hz), 3.4 (1H, m), 2.9 (1H, m), 2.54 (2H, m), 2.0 (2H, m), 1.53 (1H, m), 1.45 (1H, m). HRMS Calcd for C₂₆H₃₀N₂O: 386.2358. Found: 386.2318.

### EXAMPLE 25

Cis-3-benzylamino-2-benzhydrylazepine (X=H, n = 2) M.p. 111-112°C. ¹H NMR (CDCl₃) δ 6.94-7.45 (15H, m), 4.33 (1H, d, J = 10 Hz), 3.52 (1H, d, J = 12 Hz), 3.34 (1H, d, J = 12 Hz), 3.21 (1H, dd, J = 2.1, 10 Hz), 3.16 (1H, bd), 2.4-2.58 (2H, m), 1.8 (1H, m), 1.56 (3H,m), 1.32 (2H, m).

### EXAMPLE 26

Trans-3-benzylamino-2-benzhydrylazepine (X=H, n = 2) M.p. 186-187°C (HCl salt). ¹H NMR (CDCl₃) δ 7.0-7.5 (15H, m), 3.88 (1H, d, J = 11 Hz), 3.45-3.6 (2H, m), 3.22 (1H, d, J = 12 Hz), 3.0 (1H, d, J = 12 Hz), 2.45-2.62 (2H, m), 1.75 (1H, m), 1.5 (2H, m), 1.08-1.25 (3H, m). HRMS Calcd for C₂₆H₃₁N₂: 371.2487. Found: 371.2495.

The title compounds of Examples 27-33 have the following general formula and were prepared by a procedure similar to that of Example 1.

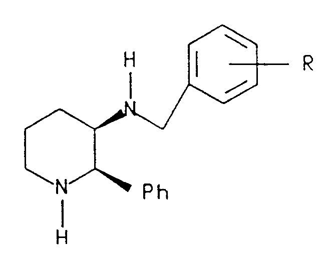

### EXAMPLE 27

Cis-3-benzylamino-2-phenylpiperidine (R = H). M.p. 250°C (HCl salt). ¹H NMR (CDCl₃) δ 6.94-7.0 (10H, m), 3.89 (1H, d, J = 2.3 Hz), 3.52 (1H, d, J = 13 Hz), 3.32 (1H, d, J = 13 Hz), 3.25 (1H, bd, J = 12 Hz), 2.88 (1H, d, J = 2.5 Hz), 2.78 (1H, dt, J = 12, 3 Hz), 2.4 (1H, d, J = 12 Hz), 1.8-1.98 (1H, m), 1.6 (1H, tt, J = 12, 2.5 Hz), 1.42 (1H, d, J = 12 Hz).

### EXAMPLE 28

### Cis-3-(2-fluorobenzylamino)-2-phenylpiperidine

(R = 2-F). M.p. > 260°C (dec., HCl salt). ¹H NMR (CDCl₃) δ 7.31-7.2 (5H, m), 7.15-7.07 (1H, m), 6.97-6.85 (3H, m), 3.88 (1H, d, J = 3 Hz), 3.64 (1H, d, J = 14 Hz), 3.50 (1H, d, J = 14 Hz), 3.36-3.2 (1H, m), 2.87-2.73 (2H, m), 2.07 (1H, bd, J = 13 Hz), 1.88 (1H, qt, J = 13, 4 Hz), 1.67-1.58 (1H, m), 1.43 (1H, bd, J = 13 Hz). ¹³C NMR (CDCl₃) δ 162.6, 159.4, 142.6, 130, 129.8, 128.2, 128, 127, 127.8, 127.6, 126.8, 126.4, 123.73, 123.7, 115, 114.7, 64.3, 55.5, 47.8, 44.5, 44.4, 29.1, 29.4. HRMS Calc'd for C₁₈H₂₁N₂F: 284.1689. Found: 284.1701.

### EXAMPLE 29

### Cis-3-(2,6-difluorobenzylamino)-2-phenylpiperidine

(R = 2,6-di F). M.p. > 260°C (dec., HCl salt). ¹H NMR (CDCl₃) δ 7.33-7.02 (6H, m), 6.7 (2H, t, J = 8 Hz), 3.86 (1H, d, J = 2 Hz), 3.63 (1H, d, J = 14 Hz), 3.52 (1H, d, J = 14 Hz), 3.24 (1H, bd, J = 10 Hz), 2.83-2.74 (1H, m), 2.09 (1H, bd, J = 13 Hz), 1.9 (1H, qt J = 14, 4 Hz), 1.63 (1H, tt, J = 14, 4 Hz), 1.4 (1H, bd, J = 12 Hz). ¹³C NMR (CDCl₃) δ 142.1, 128.4, 128.3, 126.7, 126, 111.1, 110.8, 110.7, 63.8, 55.2, 47.7, 38.5, 28.9, 20.4. HRMS Calc'd for : 302.1595. Found: 302.1607.

### EXAMPLE 30

### Cis-3-(2-methylbenzylamino)-2-phenylpiperidine

(R = 2-CH₃). M.p. 254°C. (dec., HCl salt). ¹H NMR (CDCl₃) δ 7.31-7.21 (4H, m), 7.09-6.96 (4H, m), 3.9 (1H, d, J = 2 Hz), 3.54 (1H, d, J = 14 Hz), 3.28 (1H, d, J = 14 Hz), 3.22-3.14 (1H, m), 2.91-2.87 (1H, m), 2.79 (1H, td, J = 8, 4 Hz), 2.14 (1H, bd, J = 9 Hz), 1.98 (3H, s), 1.97-1.75 (1H, m), 1.7-1.48 (3H, m). ¹³C NMR (CDCl₃) δ 142.7, 138.6, 136.4, 130, 128.4, 128.2, 126.7, 126.6, 125.5, 64.3, 56.2, 49.7, 29.3, 20.5, 18.5. HRMS Calc'd for C₁₉H₂₄N₂: 280.1939. Found: 280.1952.

### EXAMPLE 31

Cis-3-(2-trifluoromethylbenzylamino)-2-phenylpiperidine (R = 2-CF₃). M.p. 249°C (dec., HCl salt). ¹H NMR (CDCl₃) δ 7.49 (1H, d, J = 8 Hz), 7.49-7.16 (8H, m), 3.89 (1H, d, J = 2 Hz), 3.7 (1H, d, J = 15 Hz), 3.57 (1H, d, J = 15 Hz), 3.25 (1H, bd, J = 12 Hz), 2.86-2.74 (2H, m), 2.08 (1H, bd, J = 12 Hz), 1.93-1.8 (1H, m), 1.67-1.55 (2H, m), 1.44 (1H, bd, J - 14 Hz). ¹³C NMR (CDCl₃) δ 142.7, 139.8, 131.5, 129.7, 128.2, 126.8, 126.5, 126.2, 125.4, 125.4, 64.6, 56.2, 47.8, 47.0, 29, 20.5. HRMS Calc'd for C₁₉H₂₁N₂F₃: 334.1657. Found: 334.1665.

### EXAMPLE 32

Cis-3-(2-chlorobenzylamino)-2-phenylpiperidine (R = 2-Cl). M.p. 256°C (dec., HCl salt). ¹H NMR (CDCl₃) δ 7.31-6.97 (9H, m), 3.88 (1H, d, J = 2 Hz), 3.63 (1H, d, J = 15 Hz), 3.48 (1H, d, J = 15 Hz), 3.25 (1H, bd, J = 10 Hz), 2.87-2.74 (2H, m), 2.09 (1H, bd, J = 15 Hz), 1.9 (1H, qt, J = 13, 4 Hz), 1.68-1.57 (1H, m), 1.43 (1H, bd, J = 13 Hz). ¹³C NMR (CDCl₃) δ 142.5, 138.1, 133.6, 129.7, 129.1, 128.3, 127.7, 126.8, 126.4, 64.3, 55.6, 48.7, 47.8, 29, 20.4 HRMS Calc'd for C₁₈H₂₁N₂Cl: 300.1394. Found: 300.1394.

### EXAMPLE 33

Cis-3-(3-trifluoromethylbenzylamino)-2-phenylpiperidine (R = 3-CF₃). M.p. 240°C (dec., HCl salt). ¹H-NMR (CDCl₃) δ 7.41-7.14 (9H, m), 3.88 (1H, d, J = 2 Hz), 3.55 (1H, d, J = 14 Hz), 3.38 (1H, d, J = 14 Hz), 3.22 (1H, bd, J = 14 Hz), 2.84-2.74 (2H, m), 2.01 (1H, bd, J = 14 Hz), 1.85 (1H, qt, J = 12, 4 Hz), 1.63-1.54 (1H, m), 1.45 (1H, bd, J = 13 Hz). ¹³C NMR (CDCl₃) δ 142.8, 142.1, 131.1, 128.4, 128.3, 127, 126.4, 124.5, 123.3, 123.3, 64.5, 55.8, 51, 47.7, 29.4, 20.4. HRMS Calc'd for C₁₉H₂₁N₂F₃: 334.1657: Found: 334.1663.

The title compounds of examples 34-55 have the following general formula and were prepared by a procedure similar to that described in Example 1.

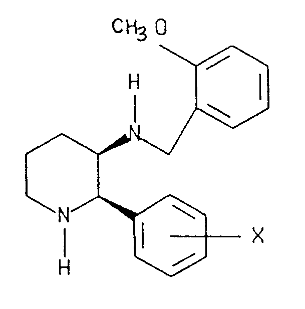

### EXAMPLE 34

Cis-3-(2-methoxybenzylamino)-2-(2-fluorophenyl)-piperidine (X = 2-F). M.p. 253°C (HCl salt). ¹H NMR (CDCl₃) δ 8.03 (1H, t, J = 7 Hz), 7.62-7.54 (1H, m), 7.47-7.35 (2H, m), 7.27-7.19 (2H, m), 6.94 (2H, dd, J = 9, 2 Hz), 5.25 (1H, d, J = 4 Hz), 5.25 (1H, d, J = 13 Hz), 4.03-4.00 (1H, m), 3.87 (1H, d, J = 13 Hz), 3.75 (3H, s), 3.67 (1H, bd, J = 13 Hz), 3.42-3.37 (2H, m), 2.6-2.42 (2H, m), 2.38-2.3 (1H, m), 2.08-1.96 (1H, m). HRMS Calcd for C₁₉H₂₃N₂OF: 314.1795. Found: 314.1778.

### EXAMPLE 35

Cis-3-(2-methoxybenzylamino)-2-(2-chlorophenyl)-piperidine (X = 2-Cl). M.p. 264°C (HCl salt). ¹H NMR (CDCl₃) δ 8.15 (1H, d, J = 6 Hz), 7.66-7.5 (1H, m), 7.39 (1H, t, J = 8 Hz), 7.15 (1H, d, J = 6 Hz), 6.94 (2H, t, J = 8 Hz), 5.21 (1H, d, J = 3 Hz), 4.19-4.1 (2H, m), 3.27 (1H, d, J = 12 Hz), 3.78 (3H, s), 3.76-3.64 (1H, m), 3.52-3.4 (1H, m), 2.64-2.44 (2H, m), 2.38-2.26 (1H, m), 2.16-1.96 (1H, m). HRMS Calc'd for C₁₉H₂₃N₂OCl: 330.1499. Found: 330.1412

### EXAMPLE 36

Cis-3-(2-methoxybenzylamino)-2-(2-methylphenyl)-piperidine (X = 2-CH₃). M.p. 260°C (HCl salt). ¹H NMR (CDCl₃) δ 7.97 (1H, bd, J - 8 Hz), 7.49-7.32 (4H, m), 7.08 (1H, d, J = 6 Hz), 6.95-6.88 (2H, m), 5.04 (1H, d, J = 3 Hz), 4.1 (1H, d, J = 14 Hz), 3.88-3.8 (2H, m), 3.68 (3H, s), 3.49-3.36 (1H, m), 2.59-2.27 (4H, m), 2.25 (3H, s), 2.0 (1H, bd, J = 10 Hz). HRMS Calc'd for C₂₀H₂₆N₂O: 310.2045. Found: 310.2080. C, 62.66; H, 7.36; N, 7.31. Found: C, 62.75; H, 7.46; N, 7.2.

### EXAMPLE 37

Cis-3-(2-methoxybenzylamino)-2-(3-trifluoromethylphenyl)-piperidine (X = 3-CF₃). M.p. 268°C (HCl salt). ¹H NMR (CDCl₃) δ 8.03-7.94 (2H, m), 7.84 (1H, d, J = 8 Hz), 7.77 (1H, t, J = 8 Hz), 7.37 (1H, t, J = 8 Hz), 7.16 (1H, d, J = 8 Hz), 6.93 (2H, t, J = 7 Hz), 5.05 (1H, d, J = 2 Hz), 4.14 (1H, d, J = 13 Hz), 3.86 (1H, d, J = 13 Hz), 3.72 (3H, s), 3.7-3.62 (1H, m), 3.3-3.2 (1H, m), 2.49-2.34 (2H, m), 2.3-2.18 (1H, m), 2.01 (1H, bd, J = 14 Hz).

### EXAMPLE 38

Cis-3-(2-methoxybenzylamino)-2-(3-fluorophenyl)-piperidine (X = 3-F). M.p. 264°C (HCl salt). ¹H NMR (CDCl₃) δ 7.62-7.5 (3H, m), 7.38 (1H, t, J = 7 Hz), 7.3-7.21 (2H, m), 6.93 (2H, t, J = 8 Hz), 5.03 (1H, d, J = 3 Hz), 4.16 (1H, d, J = 15 Hz), 4.06-3.96 (1H, m), 3.85 (1H, d, J = 13 Hz), 3.75 (3H, s), 3.66 (1H, bd, J = 12 Hz), 2.47-2.40 (2H, m), 2.30-2.15 (1H, m), 2.06-1.92 (1H, m). HRMS Calc'd for C₁₉H₂₃N₂OF: 314.1795. Found: 314.1790.

### EXAMPLE 39

Cis-3-(2-methoxybenzylamino)-2-(3-chlorophenyl)-piperidine (X = 3-Cl). M.p. 258-260°C (HCl salt). ¹H NMR (CDCl₃) δ 7.72 (1H, bs), 7.7-7.58 (1H, m), 7.54 (2H, d, J = 4 Hz), 7.4 (1H, t, J = 8 Hz), 7.2 (1H, d, J = 7 Hz), 6.97-6.92 (2H, m), 5.01 (1H, d, J = 4 Hz), 4.17 (1H, d, J = 13 Hz), 3.99 (1H, bs), 3.88 (1H, d, J = 13 Hz), 3.75 (3H, s), 3.69-3.54 (1H, m), 3.17-3.14 (1H, m), 2.49-2.4 (2H, m), 2.3-2.16 (1H, m), 2.05-1.94 (1H, m). HRMS Calc'd for C₁₉H₂₃N₂OCl: 330.1499. Found: 330.1508.

### EXAMPLE 40

Cis-3-(2-methoxybenzylamino)-2-(3-methoxyphenyl)-piperidine (X = 3-OMe). M.p. 252°C (HCl salt). ¹H NMR (CDCl₃) δ 7.49-7.34 (2H, m), 7.28-7.16 (3H, m), 7.07 (1H, d, J - 6 Hz), 6.96-6.91 (2H, m), 4.94 (1H, d, J = 4 Hz), 4.15 (1H, d, J = 13 Hz), 3.96 (1H, bs), 3.86 (1H, d, J = 13 Hz), 3.83 (3H, s), 3.69 (3H, s), 3.68-3.6 (1H, m), 3.28-3.22 (1H, m), 2.49-2.35 (2H, m), 2.32-2.16 (1H, m), 2.06-1.94 (1H, m). HRMS Calc'd for C₂₀H₂₆N₂O₂: 326.1994. Found: 326.1983. C, 60.15; H, 7.07; N, 7.01. Found: C, 59.78; H, 6.75; N, 7.01.

### EXAMPLE 41

Cis-3-(2-methoxybenzylamino)-2-(3-methylphenyl)-piperidine (X = 3-CH₃). M.p. 243°C (HCl salt). ¹H NMR (CDCl₃) δ 7.15 (2H, dd, J = 8.7 Hz), 7.07-6.94 (4H, m), 6.79 (1H, t, J = 7 Hz), 6.67 (1H, d, J = 8 Hz), 3.83 (1H, d, J = 2 Hz), 3.68 (1H, d, J = 14 Hz), 3.44 (3H,s), 3.4 (1H, d, J = 14 Hz), 3.26 (1H, bd, J = 12 Hz), 2.85-2.73 (1H, m), 2.3 (3H, s), 2.12 (1H, bd, J = 14 Hz), 1.92 (1H, qt, J = 13, 4 Hz), 1.58 (1H, tt, J = 14 Hz), 1.38 (1H, bd, J = 13 Hz). HRMS Calc'd for C₂₀H₂₆N₂O: 310.2045. Found: 310.2069. C, 62.66; H, 7.36; N, 7.31. Found: C, 62.61; H, 7.44; N, 7.24.

### EXAMPLE 42

Cis-3-(2-methoxybenzylamino)-2-(4-phenylphenyl)-piperidine (X = 4-Ph). M.p. 255°C (HCl salt). ¹H NMR (CDCl₃) δ 7.77-7.7 (4H, m), 7.63-7.44 (3H, m), 7.41 (2H, t, J = 2 Hz), 7.39-7.31 (2H, m), 7.15 (1H, dd, J = 6, 2 Hz), 6.92 (1H, t, J = 7 Hz), 6.79 (1H, d, J = 8 Hz), 5.03 (1H, bs), 4.13 (1H, d, J = 13 Hz), 3.87 (2H, d, J = 13 Hz), 3.6 (4H, s), 3.34-3.3 (2H, bs), 2.58-2.1 (3H, m), 2.00-1.89 (1H, m). HRMS Calc'd for C₂₅H₂₈N₂O: 372.2202. Found: 372.2220.

### EXAMPLE 43

Cis-3-(2-methoxybenzylamino)-2-(4-fluorophenyl)-piperidine (X = 4-F). M.p. 252°C (HCl salt). IR (KBr) max 3280, 2600, 1605, 1520, 1240, 1020 cm⁻¹. ¹H NMR (CDCl₃) δ 7.25-7.12 (3H, m), 6.99-6.94 (3H,m), 6.8 (1H, t, J = 6 Hz), 6.68 (1H, d, J = 8 Hz), 3.83 (1H, bs), 3.67 (1H, d, J = 14 Hz), 3.49 (3H, s), 3.38 (1H, d, J = 14 Hz),3.26-3.2 (1H, m), 2.82-2.71 (2H, m), 2.11 (1H, bd, J = 13 Hz), 1.97 -1.83 (1H, m), 1.63-1.52 (1H, m), 1.38 (1H, bd, J = 13 Hz). ¹³C NMR (CDCl₃) δ 157.6, 138.3, 129.6, 128.3, 127.9, 127.8, 120, 114.9, 114.6, 109.8, 63.4, 54.8, 54.6, 47.8, 46.7,28.2, 20.3. HRMS Calc'd. for C₁₉H₂₃N₂OF: 314.1795. Found: 314.1802.

### EXAMPLE 44

Cis-3-(2-methoxybenzylamino)-2-(4-methylphenyl)-piperidine (X = 4-CH₃). M.p. 233°C (HCl salt). IR (KBr) max 3400, 2700, 1610, 1570, 1460, 1260, 1040 cm⁻¹. ¹H NMR (CDCl₃) δ 7.18-7.11 (5H, m) 6.97 (1H, dd, J = 7, 2 Hz), 6.79 (1H, t, J = 8 Hz), 6.67 (1H, d, J = 8 Hz), 3.84 (1H, d, J = 2 Hz), 3.67 (1H, d, J = 14 Hz), 3.45 (3H, s), 3.4 (1H, d, J= 14 Hz), 3.25 (1H, bd, J = 8 Hz), 2.82-2.73 (2H, m), 2.31 (3H, s), 2.11 (1H, bd, J = 13 Hz), 1.91 (1H, qt, J = 9, 4 Hz), 1.57 (1H, tt, J = 14, 4 Hz), 1.37 (1H, bd, J = 13 Hz). ¹³C NMR (CDCl₃) δ 157.6, 139.4, 135.9, 129.6, 128.8, 128.4, 127.7, 126.2, 120, 109.8, 63.7, 54.8, 54.7, 47.8, 46.7, 28.2, 21.0, 20.4. HRMS Calcd for C₂₀H₂₆N₂O: 310.2045. Found: 310.2043.

### EXAMPLE 45

Cis-3-(2-methoxybenzylamino)-2-(4-chlorophenyl)-piperidine (X = 4-Cl). M.p. 247°C (HCl salt). IR (KBr) max 2950, 2640, 1610, 1570, 1500, 1450, 1250 cm⁻¹. ¹H NMR (CDCl₃) δ 7.26-7.13 (5H, m), 6.97 (1H, dd, J = 7, 2 Hz), 6.81 (1H, t, J = 8 Hz), 6.68 (1H, d, J = 8 Hz), 3.84 (1H, d, J = Hz), 3.7 (1H, d, J = 14 Hz), 3.48 (3H, s), 3.37 (1H, d, J = 14 Hz), 3.26 (1H, bd, J = 8 Hz), 2.83-2.72 (2H, m), 2.12 (1H, bd, J = 9 Hz), 1.91 (1H, qt, J = 13, 4 Hz), 1.58 (1H, tt, J = 13, 4 Hz), 3.83 (1H, bd, J = 13 Hz). ¹³C NMR (CDCl₃) δ 157.6, 140.6, 132.4, 129.7, 128.2, 128, 127.7, 120, 109.9, 63.3, 54.8, 54.5, 47.7, 46.8, 28, 20. HRMS Calc'd for C₁₉H₂₃N₂OCl: 330.1498. Found: 330.1489. C, 56.52; H, 6.24; N, 6.94. Found: C, 56.52; H, 6.2; N, 6.86.

### EXAMPLE 46

Cis-3-(2-methoxybenzylamino)-2-(4-methoxyphenyl)-piperidine (X = 4-OMe). M.p. 245°C (HCl salt). ¹H NMR (CDCl₃) δ 7.14 (3H, t, J = 8 Hz), 6.97 (1H, dd, J = 7, 2 Hz), 6.84-6.77 (3H, m), 6.67 (1H, d, J = 8 Hz), 3.81 (1H, d, J = 2 Hz), 3.78 (1H, s), 3.67 (1H, d, J = 14 Hz), 3.47 (3H, s), 3.4 (1H, d, J = 14 Hz), 3.24 (1H, bd, J = 10 Hz), 2.81-2.72 (2H, m), 2.1 (1H, bd, J = 14 Hz), 1.9 (1H, qt, J = 14, 4 Hz), 1.56 (1H, tt, J = 14, 4 Hz), 1.36 (1H, bd, J = 14 Hz). ¹³C NMR (CDCl₃) δ 158.3, 157.6, 134.6, 129.6, 128.4, 127.7, 127.3, 120, 113.5, 109.8 63.4, 55.2, 54.8, 54.7, 47.8, 46.7, 28.2, 20.3. HRMS Calc'd for C₂₀H₂₆N₂O₂: 326.1996. Found: 326.1968. C, 60.15, H, 7.07; N, 7.01. Found: C, 59.36; H, 6.79; N, 6.82.

### EXAMPLE 47

Cis-3-(2-methoxybenzylamino)-2-(4-trifluoromethylphenyl)-piperidine (X = 4-CF₃). M.p. 250°C (dec., HCl salt). ¹H NMR (CDCl₃) δ 7.51 (2H, d, J = 8 Hz), 7.33 (2H, d, J = 8 Hz), 7.13 (1H, t, J = 8 Hz), 6.93 (1H, d, J = 8 Hz), 6.77 (1H, t, J = 8 Hz), 6.63 (1H, d, J = 8 Hz), 3.89 (1H, s), 3.67 (1H, d, J = 14 Hz), 3.39 (3H, s), 3.33 (1H, d, J = 14 Hz), 3.24 (1H, bd, J = 12 Hz), 2.82-2.74 (2H, m), 2.13 (1H, bd, J = 14 Hz), 1.98-1.78 (1H, m), 1.64-1.46 (1H, m), 1.38 (1H, bd, J = 14 Hz). ¹³C NMR (CDCl₃) δ 157.4, 146.5, 129.5, 127.8, 126.5, 124.8, 124.7, 119.8, 109.7, 63.6, 54.4, 54.3, 47.5, 46.6, 28, 10. HRMS Calcd for C₂₀H₂₃N₂OF₃: 364.1762. Found: 364.1710.

### EXAMPLE 48

Cis-3-(2-methoxybenzylamino)-2-(4-bromophenyl)-piperidine (X = 4-Br). M.p. 250°C (HCl salt). ¹H NMR (CDCl₃) δ 7.36 (2H, d, J = 8 Hz), 7.14-7.05 (3H, m), 6.95 (1H, dd, J = 8, 2 Hz), 6.79 (1H, t, J = 8 Hz), 6.67 (1H, d, J = 8 Hz), 3.79 (1H, d, J = 2 Hz), 3.66 (1H, d, J = 14 Hz), 3.48 (3H, s), 3.34 (1H, d, J = 14 Hz), 3.22 (1H, bd, J = 14 Hz), 2.78-2.68 (2H, m), 2.17 (1H, bd, J = 14 Hz), 1.96-1.78 (1H, m), 1.56 (1H, tt, J = 14, 4 Hz), 1.38 (1H, bd, J = 14 Hz). ¹³C NMR (CDCl₃) δ 157.6, 141.4, 131.1, 129.7, 128.1, 128, 127.9, 120.4, 120, 109.8, 63.4, 54.8, 54.4, 47.6, 46.8, 28.1, 20.2. HRMS Calc'd for C₁₉H₂₃N₂OBr: 374.0980. Found: 374.0926. C, 50.91; H, 5.62; N, 6.25. Found: C, 51.41; H, 5.48; N, 6.23.

### EXAMPLE 49

Cis-3-(2-methoxybenzylamino)-2-(4-hydroxymethylphenyl)-piperidine (X = 4-CH₂OH). M.p. 248°C (HCl salt). ¹H NMR (CDCl₃) δ 7.2-7.04 (5H, m), 6.95 (1H, dd, J = 8, 2 Hz), 6.7 (1H, t, J = 8 Hz), 6.64 (1H, d, J = 8 Hz), 4.6 (2H, s), 3.82 (1H, d, J = 2 Hz), 3.62 (1H, d, J = 14 Hz), 3.43 (3H, s), 3.37 (1H, d, J = 14 Hz), 3.24 (1H, bd, J = 8 Hz), 2.8-2.68 (2H, m), 1.96-1.8 (1H, m), 1.56 (1H, tt, J = 14, 4 Hz), 1.36 (1H, bd, J = 8 Hz). HRMS Calc'd for C₂₀H₂₆N₂O₂: 326.1994. Found: 326.1979. C, 60.15; H, 7.07; N, 7.02. Found: C, 60.04; H, 6.93; H, 6.83.

### EXAMPLE 50

Cis-3-(2-methoxybenzylamino)-2-(3-fluoro-4-methoxyphenyl)-piperidine (X = 3-F, 4-OMe). M.p. 250°C (dec., HCl salt). ¹H NMR (CDCl₃) δ 7.15 (1H, dt, J = 8, 2 Hz), 7.01-6.93 (3H, m), 6.89-6.78 (2H, m), 6.7 (1H, d, J = 8 Hz), 3.87 (3H, s), 3.78 (1H, d, J = 2 Hz), 3.68 (1H, d, J = 14 Hz), 3.52 (3H, s), 3.38 (1H, d, J = 14 Hz), 3.22 (1H, bd, J = 9 Hz), 2.75 (2H, td, J = 13, 3 Hz), 2.1 (1H, bd, J = 13 Hz), 1.86 (1H, qt, J = 13, 4 Hz), 1.56 (1H, tt, J = 13, 3 Hz), 1.35 (1H, bd, J = 13 Hz). ¹³C NMR (CDCl₃) δ 157.6, 135.8, 129.7, 128.2, 128, 121.8, 121.7, 120, 114.3, 114.1, 113, 109.8, 63, 56.3, 54.7, 54.5, 47.7, 46.8, 28.2, 20.3

### EXAMPLE 51 Cis-3-(2-methoxybenzylamino)-2-(2,3-difluoro

phenyl)-piperidine (X = 2,3-diF). M.p. 243°C (HCl salt). ¹H NMR (CDCl₃) δ 7.21-7.12 (2H, m), 7.09-7.01 (1H, m), 6.98 (1H, dd, J = 7.2 Hz), 6.81 (1H, t, J = 7 Hz), 6.69 (1H, d, J = 8 Hz), 4.17 (1H, s), 3.61 (1H, d, J = 14 Hz), 3.54 (3H, s), 3.36 (1H, d, J = 14 Hz), 3.23 (1H, d, J = 14 Hz), 2.89 (1H, bs), 2.79 (1H, td, J = 12, 3 Hz), 2.03 (1H, bd, J = 13 Hz), 1.85 (1H, qt, J = 13, 4 Hz), 1.68-1.56 (1H, m), 1.41 (1H, bd, J = 14 Hz). ¹³C NMR (CDCl₃) δ 157.5, 132.6, 132.4, 129.5, 128.3, 127.9, 123.6, 122.8, 120.2, 115.3, 115.1, 109.9, 58.3, 54.8, 53.2, 47.1, 28.6, 20.4.

### EXAMPLE 52

Cis-3-(2-methoxybenzylamino)-2-(2,3-dichlorophenyl)-piperidine (X = 2,3-diCl). M.p. 249°C (HCl salt). ¹H NMR (CDCl₃) δ 7.42 (1H, d, J = 8 Hz), 7.35 (1H, d, J = 8 Hz), 7.19 (1H, t, J = 8 Hz), 7.14 (1H, t, J = 8 Hz), 6.91 (1H, d, J = 8 Hz), 6.79 (1H, t, J = 8 Hz), 6.68 (1H, d, J = 8 Hz), 4.19 (1H, d, J = 2 Hz), 3.55 (1H, d, J = 12 Hz), 3.53 (3H, s), 3.32 (1H, d, J = 14 Hz), 3.23 (1H, bd, J = 12 Hz), 3.03-2.98 (1H, m), 2.81 (1H, td, J = 13, 3 Hz), 2.01 (1H, bd, J = 13 Hz), 1.97-1.75 (1H, m), 1.7-1.62 (1H, m), 1.42 (1H, bd, J = 12 Hz).

### EXAMPLE 53

Cis-3-(2-methoxybenzylamino)-2-(4-ethylaminophenyl)-piperidine (X = 4-NEt). M.p. 241°C (HCl salt). ¹H NMR (CDCl₃) δ 7.14 (1H, t, J = 8 Hz), 7.08-6.94 (3H, m), 6.78 (1H, t, J = 8 Hz), 6.67 (1H, d, J = 8 Hz), 6.52 (2H, d, J = 8 Hz), 3.77 (1H, bs), 3.69 (1H, d, J = 14 Hz), 3.5 (3H, s), 3.43 (1H, d, J = 14 Hz), 3.33 (1H, bd, J = 2 Hz), 3.12 (1H, q, J = 8 Hz), 2.84-2.68 (1H, m), 2.09 (1H, bd, J = 4 Hz), 1.96-1.49 (1H, m), 1.61-1.49 (1H, m), 1.35 (1H, bd, J = 14 Hz), 1.25 (3H, t, J = 8 Hz).

### EXAMPLE 54

Cis-3-(2-methoxybenzylamino)-2-(3-methyl-4-methoxyphenyl)-piperidine (X = 3-Me, 4-OMe). M.p. 248°C (HCl salt). IR (KBr) max 3540, 2600, 1610, 1560, 1460, 1270, 1030 cm⁻¹. ¹H NMR (CDCl₃) δ 7.13 (1H, t, J = 8 Hz), 7.02 (1H, d, J = 8 Hz), 6.94-6.9 (2H, m), 6.74 (1H, t, J = 8 Hz), 6.7 (1H, d, J = 8 Hz), 6.64 (1H, d, J = 8 Hz), 3.79 (3H, s), 3.78 (1H, s), 3.67 (1H, d, J = 1 Hz), 3.43 (3H, s), 3.38 (1H, d, J= 14 Hz), 3.21 (1H, bd, J = 14Hz), 2.14 (3H, s), 2.11-2.07 (1H, m), 1.93-1.74 (1H, m), 1.59-1.53 (1H, m), 1.38-1.33 (1H, m). ¹³C NMR (CDCl₃) δ 157.6, 156.5, 134.1, 129.6, 128.6, 128.4, 127.7, 126.1, 124.4, 119.9, 109.7, 109.6, 63.3, 55.4, 54.7, 53.4, 47.8, 46.6, 28.1, 20.4, 16.3. HRMS Calc'd for C₂₁H₂₈N₂O₂: 340.2151. Found: 340.2172.

### EXAMPLE 55

Cis-3-(2-methoxybenzylamino)-2-(2-fluoro-6-chlorophenyl)-piperidine (X = 2-F, 6-Cl). M.p. 245-246°C (HCl salt). IR (KBr) max 3280, 2700, 1610, 1580, 1500, 1450, 1260, 1010 cm⁻¹. ¹H-NMR (CDCl₃) δ 7.16-7.1 (3H, m), 6.99-6.82 (2H, m), 6.79 (1H, t, J = 8 Hz), 6.68 (1H, d, J = 8 Hz), 4.37 (1H, d, J = 2 Hz), 3.68 (1H, d, J = 14 Hz), 3.55 (1H, s), 3.47 (1H, d, J = 14 Hz), 3.2 (1H, bd, J = 14 Hz), 2.87-2.78 (1H, m), 2.7 (1H, t, J = 14 Hz), 2.4-2.0 (1H, m), 1.84-1.6 (2H, m), 1.36 (1H, bd, J = 14 Hz). ¹³C NMR (CDCl₃) δ 157.4, 129.3, 128.3, 128.2, 127,8, 125.7, 125.6, 120.3, 115.4, 115, 109.9, 62.8, 62.8, 54.9, 53, 47.9, 47.3, 28.6, 20.8. HRMS Calc'd for C₁₉H₂₂N₂OClF: 348.1405. Found: 348.1369.

The title compounds of Examples 56-60 have the following general formula and were prepared by a procedure similar to that described in Example 1.

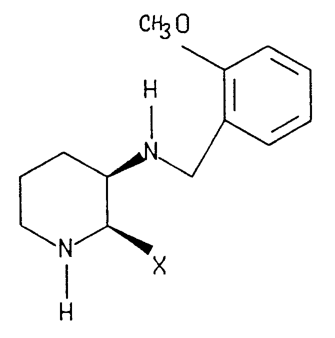

### EXAMPLE 56

### 3-(2-Methoxybenzylamino)-piperidine (X = H).

M.p. 198°C (HCl salt). ¹H NMR (HCl salt CD₃OH) δ 7.48 (2H, t, J = 6 Hz), 7.12 (1H, d, J = 6 Hz), 7.04 (1H, t, J = 8 Hz), 4.33 (2H, a, J = 4 Hz), 3.95 (3H, s), 3.8 (1H, bd, J = 9 Hz), 3.7-3.54 (1H, m), 3.41 (1H, bd, J = 9 Hz), 3.25 (1H, t, J = 12 Hz), 3.18-3.01 (1H, m), 2.48-2.4 (1H, m), 2.24-2.1 (1H, m), 2.01-1.79 (1H, m). HRMS Calc'd for C₁₃H₂₂N₂O: 220.1576. Found: 220.1587.

### EXAMPLE 57

Cis-3-(2-methoxybenzylamino-)-2-(5-indanyl)- piperidine (X = 5-indane). M.p. 243°C (HCl salt). ¹HNMR (CDCl₃) δ 7.24-7.11 (3H, m), 6.97 (2H, t, J = 8 Hz), 6.79 (1H, t, J = 8 Hz), 6.65 (1H, d, J = 8 Hz), 3.83 (1H, bs), 3.68 (1H, d, J = 14 Hz), 3.43 (3H, s), 3.39 (1H, d, J = 14 Hz), 2.23 (1H, bd, J = 14 Hz), 2.88-2.72 (6H, m), 2.13-1.86 (5H,m), 1.56 (1H, tt, J = 13, 4 Hz), 1.37 (1H, bd, J = 14 Hz).

### EXAMPLE 58

Cis-3-(2-methoxybenzylamno)-2-(1-naphthyl)- piperidine (X = 1-naphthyl). M.p. 251°C (HCl salt). ¹HNMR (HCl salt, CD₃OH) δ 8.16 (1H, d, J = 6 Hz), 8.08 (1H, d, J = 7 Hz), 8.04-7.98 (1H, m), 7.94-7.86 (1H, m), 7.71 (1H, t, J = 8 Hz), 7.64-7.61 (¹H, m), 7.17 (1H, t, J = 8 Hz), 6.84 (1H, d, J = 6 Hz), 6.66 (2H, t, J = 8 Hz), 5.73 (1H, bs), 4.06-3.99 (1H, m), 3.8-3.74 (2H, m), 3.49-3.4 (4H, m), 2.72-2.44 (3H, m), 6.84 (1H, bd, J = 8 Hz). HRMS Calc'd for C₂₃H₂₆N₂O: 346.2045. Found: 346.2062.

### EXAMPLE 59

Cis-3-(2-methoxybenzylamino)-2-(2-naphthyl)- piperidine (X = 2-naphthyl). M.p. > 250°C (dec., HCl salt). ¹H NMR (CDCl₃) δ 7.87-7.78 (3H,m), 7.69 (1H, d, J = 8 Hz), 7.5-7.39 (2H, m), 7.14 (1H, d, J = 8 Hz), 7.1 (1H, t, J = 8 Hz), 6.92 (1H, d, J = 8 Hz), 6.75 (1H t, J = 8 Hz), 6.47 (1H, d, J = 8 Hz), 4.02 (1H, d, J = 2 Hz), 3.66 (1H, d, J = 14 Hz), 3.37-3.2 (2H, m), 2.97 (3H, s), 2.89 (1H, bs), 2.88-2.79 (1H, m), 2.16 (1H, bd, J = 14 Hz), 1.98 (1H, qt, J = 8, 3 Hz), 1.63 (1H, tt, J = 4, 12 Hz), 1.43 (1H, bd, J = 13 Hz).

### EXAMPLE 60

Cis-3-(2-methoxybenzylamino)-2-cyclopentyl- piperidine (X = cyclopentyl). M.p. 161°C (HCl salt). IR (KBr) ν max 3480, 3420, 2960, 1610, 1500, 1260, 1020 cm⁻¹. ¹HNMR (CDCl₃) δ 7.48 (1H, d, J = 8 Hz), 7.17 (1H, t, J = 8 Hz), 6.9 (1H, t, J = 8 Hz), 6.8 (1H, d, J = 8 Hz), 3.78 (3H, s), 3.67 (1H, d, J = 13 Hz), 3.57 (1H, d, J = 13 Hz), 2.97 (1H, bd, J = 13 Hz), 2.69-2.64 (2H, m), 2.47 (1H, t, J = 9 Hz), 2.3-2.2 (2H, m), 1.75 (1H, bd, J = 9 Hz), 1.6-1.16 (7H, m), 1.0-0.9 (1H, m). ¹³C NMR (CDCl₃) δ 157.9, 130.6, 128.5, 127.5, 120.2, 110, 61.3, 59.2, 55.1, 47.9, 47.2, 39, 29.2, 27.3, 26.2, 25.8, 24.1, 23.1. HRMS Calc'd for C₁₈H₂₈N₂O: 288.2201. Found: 288.2172.

The title compounds of Example 61-62 have the following general formula and were prepared by a procedure similar to that described in Example 1.

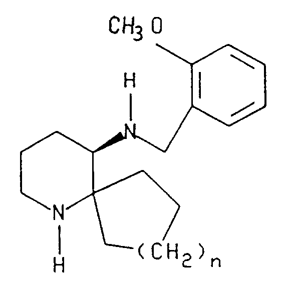

### EXAMPLE 61

5-(2-Methoxybenzylamino)-1-aza-spiro[5.5]undecane (n = 2) M.p. 257°C (HCl salt). IR (KBr) ν max 2940, 1605, 1580, 1500, 1460, 1250, 1020 cm⁻¹. ¹H NMR (CDCl₃) δ 7.27-7.18 (2H, m), 6.89 (1H, t, J = 8 Hz), 6.84 (1H, d, J = 8 Hz), 3.86 (1H, d, J = 14 Hz), 3.82 (3H, s), 3.68 (1H, d, J = 14 Hz), 2.74-2.68 (2H, m), 2.25-2.08 (1H, m), 1.81-1.25 (13H, m). HRMS Calc'd for C₁₈H₂₈N₂O: 288.2202. Found 288.2182.

### EXAMPLE 62

10-(2-methoxybenzylamino)-6-aza-spiro[4.5]decane (n = 1). M.p. 247°C (HCl salt). IR (KBr) max 2960, 2700, 1605, 1580, 1500, 1480, 1260, 1030 cm⁻¹. ¹H NMR (CDCl₃) δ 7.23-7.18 (2H, m), 6.89 (1H, t, J = 8 Hz), 6.84 (1H, d, J = 8 Hz), 3.89 (1H, d, J = 14 Hz), 3.83 (3H, s), 3.66 (1H, d, J = 14 Hz), 2.76-2.7 (2H, m), 2.31 (1H, dd, J = 8, 3 Hz), 1.81-1.24 (12H, m). HRMS Calc'd for C₁₇H₂₆N₂O; 274.2085. Found: 274.2057.

### EXAMPLE 63

### Cis-3-(2-methoxybenzylamino)-2-phenylpiperidne

### A. 4-Phenyl-1-(tert.-butyldimethylsilyl)azetidin-2-one

4-Phenylazetidin-2-one (10.4 gm, 71.0 mmole) (Graf, Chem. Ber, 111 (1963); Durst et al. J. Org. Chem., 35, 2043 (1970)) was dissolved in DMF (200 ml) and was treated with tert.-butyldimethylsilyl chloride (12.8 gm, 85 mmol) and triethylamine (11.8 ml, 85 mmol). The mixture was stirred at room temperature for 16 hrs. and taken up in ether (500 ml). The ethereal solution was washed with 1 N hydrochloric acid (1 x 100 ml), water (2 x 50 ml) and brine (1 x 50 ml). After the solution was dried (anhyd. magnesium sulfate) and evaporated, the residue was flash chromatographed on SiO₂-gel column. Elution with 15% ethyl acetate in hexane afforded 4-phenyl-1-(tert.-butyldimethylsilyl)azetidin-2-one (18.4 gm, 99%) as an oil which solidified on standing.
¹H NMR (CDCl₃) δ 7.37-7.29 (5H, m), 4.51(1H, dd, J = 6, 3 Hz), 3.5 (1H, dd, J = 16, 6 Hz), 2.93 (1H, dd, J = 16, 3 Hz), 0.9 (3H, s), 0.89 (9H, s), 0.19 (3H, s).

### B. 3-(3'-Chloropropyl)-4-phenyl-1-(tert.-butyldimethylsilyl)azetidin-2-one

To a stirred solution of 4-phenyl-1-(tert.-butyldimethylsilyl)azetidin-2-one (9.75 gm, 37 mmoles) in THF (100 ml) at -50°C, a freshly prepared solution of lithium diethylamide (1M in THF, 44 ml, 45 mmole) was added rapidly under nitrogen. The reaction mixture was stirred further for 15 min. at -50C and then a solution of 1-bromo-3-chloropropane (7.4 ml, 75 mmole) in THF (20 ml) was added. The resulting mixture was stirred for 15 min. at -50°C, after which ammonium chloride (saturated aqueous solution) was added. After the mixture was taken up in ether (2 x 300 ml), it was washed with saturated aqueous sodium chloride. The ether solution was dried (magnesium sulfate) and concentrated, and the residue (17.0 gm) was chromatographed on a silicon dioxide-gel column. Elution with 5% ethyl acetate in hexane afforded 3-(3'-chloropropyl)-4-phenyl-1-(tert.-butyldimethylsilyl)a zetidin-1-one as an oil (7.6 gms, 58%).
¹H NMR (CDCl₃) δ 7.2-7.4 (1H, m), 4.18 (1H, d, J = 2.5 Hz), 3.5 (2H, t, J = 5 Hz), 3.04 (1H, dt, J = 2.5, 7.5 Hz), 1.7-2.05 (4H, m), 0.9 (9H, s ), 0.2 (3H, s).

### C. Cis-Methyl-2-phenylpiperidine-3-carboxylate

3-(3'-Chloropropyl)-4-phenyl-1-(tert.-butyldimethylsilyl)azetidin-2-one (3.07 gm, 9.0 mmole) was dissolved in 10% methanolic sulfuric acid and refluxed for 16 hours. At the end of this period, the reaction mixture was cooled, the sulfuric acid was neutralized with sodium bicarbonate and the mixture was taken up in ether (2 x 200 ml). The ethereal solution was washed with water (2 x 50 ml) and dried (anhyd. magnesium sulfate). Evaporation afforded essentially pure 5-chloro-2-carbomethoxy-1-phenylpent-1-ylamine as an oil (2.11 gms). The 5-chloro-2-carbomethoxy- 1-phenylpent-1-ylamine thus obtained was dissolved in the dimethylformamide ("DMF") (20 ml) and sodium iodide (2.11 gm) and sodium bicarbonate (2.11 gm) were added. The resulting mixture was refluxed for 15 min. At the end of this period, the reaction mixture was cooled and taken up in ether (200 ml). The ethereal solution was washed with water (2 x 50 ml) and dried (anhyd. magnesium sulfate). Evaporation of the ether afforded chromatographically pure cis-methyl 2-phenylpiperidine-3-carboxylate as an oil (1.54 gm, 78%).
¹H NMR (CDCl₃) δ 7.31-7.5 (5H, m), 3.95 (1H, d, J = 3.5 Hz), 3.42 (3H, s), 3.39-3.30 (1H, m), 3.01-2.93 (1H, m), 2.84-2.74 (1H, m), 2.22-2.11 (1H, m), 1.90-1.66 (3H, m), 1.53-1.46 (1H, m).

### D. Cis-methyl 2-phenylpiperidine-1-(benzyloxycarbonyl)-3-carboxylate

Cis-methyl-2-phenylpiperidine-3-carboxylate (1.54 gm, 7.0 mmole), triethylamine (1.5 ml, 11.0 mmole) and benzyl chloroformate (1.5 ml, 11.0 mmole) were mixed in methylene chloride (45 ml) at 25C and stirred for 15 hours. At the end of this period, the reaction mixture was taken up in ether (100 ml), washed with water (2 x 50 ml) and dried (anhyd. magnesium sulfate). The solvent was removed under reduced pressure to afford a residue which was chromatographed on a flash SiO₂-gel column. Elution with 1:1 ethyl acetate/hexane afforded cis-methyl 2-phenylpiperidine-1-(benzyloxycarbonyl)-3-carboyxlate as an oil (1.91 gm, 77%).
¹H NMR (CDCl₃) δ 7.34-7.12 (10H, m), 5.97 (1H, bd), 5.30-5.1 (1H, m), 5.17 (1H, s), 4.15-3.90 (1H, m), 3.59 (3H, s), 2.98-2.91 (1H, m), 2.75 (1H, bt, J = 12 Hz), 2.14-2.00 (2H, m), 1.85-1.48 (2H, m).
¹³C NMR (CDCl₃) δ 172.9, 138.3, 126.7, 128.5, 128.0, 127.9, 127.3. 67.4, 54.6, 51.8, 39.7, 25.1, 21.5.

### E. Cis-2-phenylpiperidine-1-(benzyloxycarbonyl)-3-carboxamide

To a suspension of ammonium chloride (1.66 gm, 31 mmole) in benzene (60 ml) at -5°C, was slowly added a 2M solution (15.6 ml, 31 mmole) of trimethyl aluminum in hexane. After addition was complete, the reaction mixture was allowed to warm to room temperature and was stirred for 1 hr until gas evolution had ceased. A solution of cis-methyl 2-phenylpiperidine-1-(benzyloxycarbonyl)-3-carboxylate (2.2 gm, 6.2 mmole) in benzene (10 ml) was added and the solution was maintained at 50°C for 16 hours. The reaction mixture was cooled to room temperature and was carefully quenched with 5% HCl. The resulting mixture was filtered through diatomaceous earth (Celite (trademark)) and the residue was washed with methylene chloride (200 ml). The organic layer was separated while the aqueous layer was made basic and extracted with methylene chloride (200 ml). The organic extracts were combined, dried (anhyd. magnesium sulfate) and concentrated in vacuo to afford a residue which was suspended in 1:1 ether-pentane to afford cis-2-phenylpiperidine-1-(benzyloxycarbonyl)-3-carboxamide as a white solid (1.4 gm, 66%). M.p. 171°C.
¹H NMR (CDCl₃) δ 7.35-7.28 (10H, m), 5.86 (1H, d, J = 4.9 Hz), 5.66-5.58 (1H, m), 5.48-5.37 (1H, m), 5.21 (1H, d, J = 12 Hz), 5.13 (1H, d, J = 12 Hz), 4.02 (1H, bd, J = 13 Hz), 2.9-2.74 (2H, m), 2.11-1.98 (2H, m), 1.86-1.76 (1H, m), 1.66-1.5 (1H, m).
HRMS Calc'd for C₂₀H₂₂N₂O₃: 338,1630. Found: 338.1634.

### F. Cis-1-(benzyloxycarbonyl)-3-amino-2-phenylpiperidine

Cis-2-phenylpiperidine-1-(benzyloxycarbonyl)-3-carboxami-de (1.4 gm, 4.1 mmole) was dissoved in dry tert.-butanol (40 ml) at 50°C, and lead tetraacetate (1.9 gm, 4.3 mmole) was added. The resulting brown reaction mixture was refluxed for 0.5 hours. Additional lead tetraacetate (1.9 gm, 4.3 mmole) was added over a period of 1 hour. At the end of this period, the reaction mixture was poured into cold 1N hydrochloric acid and filtered through diatomaceous earth (Celite (trademark)). The aqueous phase was extracted with ethyl acetate (3 x 100 ml) and the combined organic layers were washed successively with water, 5% aqueous sodium hydroxide, water, and brine and dried (anhyd. magnesium sulfate). Evaporation of the solvent under reduced pressure afforded a residue which was chromatographed on a SiO₂-gel column. Elution with 25% ethyl acetate in hexane afforded chromato- graphically homogeneous cis-1-(benzoyloxycarbonyl)-3-(N-tert-butoxycarbonyl)-2-phenylpiperidine (1.1 gm) as oil. This was dissovled in ethyl acetate (20 ml) and hydrogen chloride gas was bubbled through it for 5 min. Then the reaction mixture was taken up in aqueous ammonia and extracted with methylene chloride (2 x 200 ml). The organic extracts were combined, dried and evaporated to afford chromatographycally pure cis-1-(benzyloxycarbonyl)-3-amino-2-phenylpiperidine as oil (0.830 gms, 65%).

Cis-1-(benzoyloxycarbonyl)-3-(N-tert.-butoxycarbonyl)-2-phenylpiperidine: ¹H NMR (CDCl₃) δ 7.39-7.16 (10H, m), 5.46 (1H, bd, J = 6 Hz), 5.13 (1H, d, J = 13 Hz), 4.98 (1H, d, J = 13 Hz), 4.14-3.93 (2H, m), 3.23 (1H, bt), 1.9-1.5 (5H, m), 1.39 (9H, s).

Cis-1-(benzyloxycarbonyl)-3-amino-2-phenylpiperi- dine: ¹H NMR (CDCl₃) 7.42-7.36 (2H, m), 7.32-7.12 (8H, m), 5.26 (1H, d, J = 5 Hz), 5.07 (1H, d, J = 12 Hz), 4.95 (1H, d, J = 12 Hz), 4.06 (1H, bd, J = 12, 5 Hz), 3.12- 3.08 (2H, m), 1.88-1.53 (4H, m).

### G. Cis-3-(2-methoxybenzylamino)-2-phenylpiperidine

Cis-1-(benzyloxycarbonyl)-3-amino-2-phenylpiperidine (0.78 gm, 2.5 mmole) was dissolved in methanol (25 ml) and the pH of the medium was adjusted to 5 with the help of methanolic hydrochloric acid. To it crushed molecular sieves (1.0 gm), sodium cyanoborohydride (0.163 gm, 2.5 mmole) and o-methoxybenzaldehyde (0.411 gm, 3.0 mmole) were added, and the resulting reaction mixture was stirred at room temperature for 16 hours. At the end of this period, the reaction mixture was filtered through diatomaceous earth (Celite (trademark)) and the filtrate was taken up in aqueous ammonium hydroxide. The aqueous phase was extracted with methylene chloride (3 x 60 ml) and dried (anhyd. magnesium sulfate). The solvents were removed under reduced pressure to afford an oily residue (1.18 gm). This was dissolved in ethanol (27 ml) and 10% palladium on carbon (1.2 gm) and ammonium formate (0.864 gm, 14 mmole) were added. The resulting reaction mixture was stirred at 25C for 16 hrs. At the end of this period, the reaction mixture was filtered through diatomaceous earth (Celite (trademark)), which was washed with ethanol (50 ml) and methylene chloride (100 ml). The solvents were removed under vacuum to afford a solid which was taken up in aqueous ammonium hydroxide and extracted with methylene chloride (3 x 60 ml). The organic extracts were combined and dried (anhyd. magnesium sulfate). Evaporation of the solvents under pressure afforded a yellow oil from which cis-3-(2-methoxybenzylamino)- 2-phenylpiperidine (728 mg, 83%) was isolated as white solid by treatment with ether-HCl. This was crystallized from ethanol/methanol to afford the hydrochloride salt of the title compound (0.58 mg, m.p. 250C).

### EXAMPLE 64

### (+) S,S-Cis-3-(2-methoxybenzylamino)-2-phenylpiperidine

The title compound was prepared according to the procedure of Example 63, starting with enantiomerically pure (+)R-4-phenylazetidin-2-one.
M.p. 249°C (dec., HCl salt). [α]_{D} = +77 (c=1, CH₃OH).

### EXAMPLE 65

### (-)R,R-Cis-3-(2-methoxybenzylamino)-2-phenylpiperidine

The title compound was prepared by the procedure described in Example 63, starting with enantiomerically pure (-)S-4-phenylazetidin-2-one.
M.p. 251°C (dec., HCl salt). [α]_{D} = -79° (c = 1, CH₃OH).

The title compounds of examples 66-70 have the following general formula and were prepared by a procedure simlar to that described in Example 1.

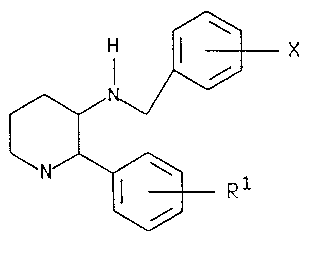

### EXAMPLE 66

Trans-3-(2-chlorobenzylamino)-2-phenylpiperidine (R₁=H, X=2-Cl). M.p. > 255°C (dec., HCl salt). ¹H NMR (CDCl₃) δ 7.42-7.02 (9H, m), 3.69 (1H, d, J = 13.9 Hz), 3.56 (1H, d, J = 13.8 Hz), 3.80 (1H, d, J = 9.1 Hz), 3.09 (1H, bd, J = 11.4 Hz), 2.75 (1H, dt, J = 11.8, 2.9 Hz), 2.62-2.54 (1H, m), 2.29-2.23 (1H, m), 1.79-2.23 (2H, m), 1.34-1.24 (1H, m). HRMS Calc'd for C₁₈H₂₁N₂Cl: 300.1392. Found: 300.1387.

### EXAMPLE 67

Cis-3-benzylamino-2-(3-trifluorophenyl)-piperidine (R₁=3-CF₃, X = H). M.p. > 270°C (dec., HCl salt). ¹H NMR (HCl salt, MeOH-CDCl₃) δ 8.85 (2H, bs), 8.74-8.44 (2H, m), 8.21 (1H, 2), 8.16-8.02 (4H, m), 5.97 (1H, bs), 4.86 (1H, bs), 4.58 (1H, t, J = 10 Hz), 4.46-4.36 (2H, m), 4.2-4.14 (2H, m), 3.24-3.08 (3H, m), 2.75 (1H, bd, J = 10 Hz). HRMS Calc'd for C₁₉H₂₁N₂CF₃: 296.1889. Found: 296.1904.

### EXAMPLE 68

Cis-3-benzylamino-2-phenylpiperidine (R¹ = H, X = H). M.p. 250°C (dec. HCl salt). ¹H-NMR (CDCl) δ 6.94-7.4 (10H, m), 3.89 (1H, d, J=2.3 Hz), 3.52 (1H, d, J=13 Hz), 3.32 (1H, d, J=13 Hz), 3.25 (1H, bd, J=12 Hz), 2.88 (1H, d, J=2.5 Hz), 2.78 (1H, dt, J=12, 3Hz), 2.4 (1H, d, J=12 Hz), 1.8-1.98 (1H, m), 1.6 (1H, d, J=12, 2.5 Hz), 1.42 (1H, d, J=12 Hz).

### EXAMPLE 69

Trans-3-(2-methoxybenzylamino)-2-phenylpiperidine (R₁ = H, X = 2-OMe). M.p. > 250°C (dec., HCl salt). ¹H NMR (CDCl₃) δ 7.29-7.24 (5H, m), 7.14 (1H, t, J = 8 Hz), 6.97 (1H, d, J = 8 Hz), 6.81 (1H, t, J = 8 Hz), 6.67 (1H, d, J = 8 Hz), 3.68 (1H, d, J = 14 Hz), 3.47 (1H, d, J = 14 Hz), 3.39 (3H, s), 3.38-3.34 (1H, m), 3.06 (1H, bd, J = 14 Hz), 2.73 (1H, td, J = 9, 3 Hz), 2.51 (1H, td, J = 8, 3 Hz), 2.32-2.2 (1H, m), 1.76-1.5 (2H, m), 1.36-1.2 (1H, m). MS (M⁺298.18).

### EXAMPLE 70

Cis-3-benzylamino-2-(4-phenylphenyl)piperidine (R₁=4-Ph, X = H) M.p. > 268°C (HCl salt). ¹H NMR (CD₃OH, HCl salt) δ 7.8 (4H, m), 7.59 (2H, d, J = 5 Hz), 7.40 (2H, t, J = 3 Hz), 7.38=7.24 (6H, m), 4.98 (1H, bs), 3.98 (1H, bs), 3.87 (1H, d, J = 10 Hz), 3.68-3.58 (2H, m), 3.34-3.22 (3H, m), 2.46-2.16 (3H, m), 2.01-1.90 (1H, m). HRMS Calc'd for C₂₄H₂₆N₂: 342.2096. Found: 342.2057.

The title compounds of Examples 71-75 have the following general formula and were prepared by a procedure similar to that described in Example 1.

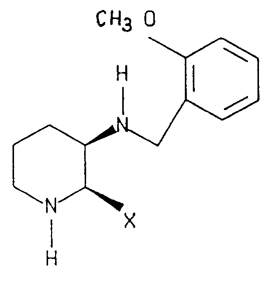

### EXAMPLE 71

Cis-3-(2-methoxybenzylamino)-2-(3-thienyl)-piperidine (X=3-thienyl). M.p. > 239°C (HCl salt). ¹H NMR (CDCl₃) δ 7.25-7.11 (3H, m), 7.03 (1H, dd, J = 7.3, 1.7 Hz), 6.85-6.82 (2H, m), 6.73 (1H, d, J = 8.2 Hz), 3.94 (1H, bs), 3.73 (1H, d, J = 13.7 Hz), 3.57 (3H, s), 3.45 (1H, d, J = 13.7 Hz), 3.20 (1H, bd, J = 10.4 Hz), 2.82 (1H, d, J = 2.7 Hz), 2.76 (1H, dt, J = 12.5, 3.1 Hz), 2.11 (1H, bd, J = 13.4 Hz), 1.97-1.84 (1H,m), 1.57 (1H, tt, J = 13.4, 3.5 Hz), 1.36 (1H, bd, J = 13.2 Hz). HRMS Calc'd for C₁₇H₂₂N₂OS: 302.1535. Found: 302.1444.

### EXAMPLE 72

Cis-3-(2-methoxybenzylamino)-2-benzylpiperidine (X=benzyl). M.p. > 241°C (HCl salt). ¹H NMR (CDCl₃) δ 7.37 (1H, dd, J = 7.3, 1.6 Hz), 7.29-7.2 (6H, m), 6.93 (1H, dt, J = 7.4, 1.0 Hz), 6.88 (1H, dd, J = 8.2, 0.7 Hz), 3.89 (1H, d, J = 13.5 Hz), 3.85 (1H, s), 3.70 (1H, d, J = 13.5 Hz), 3.00-2.89 (2H, m), 2.82 (1H, s), 2.79 (1H, d, J = 3.6 Hz), 2.71-2.67 (1H, m), 2.57 (1H, dt, J = 10.7, 3.2 Hz), 1.97-1.92 (1H, m), 1.75-1.63 (1H, m), 1.44-1.36 (2H, m). HRMS Calc'd for C₂₀H₂₆N₂O: 310.2045. Found: 310.2073.

### EXAMPLE 73

Cis-3-(2-methoxybenzylamino)-2-cyclohexylpiperidine (X=cyclohexyl). M.p. > 225°C (HCl salt). ¹H NMR (CDCl₃) δ 7.13-7.31 (2H, m), 6.9 (1H, t, J = 8 Hz), 6.82 (1H, d, J = 9 Hz), 3.9 (1H, d, J = 14 Hz), 3.81 (3H, s), 3.6 (1H, d, J = 15 Hz), 3.11 (1H, bd, J = 9 Hz), 2.72 (1H, bs ), 2.6 (1H, t, J = 10 Hz), 2.19 (1H, d, J = 9 Hz), 2.11 (1H, bd, J = 12 Hz), 2.01-1.53 (1H, m), 1.38-1.04 (6H, m), 0.92-0.65 (2H, m). HRMS Calc'd for C₁₉H₃₀N₂O: 302.2358. Found: 302.2352.

### EXAMPLE 74

Cis-3-(2-methoxybenzylamino)-2-tert.butylpiperidine (X=tert. butyl). Mp. > 251°C (HCl salt). ¹H NMR (CDCl₃) δ 7.33 (1H, dd, J = 7.3, 1.6 Hz), 7.21 (1H, dd, J = 7.8, 1.7 Hz), 6.90 (1H, dt, J = 7.4, 0.95 Hz), 6.84 (1H, d, J = 8.2 Hz), 3.91 (1H, d, J = 13.6 Hz), 3.81 (3H, s), 3.55 (1H, d, J = 13.6 Hz), 3.13 (1H, bd, J = 12.1 Hz), 2.88 (1H, bs), 2.61 (1H, dt, J = 12.3, 2.9 Hz), 2.19 (1H, d, J = 1.9 Hz), 2.12 (1H, bd, J = 12.9 Hz), 1.76-1.66 (1H, m), 1.35-1.22 (2H, m), 9.95 (9H, s). HRMS Calc'd for C₁₇H₂₈N₂O: 276.2201. Found: 276.2217.

### EXAMPLE 75

Cis-3-(2-methoxybenzylamino)-2-(3-furanyl)-piperidine (X = 3-furanyl). M.p. > 247°C (HCl salt). ¹H NMR (CDCl₃) δ 7.34 (2H, d, J = 1.4 Hz), 7.19 (1H, dt, J = 7.7, 1.7 Hz), 7.11 (1H, dd, J = 7.3, 1.6 Hz), 6.85 (1H, t, J = 7.4 Hz), 6.77 (1H, d, J = 8.1 Hz), 6.15 (1H, td, J = 1.2 Hz), 3.8 (2H, d, J = 14.0 Hz), 3.65 (3H, s), 3.54 (1H, d, J = 13.6 Hz), 3.14 (1H, bd, J = 12.7 Hz), 2.75 (2H, dt, J = 12.1, 3.2 Hz), 2.09 (1H, bd, J = 13.6 Hz), 1.93-1.83 (1H, m), 1.54 (1H, tt, J = 13.2, 3.5 Hz), 1.36 (1H, bd, J = 13.1 Hz). HRMS Calc'd for C₁₇H₂₂N₂O₂: 286.1681. Found: 286.1682.

### EXAMPLE 76

### Cis-3-(2-methoxybenzylamino)-2-phenylazacycloheptane

The title compound was prepared according to the procedure of Example 63 starting with (±)-4-phenylazetidine-2-one and using 1-bromo-4-chlorobutane in procedure B instead of 1-bromo-3-chloropropane. M.p. 230-230°C (dec HCl salt).
¹H-NMR (CDCl₃) δ 1.21 (m, 1H) 1.55 (m, 1H), 1.80 (m, 5H), 2.75 (m, 1H), 3.06 (m, 1H), 3.36 (m, 1H), 3.39 (s, 1H), 3.45 (d, 1H, J = 13 Hz), 3.50 (m, 1H), 6.62 (d, 1H, J = 6Hz), 6.76 (t, 1H, J = 6 Hz), 6.91 (d, 1H, J = 6 Hz), 7.12 (m, 2H), 7.22 (m, 4H). HRMS Calc'd for C₂₀H₂₇N₂O: 311.2124. Found: 311.2132.

The title compounds of Examples 77-81 have the following general formula and were prepared similar to that described in Example 1.

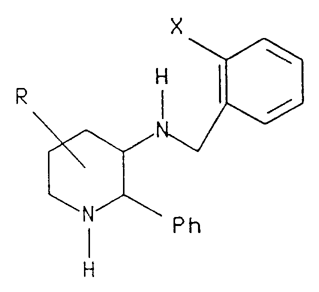

### EXAMPLE 77

### 3α-(Benzylamino)-3β-methyl-2β-phenylpiperidine (R=CH₃, X=H)

M.p. 269°C (dec., HCl salt).
¹H-NMR δ (CDCl₃, 300 MHz) 7.1-7.45 (m, 10H), 3.81 (dd, J=13Hz, 1H), 3.71 (s, 1H), 3.66 (dd, J=13Hz, 1H), 3.05-3.1 (m, 1H), 2.78 (dt, J=3 and 11Hz, 1H), 1.4-2.0 (m, 4H), 1.15 (s, 3H). ¹³C-NMR (CDCl₃) δ 141.6, 141.1, 128.8, 128.3, 127.83, 127.81, 127.3, 126.6, 70.0, 65.9, 54.8, 47.9, 45.5, 37.1, 23.5, 18.9 and 15.3. Calc'd for C₁₉H₂₄N₂·2HCl·1/8H₂O C, 64.18; H, 7.44; N, 7.88. Found: C, 64.12; H, 7.36; N, 7.85. HRMS Calcd for C₁₉H₂₄N₂: 280.193. Found: 280.1932.

### EXAMPLE 78

### 3β-(Benzylamino)-3α-methyl-2β-phenylpiperidine (R=CH₃, X=H)

M.p. 238°C (HCl salt).
¹H-NMR δ (CDCl₃, 300 MHz) 7.1-7.4 (m, 10H), 3.64 (dd, J=13Hz, 1H), 3.52 (dd, J=13Hz, 1H), 3.50 (s, 1H), 3.13-3.18 (m, 1H), 2.73 (dt, J=12 and 3Hz, 1H), 2.1 (bd, J=13.8Hz, 1H), 1.45-1.52 (m, 1H), 1.25-1.4 (m, 1H), 0.93 (s, 3H). ¹³C-NMR (CDCl₃) δ 142.1, 141, 129.1, 128.1, 127.9, 127.5, 127, 126.4, 72.1, 53.3, 47.9, 45.3, 34.5, 24.7, 22.5. HRMS Calcd for C₁₉H₂₄N₂: 280.193. Found: 280.1930.

### EXAMPLE 79

### 3α-(2-Methoxybenzylamino)-3β-methyl-2β-phenylpiperidine (R=CH₃, X=OCH₃)

M.p. 233°C (dec., HCl salt).
¹H-NMR δ (CDCl₃, 300MHz) 7.15-7.46 (m, 7H), 6.85 (dt, J=7 and 1Hz, 1H), 6.74 (d, J=8Hz, 1H), 3.7 (s, 1H), 3.68 (dd, J=13Hz, 1H), 3.6 (s, 3H), 3.16 (m, 1H), 2.76 (dt, J=3 and 11Hz, 1H), 1.97 (bd, J=11Hz, 1H), 1.55-1.83 (m, 3H), 1.14 (s, 3H), HRMS Calcd for C₂₀H₂₆N₂O: 310.2046. Found: 310.2038.

### EXAMPLE 80

### 3β-(2-Methoxybenzylamino)-3α-methyl-2β-phenylpiperidine (R=CH₃, X=OCH₃)

M.p. 242°C (HCl salt).
¹H-NMR δ (CDCl₃, 300MHz) 7.15-7.4 (m, 7H), 6.91 (dt, J=7 and 1 Hz, 1H), 6.81 (d, J=8 Hz, 1H), 3.73 (s, 3H), 3.63 (dd, J=13Hz, 1H), 3.52 (s, 1H), 3.5 (dd, J=13Hz, 1H), 3.13-3.21 (m, 1H), 2.75 (dt, J=12 and 3Hz, 1H), 2.16 (bd, J=14Hz, 1H), 1.73-1.91 (m, 1H), 1.48 (bd, J=13Hz, 1H), 1.33 (dt, J=14 and 4Hz, 1H), 0.93 (s, 3H). HRMS Calc'd for C₂₀H₂₆N₂O: 310.2045. Found: 310.2092.

### EXAMPLE 81

### 3α-(2-Methoxybenzylamino)-5β-methyl-2β-phenylpiperidine (R=CH₃, X=OCH₃)

M.p. 208°C (dec.).
¹H-NMR δ (CDCl₃, 300 MHz) 7.22-7.31 (m, 5H), 7.16 (dt, J=7 and 1Hz, 1H), 6.99 (dd, J=8 and 1Hz, 1H), 6.81 (dt, J=8 and 1Hz, 1H), 6.69 (bd, J=8Hz, 1H), 3.66 (dd, J=13, 1H), 3.46 (dd, J=13Hz, 1H), 3.43 (s, 3H), 3.36 (d, J=9Hz, 1H), 2.97 (dd, J=11 and 3Hz, 1H), 2.67-2.86 (m, 2H), 1.97-2.09 (m, 1H), 1.43-1.58 (m, 2H). 1.13 (d, J=7Hz, 3H). HRMS Calc'd for C₂₀H₂₆N₂O:310.2046. Found: 310.2045.

### EXAMPLE 82

### 3β-(3-Thienylmethylamino)-2β-(3-thienyl)piperidine

The title compound was prepared by a procedure similar to that described in Example 1.
M.p. 262°C. (HCl salt).
¹H-NMR δ (CDCl₃, 300 MHz) 7.05-7.3 (m, 3H), 6.97 (dd, J=5 and 1Hz, 1H), 6.88 (m, 1H), 6.77 (dd, J=5 and 1Hz, 1H), 3.98 (d, J=2Hz, 1H), 3.6 (dd, J=14, 1H), 3.44 (dd, J=14Hz, 1H), 3.16-3.21 (m, 1H), 2.89-2.92 (m, 1H), 2.76 (dt, J=12 and 3Hz, 1H), 1.3-2.1 (m, 4H). ¹³C-NMR (CDCl₃) δ 144.1, 142, 127.5, 126.2, 125.7, 125.3, 120, 120.3, 61.2, 54.8, 47.5, 46.4, 28.7, 20.5. HRMS Calc'd for C₁₄H₁₈N₂S₂: 278.0906. Found: 278.0922. C, 47.86; H, 5.74; N, 7.97. Found: C, 47.87; H, 5.79; N, 7.61.

### EXAMPLE 82-A

The title compound was prepared by a procedure similar to that described in Example 1.

### Trans-3-benzylamino-2-phenylpiperidine (R=X=H)

M.p. 269°C (dec., HCl salt).
¹H-NMR δ (CDCl₃, 300 MHz) 6.9-7.4 (m, 10H), 3.63 (dd, J=13Hz, 1H), 3.42 (dd, J=13 Hz, 1H), 3.38 (d, J=7.4 Hz, 1H), 3.07 (bdt, J=11.6 Hz, 1H), 2.14-2.24 (m, 1H), 1.56-1.8 (m, 3H), 1.2-1.38 (m, 1H). HRMS Calc'd for C₁₈H₂₂N₂:266.1783. Found: 266.1764.

### EXAMPLE 83

### Cis-(2S,3S)-1-(4,4-bis-(4-fluorophenyl)butyl)-3-(2-methoxybenzyl)amino-2-phenyl-piperidine dimesylate

The title compound was prepared by a procedure similar to that of Example 2.
C₃₅H₃₈F₂N₂O·2CH₃SO₃H, mp. 55-60°C (41%).
¹H NMR (CDCl₃) δ 1.20-1.80 (m, 4H), 1.92-2.20 (m, 4H), 2.40 (m, 1H), 2.46-2.66 (m, 2H), 3.14 (m, 1H), 3.25 (s, 3H), 3.45 (s, 1H), 3.60-3.82 (m, 4H), 4.32 (m, 1H), 6.60 (d, 1H), 6.78-6.95 (m, 5H), 7.00 (d, 1H), 7.06-7.24 (m, 5H), 7.26-7.42 (m, 5H).
MS (m/e, %): 540 (5, M+), 364 (100), 314 (15), 148 (53), 121 (87), 91 (90).

### EXAMPLE 84

### Cis-(2S,3S)-3-(2-methoxybenzyl)amino-2-phenyl-1-[4-(thiophen-2-yl)but-1-yl]

The title compound was prepared by a procedure similar to that of Example 2.
C₂₇H₃₄N₂OS, oil.
¹H NMR (CDCl₃) δ 1.32-1.60 (m, 6H), 2.8-2.9 (m, 1H), 1.96-2.3 (m, 4H), 2.50-2.72 (m, 4H), 3.16-3.38 (m, 3H), 3.40 (s, 3H), 3.65-3.80 (m, 1H), 6.59-6.76 (m, 3H), 6.81-6.88 (m, 2H), 7.02-7.12 (m, 2H), 7.20-7.38 (m, 5H).
MS (m/e, %): 434 (60, M+), 271 (31), 258 (100), 121 (32), 91 (35).

The title compounds of Example 85-89 were prepared by a procedure similar to that described in Example 1.

### EXAMPLE 85

### Cis-3-(5-chloro-2-methoxybenzyl)amino-2-(3-fluorophenyl)piperidine dimesylate

C₁₉H₂₂ClFN₂O·2CH₃SO₃H, hygroscopic solid, (34%).
¹H NMR (CDCl₃) δ 1.40 (m, 1H), 1.60 (m, 1H), 1.78 (s, 2H), 1.87 (m, 1H), 2.09 (m, 1H), 2.79 (m, 2H), 3.25 (m, 1H), 3.36 (d, 1H), 3.51 (s, 3H), 3.62 (d, 1H), 3.87 (d, 1H, J=2Hz), 6.61 (d, 1H, J=8.7 Hz), 6.89-7.12 (m, 5H), 7.22-7.31 (m, 1H).
MS-FAB (m/e, %): 349 (100, M+), 351 (35), 178 (25), 155 (29), 119 (31).

### EXAMPLE 86

### Cis-3-(2,5-dimethoxybenzyl)amino-2-(3-methoxyphenyl)piperidine dihydrochloride

C₂₁H₂₈N₂O₃·2HCl, mp. 235-237°C, (6%).
¹H NMR (CDCl₃, free base) δ 1.42 (m, 1H), 1.60 (m, 1H), 1.83-2.20 (m, 4H), 2.72-2.87 (m, 2H), 3.27 (m, 1H), 3.40 (d, 1H), 3.43 (s, 3H), 3.65 (d, 1H), 3.72 (s, 3H), 3.77 (s, 3H), 3.86 (d, 1H), 6.58-6.70 (m, 2H), 6.73-6.88 (m, 3H), 7.18-7.28 (m, 2H).
MS-FAB (m/e, %): 357 (100, M+), 190 (18), 151 (26), 119 (29).

### EXAMPLE 87

### Cis-3-(2,5-dimethoxybenzyl)amino-1-ethyl-2-(3-fluorophenyl)piperidine dimesylate

C₂₂H₂₉FN₂O₂·2CH₃SO₃H, oil.
¹H NMR (CDCl₃, free base) δ 0.97 (t, 3H), 1.5 (m, 2H), 1.86-2.15 (m, 5H), 2.55-2.70 (m, 2H), 3.23 (m, 1H), 3.32 (s, 1H), 3.40 (d, 1H), 3.50 (s, 3H), 3.68 (d, 1H), 3.70 (s, 3H), 6.58-6.70 (m, 3H), 6.93 (m, 1H), 7.10 (dd, 2H), 7.25 (m, 1H).
MS-FAB (m/e, %): 373 (100, M+), 359 (10), 206 (46).

### EXAMPLE 88

### Cis-3-(5-chloro-2-methoxybenzyl)amino-2-(3-methoxyphenyl)piperidine dihydrochloride

C₂₀H₂₅ClN₂O₂·2HCl, mp. 256-259°C, (25%).
¹H NMR (CDCl₃, free base) δ 1.40 (m, 1H), 1.58 (m, 1H), 1.80-2.15 (m, 4H), 2.78 (m, 2H), 3.25 (m, 1H), 3.32 (d, 1H), 3.46 (s, 3H), 3.63 (d, 1H), 3.75 (s, 3H), 3.85 (d, 1H), 6.58 (d, 1H), 6.75-6.88 (m, 3H), 6.95 (d, 1H), 7.08 (dd, 1H), 7.20 (t, 1H).
¹³C NMR (CDCl₃, free base) δ 20.1, 28.2, 46.1, 47.6, 54.7, 55.0, 55.1, 63.9, 110.8, 111.1, 112.8, 118.5, 124.8, 127.2, 129.13, 129.18, 130.2, 143.9, 156.0, 159.6.

### EXAMPLE 89

### Cis-3-(5-chloro-2-methoxybenzyl)amino-2-(3-chlorophenyl)piperidine dihydrochloride

C₁₉H₂₂Cl₂N₂O·2HCl, mp. 270-273°C, (6%).
¹H NMR (d₆-DMSO, 2HCl salt) δ 1.39-1.65 (m, 3H), 1.87 (m, 4H), 2.13 (d, 1H), 2.77 (t, 2H), 3.21-3.66 (m, 6H), 3.84 (s, 1H), 6.60 (d, 1H, J=8.7 Hz), 6.94 (d, 1H, J=2.6 Hz), 7.07-7.27 (m, 5H).

### EXAMPLE 90

### (+)-(2S,3S)-3-Amino-2-phenylpiperidine

In a bottle were placed 10 g of 10% palladium-carbon, 100 ml of methanol, 150 ml of ethanol, 3.5 ml of concentrated hydrochloric acid and 5 g of the hydrochloride salt of the title compound of Example 64. The mixture was shaken under hydrogen (40 p.s.i.) overnight, 5 g of additional catalyst was added to the system and the mixture was shaken under hydrogen for 3 days. The mixture was diluted with water, filtered through diatomaceous earth (Celite (trademark)) and the Celite (trademark) was rinsed with H₂O. The filtrate was concentrated to remove most of the alcohol, the remaining liquid was extracted with chloroform and the chloroform extracts were dried (sodium sulfate) and concentrated to obtain 2.16 g of the title compound.
[α]_{D} (HCl salt) = + 62.8° (C=0.46, MeOH).
¹H NMR (CDCl₃) δ 1.68 (m, 4H), 2.72 (m, 1H), 2.95 (m, 1H), 3.16 (m, 1H), 3.80 (d, 1H, J=3), 7.24 (m, 5H).
HRMS Calc'd for C₁₁H₁₆N₂:176.1310. Found: 176.1309. Calc'd for C₁₁H₁₆N₂·2HCl·1/3H₂O: C, 51.78; H, 7.36; N, 10.98. Found: C, 51.46; H, 7.27; N, 10.77.

### EXAMPLE 91

### (+)-(2S,3S)-3-(2,5-Dimethoxybenzyl)amino-2-phenylpiperidine

Under a nitrogen atmosphere in a round-bottom flask were placed 600 mg (3.4 mmol) of (+)-(2S,3S)-3-amino-2-phenylpiperidine, 8 ml of acetic acid and 622 mg (3.7 mmol) of 2,5-dimethoxybenzaldehyde, and the mixture was stirred for 30 minutes. To the system was added 1.58 g (7.5 mmol) of sodium triacetoxyborohydride, and the mixture was stirred at room temperature overnight. The mixture was concentrated, basified with 1M aqueous sodium hydroxide and extracted with methylene chloride. The methylene chloride extracts were washed with water and extracted with 1M aqueous hydrocloric acid. The hydrochloric acid extracts were basified with 1M aqueous sodium hydroxide and extracted with methylene chloride. The methylene chloride extracts were dried (sodium sulfate) and concentated to obtain 528 mg of colorless oil. The oil was dissolved in methylene chloride, and ether saturated with hydrogen chloride was added to the solution. The resulting white solid was collected by filtration and stirred in isopropanol at 60°C for 2 hours. Filtration afforded 414 mg of the title compound as its hydrochloride. Additional material (400 mg) was obtained by extracting the initial basic layer with additional methylene chloride, drying (sodium sulfate) and concentration. [α]_{D} (HCl salt) = + 60.5° (c=0.58, CH₃OH).
¹H NMR (CDCl₃) δ 1.38 (m, 1H), 1.58 (m, 1H), 1.88 (m, 1H), 2.13 (m, 1H), 2.78 (m, 2H), 3.25 (m, 1H), 3.36 (d, 1H, J=18), 3.44 (s, 3H), 3.62 (d, 1H, J=18), 3.72 (s, 3H), 3.88 (d, 1H, J=3), 6.62 (m, 3H), 7.24 (m, 5H). Mass spectrum: m/z 326 (parent). Calc'd for C₂₀H₂₆N₂O₂·2HCl·0.25H₂O: C, 59.48; H, 7.11; N, 6.93. Found: C, 59.33; H, 6.91; N, 7.23.

### EXAMPLE 92

### 3-(2-Methoxy-5-methylbenzyl)amino-2-phenylpiperidine

### A. 3-(2-Methoxy-5-methylbenzyl)amino-2-phenylpyridine

Under a nitrogen atmosphere in a round-bottom flask were placed 1.5 g (10 mmol) of 2-methoxy-5-methylbenzaldehyde and 22 ml of acetic acid. To the system, cooled in an ice bath, was added 3.6 g (17 mmol) of sodium triacetoxyborohydride in portions. The mixture was stirred at room temperature overnight. The reaction mixture was concentrated and partitioned between aqueous sodium hydroxide and dichloromethane. The layers were separated, the aqueous phase was extracted with dichloromethane and the combined organic fractions were dried (sodium sulfate) and concentrated to obtain 2.5 g of a brown oil. The crude product was purified by flash column chromatography using 5:1 hexanes/ethyl acetate as the eluant to obtain 1.65 g of the title compound as a yellow oil.
¹H NMR (CDCl₃) δ 2.22 (s, 3H), 3.72 (s, 3H), 4.24 (d, 2H, J=4), 4.73 (broad t, 1H), 6.70 (d, 1H, J=5), 7.0 (m, 4H), 7.33 (m, 1H), 7.44 (m, 2H), 7.59 (d, 2H, J=4), 7.99 (d, 1H, J=1).

### B. 3-(2-Methoxy-5-methylbenzyl)amino-2-phenylpiperidine

In a bottle were place 600 mg (1.97 mmol) of the title compound of Example 92A, 32 ml of ethanol, 118 µl (2.07 mmol) of acetic acid and 30 mg of platinium oxide. The mixture was shaken under hydrogen (ca. 40 p.s.i.) for ca. 30 hours. During this period, additional (270 mg) platinium oxide and acetic acid (∼18 ml) wre added to the system. The reaction mixture was filtered through diatomaceous earth (Celite (trademark)), the filter cake was washed with ethanol and the filtrate was concentrated. The residue was partitioned between dichloromethane and 1M aqueous sodium hydroxide. The layers were separated and the aqueous phase was extracted with dichloromethane. The combined organic fractions were dried (sodium sulfate) and concentrated to obtain 540 mg of yellow oil. This oil was partitioned between dichloromethane and 1M aqueous hydrogen chloride, the layers were separated and the organic phase was extracted with 1M hydrochloric acid. The combined aqueous extracts were washed with dichloromethane and were made basic with 1M aqueous sodium hydroxide. The aqueous solution was extracted with dichloromethane, and the extracts were dried (sodium sulfate) and concentrated. The resulting oil was purified by flash column chromatography using 4.5-5% methanol/chloroform as the eluant to obtain 110 mg of the title compound, which was converted to its hydrochloride salt, mp 245-247°C.
M.P. 245-247°C.
¹H NMR (CDCl₃) δ 1.30-1.42 (m, 1H), 1.48-1.98 (m, 2H), 2.04-2.16 (m, 1H), 2.18 (s, 3H), 2.68-2.70 (m, 2H), 3.18-3.30 (m, 1H), 3.35 (d, 1H, J=12), 3.40 (s, 3H), 3.58 (d, 1H, J=12), 3.85 (d, 1H, J=3), 6.53 (d, 1H, J=8), 6.71 (d, 1H, J=2), 6.88 (dd, 1H, J=4,10), 7.14-7.26 (m, 5H). HRMS Calc'd for C₂₀H₂₆N₂O:310.2041. Found: 310.2024. Anal. Calc'd for C₂₀H₂₆N₂O·2HCl·1.2H₂O: C, 59.31; H, 7.56; N, 6.92. Found: C, 59.31; H, 7.40; N, 6.85.

### EXAMPLE 93

### (2S,3S)-1-(3-Cyanoprop-1-yl)-3-(2-methoxybenzyl)amino-2-phenylpiperidine

The title compound was prepared by a procedure analogous to that of Example 2, replacing allyl bromide with 4-bromobutyronitrile.
M.P. 63-67°C (dec).
¹H NMR (CDCl₃) δ 1.40 (m, 2H), 1.80 (m, 6H), 2.12 (m, 1H), 2.28 (m, 1H), 2.52 (m, 2H), 3.08 (m, 1H), 3.21 (d, 1H, J=3), 3.34 (d, 1H, J=13), 3.40 (s, 3H), 3.60 (d, 1H, J=13), 6.58 (d, 1H, J=9), 6.68 (t, 1H, J=6), 6.78 (d, 1H, J=6), 7.02 (t, 1H, J=9), 7.20 (m, 5H). Mass spectrum: m/z 363 (parent).

### EXAMPLE 94

### (2S,3S)-1-(4-Aminobut-1-yl)-3-(2-methoxybenzyl)amino-2-phenylpiperidine)

The title compound of Example 93 (1.9 g) was dissolved in 10 ml of acetic acid in a bottle. To the system was added 1.9 g of 5% platinum/carbon (60% water), and the mixture was shaken under hydrogen (40 p.s.i.) for 4 hours. The mixture was diluted with ethanol, filtered through diatomaceous earth (Celite (trademark)) and the filtrate was concentrated. Saturated aqueous sodium bicarbonate was added to the residue until the pH of the mixture was ca. 8, and the mixture was extracted with chloroform. The chloroform extracts were dried (sodium sulfate) and concentrated to obtain 1.6 g of the title compound as an oil.
¹H NMR (CDCl₃) δ 1.50 (m, 8H), 2.02 (m, 3H), 2.52 (m, 3H), 3.18 (m, 1H), 3.26 (d, 1H, J=3), 3.32 (d, 1H, J=15), 3.43 (s, 3H), 3.64 (d, 1H, J=15), 4.77 (br s, 2H), 6.60 (d, 1H, J=9), 6.71 (t, 1H, J=6), 6.82 (d, 1H, J=6), 7.07 (t, 1H, J=9), 7.26 (m, 5H). Mass spectrum: m/z 367 (parent).

### EXAMPLE 95

### (2S,3S)-3-(2-Methoxybenzyl)amino-1-[4-(2-naphthamidobut-1-yl)]-2-phenylpiperidine

Under a nitrogen atmosphere in a round-bottom flask were placed 100 mg (0.27 mmol) of the title compound of Example 94 and 0.5 ml of methylene chloride, and the system was cooled in an ice bath. To the system was added 38 µl (0.27 mmol) of 2-naphthoyl chloride, and the mixture was stirred for 20 minutes. The mixture was poured into saturated aqueous sodium bicarbonate and extracted with chloroform. The chloroform extracts were dried (sodium sulfate) and concentrated to obtain 150 mg of an oil. The crude product was purified by flash column chromatography (6 g of silica gel) using 1:10 methanol/chloroform as the eluant to obtain 71 mg of the title compound, which was converted to its hydrochloride salt.
M.p. 105-107°C (dec.)
¹H NMR (CDCl₃) δ 1.50 (m, 6H), 1.70 (m, 2H), 2.04 (m, 3H), 2.60 (m, 2H), 3.22 (m, 1H), 3.30 (d, 1H, J=1), 3.40 (m, 5H), 3.68 (d, 1H, J=15), 6.28 (br s, 1H), 6.61 (d, 1H, J=9), 6.72 (t, 1H, J=6), 6.84 (d, 1H, J=6), 7.08 (t, 1H, J=9), 7.26 (m, 5H), 7.52 (m, 2H), 7.82 (m, 4H), 8.22 (s, 1H). Mass spectrum: m/z 521 (parent).

### EXAMPLE 96

### (2S,3S)-3-(2-(Methoxybenzyl)amino-1-[(N-naphth-2-ylmethyl)4-aminobut-1-yl)]-2-phenylpiperidine

The title compound of Example 95 is treated with borane dimethylsulfide, employing conditions analogous to those described in Example 1B, to obtain the title compound.

### EXAMPLE 97

### (2RS,3RS)-1-(5-carboethoxypent-1-yl)-3-(2-methoxybenzyl)amino-2-phenylpiperidine

The title compound was prepared by a procedure analogous to that described in Example 2, replacing allyl bromide with ethyl 6-bromohexanoate.
M.p. 80-95°C.
¹H NMR (CDCl₃) δ 1.12 (m, 5H), 1.42 (m, 6H), 1.72 (m, 1H), 1.98 (m, 3H), 2.16 (t, 2H, J=7), 2.46 (m, 1H), 2.54 (m, 1H), 3.15 (m, 1H), 3.23 (m, 1H, J=3), 3.30 (d, 1H, J=15), 3.41 (s, 3H), 3.60 (d, 1H, J=15), 4.02 (q, 2H, J=6), 6.58 (d, 1H, J=9), 6.78 (t, 1H, J=6), 6.80 (d, 1H, J=6), 7.04 (t, 1H, J=9), 7.22 (m, 5H). Mass spectrum: m/z 438 (parent).

### EXAMPLE 98

### (2RS,3RS)-1-(6-Hydroxyhex-1-yl)-3-(2-methoxybenzyl)amino-2-phenylpiperidine

Under a nitrogen atmosphere in a round-bottom flask were placed 95 mg (0.22 mmol) of the title compound of Example 97 and 1 ml of THF. The system was cooled in an ice/acetone bath, 0.44 ml (0.44 mmol) of 1M lithium aluminum hydride in ether was added to the system and the mixture was stirred for 10 minutes. The cold bath was removed, the mixture was stirred at room temperature for 20 minutes and the cold bath was replaced. To the system was added cautiously ca. 0.4 ml of 2M aqueous sodium hydroxide, and the mixture was stirred at room temperature for 20 minutes. Sodium sulfate was added to the system, the mixture was stirred for 30 minutes, solids were removed by suction filtration and the filtrate was concentrated. The crude material was purified by flash column chromatography (5 g of silica gel) using 3:47 methanol/chloroform as the eluant to obtain 49 mg of the title compound.
M.p. (HCl salt) 67-68°C(dec).
¹H NMR (CDCl₃) δ 1.18 (m, 4H), 1.42 (m, 4H), 1.72 (m, 1H), 2.00 (m, 5H), 2.50 (m, 2H), 3.16 (m, 1H), 3.23 (d, 1H, J=3), 3.30 (d, 1H, J=15), 3.41 (s, 3H), 3.52 (t, 2H, J=6), 3.62 (d, 1H, J=15), 6.58 (d, 1H, J=9), 6.69 (t, 1H, J=6), 6.81 (d, 1H, J=6), 7.05 (t, 1H, J=9), 7.22 (m, 5H). Mass spectrum: M/z 396 (parent).

### EXAMPLE 99

### (2S,3S)-1-(5-Carboxypentyl)-3-(2-methoxybenzyl)amino-2-phenylpiperidine

(2S,3S)-1-(5-Carboethoxypentyl)-3-(2-methoxybenzyl)amino-2-phenylpiperidine was prepared by the process of Example 97, utilizing the single enantiomer (2S,3S)-3-(2-methoxybenzyl)amino-2-phenylpiperidine instead of the corresponding racemate. In a round-bottom flask were placed 250 mg of this ester and 8 ml of 4M aqueous hydrochloric acid. The reaction mixture was heated at 60°C for 2 hours and concentrated. The crude product was triturated with ether and with isopranol/ether to obtain the title compound as its hydrochloride salt.
¹H NMR (DMSO-d₆) δ 1.14 (m, 2H), 1.40 (m, 2H), 1.78 (m, 3H), 2.36 (m, 10H), 3.00 (m, 1H), 3.80 (m, 4H), 4.22 (m, 1H), 6.98 (m, 2H), 7.38 (m, 2H), 7.60 (m, 3H), 7.92 (m, 2H). HRMS Calc'd for C₂₅H₃₄N₂O₃:410.2569. Found: 410.2546.

### EXAMPLE 100

### (2S,3S)-3-(2-Methoxybenzyl)amino-1-(N-methyl-5-carboxamidopent-1-yl)-2-phenylpiperidine

Under a nitrogen atmosphere in a round-bottom flask were placed 75 mg (0.17 mmol) of the title compound of Example 99 and 0.5 ml of THF. To this stirring suspension were added 47 µl (0.34 mmol) of triethylamine and 54 mg (0.34 mmol) of N,N-carbonyldiimidazole. The reaction mixture was stirred for 30 minutes and concentrated. To the system was added 0.25 ml of 40% methylamine in water. The reaction mixture was stirred for 30 minutes and poured into a mixture of saturated aqueous sodium bicarbonate and chloroform. The mixture was extracted with chloroform and the chloroform extracts were dried (sodium sulfate) and concentrated. The crude product was purified by flash column chromatography (5 g of silica gel) using 1:9 methanol/chloroform as the eluant to obtain 36 mg of the title compound as an oil.
¹H NMR δ 1.14 (m, 2H), 1.48 (m, 6H), 1.82 (m, 1H), 2.04 (m, 5H), 2.32 (m, 2H), 2.72 (d, 3H, J=5), 3.18 (m, 1H), 3.27 (d, 1H, J=3), 3.32 (d, 1H, J=15), 3.44 (s, 3H), 3.66 (d, 1H, J=15), 6.61 (d, 1H, J=9), 6.72 (t, 1H, J=6), 6.83 (d, 1H, J=6), 7.08 (t, 1H, J=9), 7.24 (m, 5H). Mass spectrum: m/z 423 (parent).

### EXAMPLE 101

### (2S,3S)-1-[4-(4-Fluorophenyl)-4-oxobut-1-yl]-3-(2-methoxybenzyl)amino-2-phenylpiperidine

The title compound was prepared by a procedure analogous to that described in Example 2, replacing allyl bromide with 4-iodobutyl-4-fluorophenylketone.
M.p. 59-60°C (dec).
¹H NMR (CDCl₃) δ 1.46 (m, 2H), 1.96 (m, 6H), 2.58 (m, 2H), 2.84 (m, 2H), 3.24 (m, 1H), 3.30 (d, 1H, J=3), 3.38 (d, 1H, J=16), 3.44 (s, 3H), 3.68 (d, 1H, J=16), 6.62 (d, 1H, J=9), 6.72 (t, 1H, J=6), 6.84 (d, 1H, J=6), 7.08 (m, 3H), 7.27 (m, 5H), 7.92 (m, 2H). HRMS Calc'd for C₂₉H₃₃N₂O₂F:460.2532. Found: 460.2528.

### EXAMPLE 102

### (2S,3S)-1-[4-(4-Fluorophenyl)-4-hydroxybut-1-yl]-3-(2-methoxybenzyl)amino-2-phenylpiperidine

Under a nitrogen atmosphere in a round-bottom flask were placed 569 mg (1.24 mmol) of the title compound of Example 101 and 2.5 ml of methanol, and the system was cooled in an ice bath. To the system was added 47 mg (1.24 mmol) of sodium borohydride in two portions. The mixture was stirred for 30 minutes, 12 mg of sodium borohydride was added and the mixture was stirred for 30 minutes. To the system was added 0.5 ml of saturated aqueous sodium bicarbonate. The mixture was diluted with chloroform, allowed to warm to room temperature and poured into a mixture of chloroform and saturated aqueous sodium bicarbonate. The mixture was extracted with chloroform, dried (sodium sulfate) and concentrated to obtain 500 mg of an oil. The crude product was purified by flash column chromatography (20 g of silica gel) using 1:19 methanol/chloroform as the eluant to obtain 295 mg of the title compound, which was converted to its methanesulfonic acid salt.
¹H NMR (CDCl₃) δ 1.50 (m, 4H), 1.94 (m, 6H), 2.40, 2.55 (2m, 1H), 2.72 (m, 1H), 2.98, 3.40 (2m, 3H), 3.52 (s, 3H), 3.66 (m, 1H), 4.57, 4.71 (2m, 1H), 6.62 (d, 1H, J=9), 6.70 (m, 1H), 6.94 (m, 3H), 7.08 (m, 1H), 7.28 (m, 6H), 7.46 (m, 1H). HRMS Calc'd for C₂₉H₃₅N₂O₂F:462.2678. Found: 462.2688.

### EXAMPLE 103

### (2S,3S)-1-(5,6-Dimethylmethylenedioxyhex-1-yl)-3-(2-methoxybenzyl)amino-2-phenylpiperidine

The title compound was prepared by a procedure analogous to that described in Example 2, replacing allyl bromide with 5,6-dimethylenedioxy-1-methylsulfonyloxyhexane.
¹H NMR (CDCl₃) δ 1.34 (s, 3H), 1.40 (s, 3H), 1.44 (m, 4H), 1.76 (m, 2H), 2.00 (m, 3H), 2.50 (m, 2H), 3.17 (m, 1H), 3.25 (m, 1H), 3.32 (d, 1H, J=15), 3.44 (s, 3H), 3.48 (m, 2H), 3.60 (m, 1H), 3.98 (m, 4H), 6.58 (d, 1H, J=9), 6.70 (t, 1H, J=6), 6.80 (d, 1H, J=6), 7.05 (t, 1H, J=9), 7.24 (m, 5H).

### EXAMPLE 104

### (2S,3S)-1-(5,6-Dihydroxyhex-1-yl)-3-(2-methoxybenzyl)amino-2-phenylpiperidine

In a round-bottom flask were placed 3.2 g of the title compound of Example 103 and 100 ml of a 1:1 mixture of methanol and dichloromethane. To the system was added 50 ml of dichloromethane saturated with hydrogen chloride, and the reaction mixture was allowed to stand at room temperature for 3 hours and concentrated. The residue was dissolved in hot isopropanol and ether was added. The solvent was poured off the resulting gum and discarded. The gum was triturated with hot isopropranol/ether and this solvent was saved. The resulting gum was scratched to form a solid (630 mg). The mother liquor was concentrated and the residual oil was triturated with hot isopropanol/ether and ether to obtain a solid (850 mg). Each of the lots of product was stirred in 50 ml of ether for 2 hours, and the solvent was removed with a pipet. The latter solid was further purified by partitioning between dichloromethane and 1M aqueous sodium hydroxide, extracing the aqueous phase with dichloromethane, drying (sodium sulfate) and concentrating the combined organic fractions and treating a dichloromethane solution of the residue with ethereal hydrogen chloride. This sequence afforded 710 mg of the title compound (hydrochloride) as a very hygroscopic off white solid.
¹H NMR (CDCl₃) δ 1.34 (m, 8H), 1.80 (m, 1H), 2.08 (m, 3H), 2.54 (m, 4H), 3.20 (m, 1H), 3.34 (m, 2H), 3.44 (s, 3H), 3.56 (m, 1H), 3.66 (d, 1H, J=12), 6.60 (d, 1H, J=9), 6.74 (t, 1H, J=6), 6.84 (d, 1H, J=6), 7.09 (t, 1H, J=9), 7.26 (m, 5H).
HRMS Calc'd for C₂₅H₃₆N₂O₃:412.2726. Found: 412.2699.

The title compound of Examples 105 and 106 were prepared by a procedure similar to that of Example 1.

### EXAMPLE 105 (2RS,3RS,5SR)-3-(2-Methoxbenzylamino)-5-methyl-2-phenylpiperidine

M.p. 179-181°C (HCl salt, dec.).
¹H NMR (CDCl₃) δ 7.20 (m, 6H), 7.00 (d, 1H, J=7 Hz), 6.76 (t, 1H, J=7 Hz), 6.66 (d, 1H, J=6 Hz), 3.97 (d, 1H, J=2 Hz), 3.64 (d, 1H, J=12 Hz), 3.51 (d, 1H, J=12 hz), 3.48 (s, 3H), 2.84 (m, 3H), 1.78 (m, 3H), 1.13 (d, 3H, J=7 Hz). HRMS calc'd for C₂₀H₂₆N₂O: 310.2045. Found: 310.2101.

### EXAMPLE 106

### (2RS,3RS,5RS)-3-(2-Methoxybenzylamino)-5-methyl-2-phenylpiperidine

M.P. 248-249°C (HCl salt, dec).
¹H NMR (CDCl₃) δ 7.18 (m, 5H), 7.07 (t, 1H, J=7 Hz), 6.90 (d, 1H, J=7 Hz), 6.72 (t, 1H, J=7 Hz), 6.60 (d, 1H, J=7 Hz), 3.77 (d, 1H, J=2 Hz), 3.60 (d, 1H, J=12 Hz), 3.38 (s, 3H), 3.34 (s, 1H, J=12 Hz), 3.14 (m, 1H), 2.77 (m, 1H), 2.32 (t, 1H, J=10 Hz), 2.02 (m, 2H), 1.18 (m, 1H), 0.81 (d, 3H, J=6 Hz). HRMS Calc'd for C₂₀H₂₆N₂O: 310.2045. Found: 310.2076. Calc'd for C₂₀H₂₆N₂O·2HCl·2/3H₂O: C, 60.75; H, 7.47; N, 7.09. Found: C, 60.78; H, 7.32; N, 6.84.

### EXAMPLE 107

### Cis-3-(2,5-dimethoxybenzylamino)-1-[4-(4-fluorophenyl)-4-oxobut-1-yl]-2-phenylpiperidine

The title compound was prepared by a procedure similar to that described in Example 101.
¹H NMR (CDCl₃) δ 1.38 (m, 2H), 1.86 (m, 6H), 2.50 (m, 2H), 1.86 (m, 6H), 2.50 (m, 2H), 2.74 (m, 2H), 3.16 (s, 1H), 3.26 (m, 2H), 3.38 (s, 3H), 3.54 (m, 4H), 6.50 (m, 3H), 7.00 (m, 2H), 7.16 (m, 5H), 7.82 (m, 2H).
HRMS Calc'd for C₃₀H₃₅N₂FO₃:490.2629. Found: 490.2633. Calc'd for C₃₀H₃₅N₂O₃F·2CH₃SO₃H·4.75H₂O: C, 50.01; H, 6.88; N, 3.64. Found: C, 49.93; H, 6.52; N, 3.56.

### EXAMPLE 108

### Cis-3-(4,5-difluoro-2-methoxybenzylamino)-2-phenylpiperidine

The title compound was prepared by a procedure similar to that described in Example 91.
¹H NMR (CDCl₃) δ 1.30 (m, 1H), 1.62 (m, 2H), 1.96 (m, 1H), 2.68 (m, 2H), 3.18 (m, 2H), 3.32 (s, 3H), 3.44 (d, 1H, J=14), 3.82 (d, 1H, J=3), 6.38 (dd, 1H, J=6,12), 6.66 (dd, 1H, J=8,10), 7.16 (m, 5H).
HRMS Calc'd for C₁₉H₂₂N₂F₂O: 332.1697. Found: 332.1698. Calcd for C₁₉H₂₂N₂OF₂·2HCl·0.85H₂O: C, 54.25; H, 6.15; N, 6.66. Found: C, 54.26; H, 5.84; N, 6.94.

### EXAMPLE 109

### Cis-3-(2-chloro-4-fluorobenzylamino)-2-phenylpiperidine

The title compound was prepared by a procedure similar to that described in Example 91.
¹H NMR (CDCl₃) δ 1.44 (m, 1H), 2.06 (m, 1H), 2.78 (m, 2H), 3.24 (m, 1H), 3.40 (d, 1H, J=12), 3.58 (d, 1H, J=12), 3.88 (d, 1H, J=3), 6.75 (m, 1H), 6.92 (m, 2H), 7.26 (m, 5H).
HRMS Calc'd for C₁₈H₂₀N₂³⁵ClF:318.1294. Found: 318.1280.

### EXAMPLE 110

### Cis-3-(2-ethoxybenzylamino)-2-phenylpiperidine

The title compound was prepared by a procedure similar to that described in Example 91.
¹H NMR (CDCl₃) δ 1.10 (t, 3H, J=5), 1.40 (m, 1H), 1.62 (m, 1H), 1.90 (m, 1H), 2.14 (m, 1H), 2.80 (m, 2H), 3.27 (m, 1H), 3.38 (d, 1H, J=15), 3.69 (m, 3H), 3.86 (d, 1H, J=2), 6.64 (d, 1H, J=8), 6.78 (t, 1H, J=6), 6.94 (d, 1H, J=6), 7.12 (t, 1H, J=8), 7.24 (m, 5H).
HRMS Calc'd for C₂₀H₂₆N₂O:310.2041. Found: 310.2045.

### EXAMPLE 111

### Cis-3-(2-hydroxybenzylamino)-2-phenylpiperidine

The title compound was prepared by a procedure similar to that described in Example 91.
¹H NMR (CDCl₃) δ 1.62 (m, 3H), 2.10 (m, 1H), 2.79 (m, 1H), 2.92 (m, 1H), 3.20 (m,1H), 3.48 (s,2H), 3.82 (d, 1H, J=2), 6.72 (m, 3H), 7.08 (m, 1H), 7.36 (m, 5H).
HRMS Calc'd for C₁₈H₂₂N₂O:282.1732. Found: 282.1724. Calcd for C₁₈H₂₂N₂O·2HCl·2H₂O: C, 55.26, H, 7.20; N, 7.16. Found: C, 55.13; H, 7.12; N, 6.84.

### EXAMPLE 112

### Cis-3-(3,5-difluoro-2-methoxybenzylamino)-2-phenylpiperidine

The title compound was prepared by a procedure similar to that described in Example 91.
¹H NMR (CDCl₃) δ 1.45 (m, 1H), 1.64 (m, 1H), 1.86 (m, 1H), 2.08 (m, 1H), 2.80 (m, 2H), 3.24 (m, 1H), 3.44 (d, 1H, J=15), 3.54 (d, 1H, J = 15), 3.68 (s, 3H) 3.90 (d, 1H, J=3), 6.57 (dd, 1H, J = 8, 9), 6.69 (dd, 1H, J=9, 12), 7.28 (m, 5H).
HRMS Calc'd for C₁₉H₂₂N₂OF₂:332.1698. Found: 332.1700. Calc'd for C₁₉H₂₂N₂OF₂·2HCl:C, 56.30; H, 5.97; N, 6.92. Found: C, 56.17; H, 5.84; N, 6.59.

### EXAMPLE 113

### Cis-3-(2-chloro-6-fluorobenzylamino)-2-phenylpiperidine

The title compound was prepared by a procedure similar to that described in Example 91.
¹H NMR (CDCl₃) δ 1.40 (m, 1H), 1.66 (m, 1H), 1.90 (m, 1H), 2.15 (m, 1H), 2.78 (m, 2H), 3.26 (m, 1H), 3.68 (d, 2H, J=18), 3.72 (d, 1H, J=18), 6.82 (m, 1H), 7.04 (m, 2H), 7.22 (m, 5H).
HRMS Calc'd for C₁₈H₂₀N₂ClF·2HCl·2/3H₂O: C, 53.56; H, 5.83; N, 6.95. Found: C, 53.63; H, 5.53; N, 6.83.

### EXAMPLE 114

### (2S,3S)-3-(5-chloro-2-methoxybenzylamino)-2-phenylpiperidine

The title compound was prepared by a procedure similar to that described in Example 91.
Mp 275-277°C (HCl salt).
¹H NMR (CDCl₃) δ 1.40 (m, 1H), 1.60 (m, 1H), 1.90 (m, 1H), 2.08 (m, 1H), 2.79 (m, 2H), 3.26 (m, 1H), 3.36 (d, 1H, J=15), 3.45 (s, 3H), 3.60 (d, 1H, J=15), 3.88 (d, 1H, J=3), 6.56 (d, 1H, J=8), 6.92 (d, 1H, J=3), 7.06 (dd, 1H, J=3, 8), 7.28 (m, 5H).
Mass spectrum: m/z 330 (parent).

### EXAMPLE 115

### Cis-3-(5-chloro-2-methoxybenzylamino)-2-phenylpiperidine

The title compound was prepared by a procedure similar to that described in Example 91.
¹H NMR (CDCl₃) δ 1.37 (m, 1H), 1.56 (m, 1H), 1.86 (m, 1H), 2.06 (m, 1H), 2.76 (m, 2H), 3.23 (m, 1H), 3.32 (d, 1H, J=15), 3.42 (5, 3H), 3.58 (d, 1H, J=15), 3.85 (d, 1H, J=3), 6.54 (d, 1H, J=8), 6.90 (d, 1H, J=3), 7.04 (dd, 1H, J=3,8), 7.24 (m, 5H).

### EXAMPLE 116

### (2S,3S)-1-(5-acetamidopent-1-yl)-3-(2-methoxybenzylamino)-2-phenylpiperidine

The title compound was prepared by a procedure similar to that described in Example 95.
¹H NMR (CDCl₃) δ 1.14 (m, 2H), 1.40 (m, 5H), 1.86 (m, 1H), 1.91 (s, 3H), 2.00 (m, 3H), 2.52 (m, 2H), 3.12 (m, 3H), 3.22 (d, 1H, J=3), 3.34 (d, 1H, J=15), 3.42 (s, 3H), 3.62 (d, 1H, J=15), 6.60 (d, 1H, J=9), 6.70 (t, 1H, J=9), 6.82 (d, 1H, J=6), 7.06 (t, 1H, J=6), 7.22 (m, 5H).
HRMS Calc'd for C₂₆H₃₇N₃O₂:423.2885. Found: 423.2869.

### EXAMPLE 117

### (2S,3S)-1-(5-aminopent-1-yl)-3-(2-methoxybenzylamino)-2-phenylpiperidine

The title compound was prepared by a procedure similar to that described in Example 94.
¹H NMR (CDCl₃) δ 1.30 (m, 7H), 1.76 (m, 3H), 2.02 (m, 3H), 2.54 (m, 3H), 3.02 (m, 1H), 3.28 (d, 1H, J=3), 3.36 (d, 1H, J=15), 3.46 (s, 3H), 3.66 (d, 1H, J=15), 6.60 (d, 1H, J=6), 6.72 (t, 1H, J=6), 6.83 (d, 1H, J=6), 7.08 (t, 1H, J=6), 7.24 (m, 5H).
HRMS Calc'd for C₂₄H₃₅N₃O:381.2780. Found: 381.2755.

### EXAMPLE 118

### (2S,3S)-1-(5-benzamidopent-1-yl)-3-(2-methoxybenzylamino)-2-phenylpiperidine

The title compound was prepared by a procedure similar to that described in Example 95.
¹H NMR δ 1.40 (m, 8H), 1.96 (m, 5H), 2.54 (m, 2H), 3.34 (m, 7H), 3.80 (m, 1H), 6.61 (d, 1H, J=9), 6.76 (t, 1H, J=6), 6.88 (d, 1H, J=9), 7.12 (t, 1H, J=6), 7.26 (m, 5H), 7.40 (m, 3H), 7.78 (d, 2H, J=6).
HRMS Calc'd for C₃₁H₃₉N₃O₂:485.3042. Found: 485.3001.

### EXAMPLE 119

### (2S,3S)-1-(6-hydroxyhex-1-yl)-3-(2-methoxybenzylamino)-2-phenylpiperidine

The title compound was prepared by a procedure similar to that described in Example 98.
¹H NMR (CDCl₃) δ 1.22 (m, 4H), 1.48 (m, 4H), 1.84 (m, 1H), 2.10 (m, 5H), 2.54 (m, 1H), 2.62 (d, 1H, J=3), 3.26 (m, 1H), 3.32 (d, 1H, J=3), 3.39 (d, 1H, J=15), 3.48 (s, 3H), 3.60 (t, 2H, J=6), 3.70 (d, 1H, J=15), 6.66 (d, 1H, J=9), 6.78 (t, 1H, J=6), 6.89 (d, 1H, J=6), 7.14 (t, 1H, J=9), 7.28 (m, 5H).
HRMS Calc'd for C₂₅H₃₆N₂O₂:396.2777. Found: 396.2738.

### EXAMPLE 120

### (2S,3S)-1-(5-carboethoxypent-1-yl)-3-(2-methoxybenzylamino)-2-phenylpiperidine

The title compound was prepared by a procedure similar to that described in Example 97.
¹H NMR (CDCl₃) δ 1.14 (m, 5H), 1.48 (m, 6H), 1.96 (m, 4H), 2.20 (t, 2H, J=7), 2.74 (m, 2H), 3.19 (m, 1H), 3.26 (d, 1H, J=3), 3.34 (d, 1H, J=15), 3.44 (s, 3H), 3.64 (d, 1H, J=15), 4.06 (q, 2H, J=6), 6.61 (d, 1H, J=9), 6.72 (t, 1H, J=6), 6.83 (d, 1H, J=6), 7.08 (t, 1H, J=9), 7.22 (m, 5H).
HRMS Calc'd for C₂₇H₃₈N₂O₃:438.2879. Found: 438.2839.

### EXAMPLE 121

### Cis-1-(5-hydroxypent-1-yl)-3-(2-methoxybenzylamino)-2-phenylpiperidine

The title compound was prepared by a procedure similar to that described in Example 98.
¹H NMR (CDCl₃) δ 1.08 (m, 2H), 1.22 (m, 4H), 1.72 (m, 3H), 2.00 (m, 3H), 2.48 (m, 1H), 2.54 (m, 1H), 3.16 (m, 1H), 3.25 (d, 1H, J=3), 3.32 (d, 1H, J=15), 3.42 (s, 3H), 3.52 (t, 2H, J=6), 3.62 (d, 1H, J=15), 6.58 (d, 1H, J=9), 6.69 (t, 1H, J=6), 6.81 (d, 1H, J=6), 7.05 (t, 1H, J=9), 7.22 (m, 5H).
HRMS Calc'd for C₂₄H₃₄N₂O₂:382.2616. Found: 382.2565.

### EXAMPLE 122

### Cis-1-(4-carboethoxybut-1-yl)-3-(2-methoxybenzylamino)-2-phenylpiperidine

The title compound was prepared by a procedure similar to that described in Example 97.
¹H NMR (CDCl₃) δ 1.22 (t, 3H, J=6), 1.46 (m, 4H), 1.74 (m, 3H), 2.02 (m, 3H), 2.16 (m, 2H), 2.54 (m, 2H), 3.28 (m, 1H), 3.26 (m, 1H), 3.34 (d, 1H, J=15), 3.46 (s, 3H), 3.62 (d, 1H, J=15), 4.06 (q, 2H, J=6), 6.61 (d, 1H, J=9), 6.72 (t, 1H, J=6), 6.83 (d, 1H, J=6), 7.08 (t, 1H, J=9), 7.28 (m, 5H).
HRMS Calc'd for C₂₆H₃₆N₂O₃:424.2723. Found: 424.2734.

### EXAMPLE 123

### Cis-1-(5-carboxypent-1-yl)-3-(2-methoxybenzylamino)-2-phenylpiperidine

The title compound was prepared by a procedure similar to that described in Example 99.
M.p. 53-65°C.
¹H NMR (DMSO-d₆) δ 1.10 (m, 2H), 1.36 (m, 2H), 1.70 (m, 3H), 2.30 (m, 10H), 2.96 (m, 1H), 3.70 (m, 2H), 3.90 (m, 2H), 4.20 (m, 1H), 6.98 (m, 2H), 7.38 (m, 2H), 7.60 (m, 3H), 7.90 (m, 2H). Mass spectrum: m/z 410 (parent).

### EXAMPLE 124

### Cis-1-(3-hydroxyprop-1-yl)-3-(2-methoxybenzylamino)-2-phenylpiperidine

The title compound was prepared by a procedure similar to that described in Example 98.
M.p. 93-96°C (dec).
¹H NMR (CDCl₃) δ 1.34 (m, 3H), 2.00 (m, 4H), 2.65 (m, 1H), 2.76 (m, 1H), 3.31 (m, 3H), 3.38 (d, 1H, J=15), 3.51 (s, 3H), 3.62 (d, 1H, J=15), 3.74 (m, 2H), 6.64 (d, 1H, J=9), 6.73 (t, 1H, J=6), 6.88 (d, 1H, J=6), 7.08 (t, 1H, J=9), 7.30 (m, 5H).
Mass spectrum: m/z 354 (parent). Calc'd for C₂₂H₃₀N₂O₂·2HCl·2.65H₂O: C, 55.61; H, 7.90; N, 5.89. Found: C, 55.62; H, 7.75; N, 5.67.

### EXAMPLE 125

### Cis-1-(3-carboxyprop-1-yl)-3-(2-methoxybenzylamino)-2-phenylpiperidine

The title compound was prepared by a procedure similar to that described in Example 99.
M.p. 100-105°C (dec).
¹H NMR (DMSO-d₆) δ 1.92 (m, 3H), 2.20 (m, 6H), 3.46 (m, 4H), 3.78 (m, 3H), 4.00 (m, 3H), 6.94 (m, 2H), 7.36 (m, 2H), 7.56 (m, 3H), 7.86 (m, 2H).
Mass spectrum: m/z 382 (parent).

### EXAMPLE 126

### Cis-1-(2-carboethoxyeth-1-yl)-3-(2-methoxybenzylamino)-2-phenylpiperidine

The title compound was prepared by a procedure similar to that described in Example 97.
M.p. 110-112°C.
¹H NMR (CDCl₃) δ 1.18 (t, 3H, J=6), 1.46 (m, 2H), 1.62 (m, 1H), 2.08 (m, 3H), 2.26 (m, 1H), 2.42 (m, 2H), 2.60 (m, 1H), 2.90 (m, 1H), 3.16 (m, 1H), 3.36 (d, 1H, J=15), 3.45 (s, 3H), 3.66 (d, 1H, J=15), 4.04 (q, 2H, J=6), 6.62 (d, 1H, J=9), 6.74 (t, 1H, J=6), 6.85 (d, 1H, J=6), 7.08 (t, 1H, J=9) 7.28 (m, 5H).
Mass spectrum: m/z 396 (parent).

### EXAMPLE 127

### Cis-1-(3-carboethoxyprop-1-yl)-3-(2-methoxybenzylamino)-2-phenylpiperidine

The title compound was prepared by a procedure similar to that described in Example 97.
M.p. 75-90°C.
¹H NMR (CDCl₃) δ 1.14 (t, 3H, J=6), 1.42 (m, 2H), 1.74 (m, 3H), 2.08 (m, 5H), 2.50 (m, 2H), 3.17 (m, 1H), 3.24 (d, 1H, J=3), 3.30 (d, 1H, J=1), 3.42 (s, 3H), 3.60 (d, 1H, J=15), 4.00 (q, 2H, J=6), 6.58 (d, 1H, J=9), 6.68 (t, 1H, J=6), 6.81 (d, 1H, J=6), 7.04 (t, 1H, J=9), 7.22 (m, 5H).
Mass spectrum: m/z 410 (parent).

### EXAMPLE 128

### Cis-1-(4-hydroxybut-1-yl)-3-(2-methoxybenzylamino)-2-phenylpiperidine

The title compound was prepared by a procedure similar to that described in Example 98.
¹H NMR (CDCl₃) δ 1.80 (m, 10H), 2.50 (m, 1H), 2.64 (m, 1H), 3.26 (m, 1H), 3.44 (m, 6H), 3.66 (m, 2H), 6.60 (d, 1H, J=9), 6.70 (t, 1H, J=9), 6.94 (d, 1H, J=6), 7.06 (t, 1H, J=6), 7.30 (m, 5H).
Mass spectrum: m/z 368 (parent). Calc'd for C₂₅H₃₄N₂O₃·2HCl·3/4 H₂O: C, 60.71; H, 7.86; N, 6.16. Found: C, 60.75; H, 7.55; N, 6.05.

### EXAMPLE 129

### Cis-1-(hex-1-yl)-3-(2-methoxybenzylamino)-2-phenylpiperidine

The title compound was prepared by a procedure similar to that described in Example 2.
M.p. 48-50°C.
¹H NMR (CDCl₃) δ 0.85 (t, 3H, J=7), 1.15 (m, 7H), 1.50 (m, 3H), 2.05 (m, 4H), 2.55 (m, 1H), 2.60 (m, 1H), 3.30 (m, 1H), 3.50 (m, 5H), 3.80 (d, 1H, J=15), 6.65 (d, 1H, J=7), 6.80 (t, 1H, J=7), 6.93 (d, 1H, J=7), 7.13 (t, 1H, J=7), 7.35 (m, 5H).
HRMS Calc'd for C₂₅H₃₆N₂O:380.2827. Found: 380.2808.

### EXAMPLE 130

### Cis-3-(2-methoxybenzylamino)-2-phenyl-1-(6-phenylhex-1-yl)piperidine

The title compound was prepared by a procedure similar to that described in Example 2.
M.p. 48-53°C.
¹H NMR (CDCl₃) δ 1.25 (m, 4H), 1.55 (m, 5H), 1.90 (m, 1H), 2.05 (m, 6H), 2.55 (m, 2H), 2.70 (m, 1H), 3.35 (m, 2H), 3.50 (s, 3H), 3.80 (m, 1H), 6.65 (s, 1H), 6.80 (s, 1H), 7.20 (m, 3H), 7.30 (m, 8H).
HRMS Calc'd for C₃₁H₄₀N₂O·2HCl·3.2H₂O: C, 63.40; H, 4.77; N, 7.95. Found: C, 63.40; H, 4.71; N, 7.89.

### EXAMPLE 131

### Cis-3-(2-methoxybenzylamino)-2-phenyl-1-(7-phenylhept-1-yl)piperidine

The title compound was prepared by a procedure similar to that described in Example 2.
M.p. 67-77°C.
¹H NMR (CDCl₃) δ 1.15 (m, 5H), 1.50 (m, 4H), 1.90 (m, 1H), 2.10 (m, 7H), 2.50 (m, 3H), 3.40 (m, 2H), 3.45 (s, 3H), 3.80 (m, 2H), 6.65 (t, 1H, J=8), 6.75 (t, 1H, J=8), 6.90 (d, 1H, J=8), 7.30 (m, 11H).
HRMS Calc'd for C₃₃H₄₄NO:470.3297. Found: 470.3281.

### EXAMPLE 132

### Cis-3-(4-methoxybenzylamino)-2-phenylpiperidine

The title compound was prepared by a procedure similar to that described in Example 91.
M.p. 264-266°C.
¹H NMR (CDCl₃) δ 1.28-1.40 (m, 1H), 1.44-1.88 (m, 2H), 1.92-2.02 (m, 1H), 2.64-2.84 (m, 2H), 3.10-3.22 (m, 1H), 3.19 (d, 1H, J=12), 3.39 (d, 1H, J=12), 3.70 (s, 3H), 3.81 (d, 1H, J=3), 6.65 (d, 2H, J=8), 6.83 (d, 2H, J=6), 7.12-7.28 (m, 5H).
HRMS Calc'd for C₁₉H₂₄N₂O:296.1885. Found: 296.1871. Anal. Calc'd for C₁₉H₂₄N₂O·2HCl·0.6H₂O: C, 60.03; H, 7.21; N, 7.37. Found: 60.08; H, 7.11; N, 7.45.

### EXAMPLE 133

### Cis-2-phenyl-3-(thien-2-ylmethylamino)piperidine

The title compound was prepared by a procedure similar to that described in Example 91.
M.p. 250-252°C.
¹H NMR (CDCl₃) δ 1.30-1.40 (m, 1H), 1.46-1.52 (m, 1H), 1.68-1.86 (m, 1H), 1.92-2.00 (m, 1H), 2.64-2.78 (m, 1H), 2.84-2.92 (m, 1H), 3.12-3.22 (m, 1H), 3.44 (d, 1H, J=12), 3.54 (d, 1H, J=12), 3.81 (d, 1H, J=3), 6.53 (d, 1H, J=4), 6.72-6.80 (m, 1H), 7.02 (d, 1H, J=6), 7.12-7.30 (m, 5H).
HRMS Calc'd for C₁₆H₂₀N₂S:272.1373. Found: 272.1327. Anal. Calc'd for C₁₆H₂₀N₂S·2HCl·1.1H₂O: C, 52.62, H, 6.67; N, 7.67. Found: C, 52.64; H, 6.38, N, 7.65.

### EXAMPLE 134

### Cis-3-(2-methoxynaphth-1-ylmethylamino)-2-phenylpiperidine

The title compound was prepared by a procedure similar to that described in Example 91.
M.p. 222-225°C.
¹H NMR (CDCl₃) δ 1.36-1.48 (m, 1H), 1.52-2.04 (m, 2H), 2.18-2.32 (m, 1H), 2.68-2.82 (m, 1H), 2.90 (d, 1H, J=3), 3.18-3.28 (m, 1H), 3.64 (s, 3H), 3.80 (d, 1H, J=12), 3.86 (d, 1H, J=4), 4.07 (d, 1H, J=12), 7.02-7.32 (m, 8H), 7.57 (d, 1H, J=8), 7.60-7.70 (m, 2H).
HRMS Calc'd for C₂₃H₂₆N₂O:346.2041. Found: 346.2043.

### EXAPLE 135

### Cis-2-phenyl-3-(thien-3-ylmethylamino)piperidine

The title compound was pepared by a procedure similar to that described in Example 91.
M.p. 264-267°C.
¹H NMR (CDCl₃) δ 1.30-1.40 (m, 1H), 1.46-1.64 (m, 1H), 1.70-1.88 (m, 1H), 1.92-2.02 (m, 1H), 2.68-2.78 (m, 1H), 2.80-2.88 (m, 1H), 3.14-3.22 (m, 1H), 3.31 (d, 1H, J=12), 3.48 (d, 1H, J=12), 3.84 (d, 1H, J=3), 6.65 (d, 1H, J=6), 6.72 (d, 1H, J=3), 7.04-7.10 (m, 1H), 7.14-7.28 (m, 5H).
HRMS Calc'd for C₁₆H₂₀N₂S:272.1342. Found: 272.1364. Anal. Calc'd for C₁₆H₂₀N₂S·2HCl·0.6H₂O:C, 53.96; H, 6.57; N, 7.87. Found: C, 53,97; H, 6.25; N, 7.77.

### EXAMPLE 136

### Cis-3-(2,5-difluorobenzylamino)-2-phenylpiperidine

The title compound was prepared by a procedure similar to that described in Example 91.
M.p. 274-276°C.
¹H NMR (CDCl₃) δ 1.28-1.40 (m, 1H), 1.44-1.62 (m, 1H), 1.66-1.84 (m, 1H), 1.90-2.00 (m, 1H), 2.64-2.76 (m, 2H), 2.10-3.20 (m, 1H), 3.32 (d, 1H, J=12), 3.44 (d, 1H, J=12), 3.81 (d, 1H, J=3), 6.50-6.58 (m, 1H), 6.62-6.78 (m, 2H), 7.10-7.26 (m, 5H).
HRMS Calc'd for C₁₈H₂₀N₂F₂:302.1590. Found: 302.1560. Anal. calc'd for C₁₈H₂₀N₂F₂·2HCl·0.2H₂O:C, 57.06; H, 5.96; N, 7.39. Found: C, 56.94; H, 5.94; N, 7.37.

### EXAMPLE 137

### Cis-3-(3-methoxybenzylamino)-2-phenylpiperidine

The title compound was prepared by a procedure similar to that described in Example 92.
M.p. 243-246°C.
¹H NMR (CDCl₃) δ 1.32-1.42 (m, 1H), 1.48-1.90 (m, 2H), 1.96-2.04 (m, 1H), 2.68-2.78 (m, 1H), 2.85 (d, 1H, J=4), 3.16-3.26 (m, 1H), 3.29 (d, 1H, J=12), 3.46 (d, 1H, J=12), 3.68 (s, 3H), 3.85 (d, 1H, J=3), 6.50-6.58 (m, 2H), 6.62-6.68 (m, 1H), 7.04 (t, 1H, J=8), 7.16-7.38 (m, 5H).
HRMS Calc'd for C₁₉H₂₄N₂O:296.1885. Found: 296.1873. Anal. Calc'd for C₁₉H₂₄N₂O·2HCl·0.3H₂O:C, 60.89; H, 6.75; N, 7.48. Found: C, 60.72; H, 6.84; N, 7.27.

### EXAMPLE 138

### (2S,3S)-1-(4-oxo-4-phenylbut-1-yl)-3-(2-methoxybenzylamino)-2-phenylpiperidine

The title compound was prepared by a procedure similar to that described in Example 101.
M.p. 217-219°C.
¹H NMR (CDCl₃) δ 1.32-1.56 (m, 2H), 1.68-2.20 (m, 6H), 2.48-2.64 (m, 2H), 2.68-3.00 (m, 2H), 3.20-3.28 (m, 1H), 3.31 (d, 1H, J=4), 3.36 (d, 1H, J=15), 3.44 (s, 3H), 3.65 (d, 1H, J=15), 6.61 (d, 1H, J=7), 6.72 (t, 1H, J=6), 6.84 (t, 1H, J=6), 7.08 (t, 1H, J=8), 7.10-7.30 (m, 6H), 7.40 (t, 1H, J=6), 7.50 (d, 1H, J=6), 7.87 (d, 2H, J=6).
HRMS Calc'd for C₂₉H₃₄N₂O₂:442.2616. Found: 442.2577.

### EXAMPLE 139

### (2S,3S)-1-(4-hydroxy-4-phenylbut-1-yl)-3-(2-methoxybenzylamino)-2-phenylpiperidine

The title compound was prepared by a procedure similar to that described in Example 102.
M.p. 190-194°C.
¹H NMR (CDCl₃) δ 1.40-2.24 (m, 10H), 2.42-2.66 (m, 1H), 2.74-2.84 (m, 1H), 3.02-3.14, 3.30-3.40 (2m, 1H), 3.44-3.62 (m, 5H), 3.66-3.82 (m, 1H), 4.50 (br s, 2H), 4.62-4.70, 4.76-4.82 (2m, 1H), 6.68 (d, 1H, J=8), 6.74-6.82 (m, 1H), 6.98 (t, 1H, J=6), 7.08-7.18 (m, 1H), 7.20-7.62 (m, 10H).
HRMS Calc'd for C₂₉H₃₆N₂O₂:444.2772. Found: 444.2745. Anal. Calc'd for C₂₉H₃₆N₂O₂·2HCl·3H₂O:C, 64.38; H, 7.56; N, 5.18. Found: C, 64.27; H, 7.31; N, 5.15.

### EXAMPLE 140

### Cis-3-(2,5-dimethoxybenzylamino)-2-phenylpiperidine

The title compound was prepared by a procedure similar to that described in Example 91.
M.p. 190-194°C.
¹H NMR (CDCl₃) δ 1.28-1.40 (m, 1H), 1.48-1.92 (m, 2H), 2.02-2.14 (m, 1H), 2.66-2.80 (m, 2H), 3.14-3.24 (m, 1H), 3.32 (d, 1H, J=18H), 3.38 (s, 3H), 3.56 (d, 1H, J=18H), 3.66 (s, 3H), 3.83 (d, 1H, J=3H), 6.48-6.62 (m, 3H), 7.10-7.26 (m, 5H).
HRMS Calc'd for C₂₀H₂₆N₂O₂:326.1995. Found: 326.1959. Anal. Calc'd for C₂₀H₂₆N₂O₂·2HCl·0.3H₂O:C, 59.34; H, 7.12; N, 6.92. Found:, C, 59.33; H, 6.96; N, 6.76.

### EXAMPLE 141

### Cis-3-(3-fluoro-4-methoxybenzylamino)-2-phenylpiperidine

The title compound was prepared by a procedure similar to that described in Example 92.
M.p. 272-274°C.
¹H NMR (CDCl₃) δ 1.34-2.04 (m, 4H), 2.68-2.82 (m, 2H), 3.12-3.26 (m, 1H), 3.22 (d, 1H, J=12), 3.40 (d, 1H, J=12), 3.82 (s, 3H), 3.85 (d, 1H, J=4), 6.60-6.76 (m, 3H), 7.10-7.32 (m, 5H).
HRMS Calc'd. for C₁₉H₂₃FN₂O:314.1791. Found: 314.1773. Anal. Calc'd for C₁₉H₂₃FN₂O·2HCl·1.1H₂O:C, 56.05; H, 6.73; N, 6.88. Found: C, 55.96; H, 6,48; N, 6.71.

### EXAMPLE 142

### Cis-3-(5-fluoro-2-methoxybenzylamino)-2-phenylpiperidine

The title compound was prepared by a procedure similar to that described in Example 91.
M.p. 270-272°C.
¹H NMR (CDCl₃) δ 1.30-1.42 (m, 1H), 1.48-2.12 (m, 3H), 2.64-2.82 (m, 2H), 3.12-3.26 (m, 1H), 3.32 (d, 1H, J=12), 3.42 (s, 3H), 3.56 (d, 1H, J=12), 3.84 (d, 1H, J=3), 6.53 (dd, 1H, J=5, 10), 6.64 (dd, 1H, J=3, 8), 6.70-6.80 (m, 1H), 7.12-7.40 (m, 5H).
HRMS Calc'd for C₁₉H₂₃FN₂O:314.1791. Found: 314.1766. Anal. Calc'd for C₁₉H₂₃FN₂O·2HCl·O.5H₂O:C, 57.58; H, 6.61; N, 7.07. Found: C, 57.35; H, 6.36; N, 7.03.

### EXAMPLE 143

### Cis-3-(5-chloro-2-methoxybenzylamino)-2-phenylpiperidine

The title compound was prepared by a procedure similar to that described in Example 91.
M.p. 270-273°C.
¹H NMR (CDCl₃) δ 1.32-1.42 (m, 1H), 1.50-2.12 (m, 3H), 2.68-2.82 (m, 2H), 3.18-3.28 (m, 1H), 3.32 (d, 1H, J=12), 3.42 (s, 3H), 3.58 (d, 1H, J=12), 3.85 (d, 1H, J=4), 6.54 (d, 1H, J=8), 6.90 (d, 1H, J=3), 7.04 (dd, 1H, J=3, 8), 7.12-7.32 (m, 5H).
HRMS Calc'd for C₁₉H₂₃ClN₂0:330.1495. Found: 330.1491. Anal. Calc'd for C₁₉H₂₃ClN₂O:330.1495. Found: 330.1491. Anal. calcd for C₁₉H₂₃ClN₂O·2HCl·O.4H₂O:C, 55.52; H, 6.33; N, 6.82. Found: C, 55.52; H, 6.33; N, 6.82. Found: C, 55.53; H, 6.10; N, 6.70.

### EXAMPLE 144

### Cis-3-(5-chloro-2-methoxybenzylamino)-1-(5,6-dihydroxyhex-1-yl)-2-phenylpiperidine

The title compound was prepared by a procedure similar to that described in Example 104.
¹H NMR (CDCl₃) δ 1.34 (m, 8H), 1.78 (m, 1H), 2.00 (m, 3H), 2.54 (m, 2H), 3.32 (m, 3H), 3.44 (s, 3H), 3.54 (m, 3H), 6.52 (d, 1H, J=9), 6.80 (br s, 1H), 7.02 (m, 1H), 7.22 (m, 5H).
Mass spectrum: m/z 446 (parent).

### EXAMPLE 145

### Cis-1-(5,6-dihydroxyhex-1-yl)-3-(2,5-dimethoxybenzylamino)-2-phenylpiperidine

The title compound was prepared by a procedure similar to that described in Example 104.
¹H NMR (CDCl₃) δ 1.38 (m, 8H), 1.78 (m, 1H), 2.00 (m, 3H), 2.50 (m, 1H), 2.60 (m, 1H), 3.30 (m, 3H), 3.40 (s, 3H), 3.60 (m, 4H), 3.65 (s, 3H), 6.56 (m, 3H), 7.26 (m, 5H).
Mass spectrum: m/z 442 (parent).

### EXAMPLE 146

### Cis-2-phenyl-3-[2-(prop-2-yloxy)benzylamino]piperidine

The title compound was prepared by a procedure similar to that described in Example 91.
¹H NMR (CDCl₃) δ 1.00 (m, 6H), 1.30 (m, 1H), 1.70 (m, 2H), 2.10 (m, 1H), 2.72 (m, 2H), 3.18 (m, 1H), 3.30 (m, 1H), 3.50 (m, 1H), 3.80 (br s, 1H), 4.06 (m, 1H), 6.66 (m, 2H), 6.90 (m, 1H), 7.05 (m, 1H), 7.20 (m, 5H).
HRMS Calc'd for C₂₁H₂₈N₂O:324.2197. Found: 324.2180. Calc'd for C₂₁H₂₈N₂O·2HCl·1.66H₂O:C, 59.02; H, 7.85; N, 6.55. Found: C, 59.07; H, 7.77; N, 6.69.

### EXAMPLE 147

### Cis-3-(3-fluoro-2-methoxybenzylamino)-2-phenylpiperidine

The title compound was prepared by a procedure similar to that described in Example 91.
¹H NMR (CDCl₃) δ 1.40 (m, 1H), 1.60 (m, 1H), 1.86 (m, 1H), 2.08 (m, 1H), 2.80 (m, 2H), 3.23 (m, 1H), 3.36 (m, 1H), 3.58 (m, 4H), 3.88 (m, 1H), 6.80 (m, 3H), 7.26 (m, 5H).
HRMS Calc'd for C₁₉H₂₃N₂OF:314.1794. Found: 314.1768. Calc'd for C₁₉H₂₃N₂OF·2HCl·1.5H₂O:C, 55.08; H, 6.80; N, 6.76. Found: C, 54.89; H, 6.48; N, 6.79.

### EXAMPLE 148

### Cis-3-(5-chloro-3-fluoro-2-methoxybenzylamino)-2-phenylpiperidine

The title compound was prepared by a procedure similar to that described in Example 91.
¹H NMR (CDCl₃) δ 1.42 (m, 1H), 1.54 (m, 1H), 1.80 (m, 1H), 2.06 (m, 1H), 2.78 (m, 2H), 3.20 (m, 1H), 3.42 (d, 1H, J=15), 3.58 (d, 1H, J=15), 3.64 (s, 3H), 3.86 (m, 1H), 6.66 (d, 1H, J=9), 6.91 (d, 1H, J=9), 7.26 (m, 5H).
HRMS Calc'd for C₁₉H₂₂N₂OClF:348.1401. Found: 348.1406.

### EXAMPLE 149

### Cis-3-(3-chloro-5-fluoro-2-methoxybenzylamino)-2-phenylpiperidine

The title compound was prepared by a procedure similar to that described in Example 91.
¹H NMR (CDCl₃) δ 1.44 (m, 1H), 1.58 (m, 1H), 1.80 (m, 1H), 2.06 (m, 1H), 2.80 (m, 2H), 3.22 (m, 1H), 3.42 (d, 1H, J=18), 3.54 (d, 1H, J=18), 3.66 (s, 3H), 3.88 (d, 1H, J=2), 6.55 (d, 1H, J=6), 6.92 (d, 1H, J=9), 7.26 (m, 5H).
HRMS Calc'd for C₁₉H₂₂N₂OClF:348.1401. Found: 348.1411. Calc'd for C₁₉H₂₂N₂OClF·2HCl·0.25H₂O:C, 53.53; H, 5.79; N, 6.57. Found: C, 53.58; H, 5.60; N, 6.41.

### EXAMPLE 150

### Cis-3-(3,5-dichloro-2-methoxybenzylamino)-2-phenylpiperidine

The title compound was prepared by a procedure similar to that described in Example 91.
¹H NMR (CDCl₃) δ 1.44 (m, 1H), 1.56 (m, 1H), 1.82 (m, 1H), 2.08 (m, 1H), 2.80 (m, 2H), 3.20 (m, 1H), 3.50 (m, 2H), 3.64 (s, 3H), 3.88 (m, 1H), 6.68 (s, 1H), 7.26 (m, 6H).
HRMS Calc'd for C₁₉H₂₂N₂OCl₂:364.1105. Found: 364.1105. Calc'd for C₁₉H₂₂N₂OCl₂·2HCl:C, 52.07; H, 5.52; N, 6.39. Found: C, 51.69; H, 5.50; N, 6.32.

### EXAMPLE 151

### Cis-3-(2-Methoxybenzylamino)-4-methyl-2-phenylpiperidine

The title compound was prepared by a procedure similar to that of Example 1.
¹H NMR (CDCl₃) δ 7.10 (m, 6H), (d, 1H, J=7 Hz), 6.68 (t, 1H, J=7 Hz), 6.68 (t, 1H, J=7 Hz), 6.55 (d, 1H, J=7 Hz), 3.97 (d, 1H, J=2 Hz), 3.56 (d, 1H, J=14 Hz), 3.34 (s, 3H), 3.28 (d, 1H, J=14 hz), 2.90 (m, 2H), 2.36 (s, 1H), 2.16 (s, 1H), 2.04 (s, 1H), 1.12 (m, 1H), 1.06 (d, 3H, J=6 Hz). HRMS Calc'd for C₂₀H₂₆N₂O:310.2045. Found: 310.2035.

### EXAMPLE 152

### (2S,3S)-1-(4-Oximino-4-phenylbut-1-yl)-3-(2-methoxybenzylamino)-2-phenylpiperidine

Under a nitrogen atmosphere in a round bottom flask were placed 445 mg (1 mmol) of (2S,3S)-1-(4-oxo-4-phenylbut-1-yl)-3-(2-methoxybenzylamino)-2-phenylpiperidine and 6 ml of ethanol. To the system were added 209 mg (3.2 mmol) of hydroxylamine hydrochloride and 417 mg (5 mmol) of sodium acetate in 6 ml of H₂O, and the mixture was stirred at room temperature overnight. The reaction mixture was concentrated and partitioned between chloroform and water. The layers were separated, and the aqueous phase was extracted two times with chloroform. The combined organic extracts were dried (sodium sulfate) and concentrated to 368 mg of gold oil. The crude product was purified by flash column chromatography using 7% in chloroform as the eluant to obtain 174 mg of the title compound as an oil.
¹H NMR (CDCl₃) δ 1.78 (m, 7H), 2.56 (m, 3H), 2.80 (m, 1H), 3.18 (m, 1H), 3.38 (m, 2H), 3.45 (s, 3H), 3.72 (m, 2H), 6.61 (d, 1H, J=8), 6.72 (t, 1H, J=6), 6.87 (d, 1H, J=6), 7.08 (t, 1H, J=8), 7.28 (m, 8H), 7.48 (m, 2H).

A sample of this compound was crystallized by slow evaporation from chloroform/methanol, and the structure was confirmed by single crystal x-ray analysis.

### EXAMPLE 153

### (2RS, 3RS, 6SR) and (2RS, 3RS, 6RS)-3-(2-Methoxy benzylamino)-6-methyl-2-phenylpiperidine

The title compound was prepared by a procedure similar to that described in Example 63.

More polar isomer, R_{f}∼0.28 (1:9 MeOH/CH₂Cl₂). M.p. 274-276°C (HCl salt). ¹H NMR (CDCl₃) δ 7.28-7.02 (m, 6H), 6.92 (d, 1H, J=6 Hz), 6.72 (t, 1H, J=6 Hz), 6.60 (d, 1H, J=8 Hz), 4.16 (d, 1H, J=3 Hz), 3.61 (d, 1H, J=14 Hz), 3.44-3.26 (m, 5H), 2.76 (d, 1H, J=4 Hz), 2.10-1.96 (m, 1H), 1.90-1.64 (m, 4H), 1.24∼1.08 (m, 4H).

Less polar isomer, R_{f}∼0.34 (1:9 MeOH/CH₂Cl₂). M.p. 203-206°C (HCl salt). ¹H NMR (CDCl₃) δ 7.32-7.06 (m, 6H), 6.90 (d, 1H, J=6 Hz), 6.76 (t, 1H, J=6 Hz), 6.63 (d, 1H, J=7 Hz), 3.90 (d, 1H, J=3 Hz), 3.63 (d, 1H, J=14 Hz), 3.39 (s, 3H), 3.36 (d, 1H, J=14 Hz), 2.84-2.64 (m, 2H), 2.14-2.02 (m, 1H), 1.72-1.30 (m, 5H), 1.16 (d, 3H, J=6 Hz).

### EXAMPLE 154

### (2S,3S)-3-(2-Methoxybenzylamino)-1-[4-(4-methylphenylsulfonamido)but-1-yl]-2-phenylpiperidine

The title compound was prepared by a procedure similar to that described in Example 95.
¹H NMR (CDCl₃) δ 1.40 (m, 6H), 1.85 (m, 1H), 1.96 (m, 5H), 2.39 (s, 3H), 2.60 (m, 1H), 2.83 (m, 1H), 2.83 (m, 1H), 3.14 (m, 1H), 3.26 (d, 1H, J=3), 3.41 (m, 4H), 3.68 (d, 1H, J=15), 6.60 (d, 1H, J=9), 6.69 (t, 1H, J=9), 6.80 (d, 1H, J=6), 7.06 (t, 1H, J=6), 7.22 (m, 7H), 7.68 (d, 2H, J=6). HRMS Calc'd for C₃₀H₃₉N₃O₃S:521.2708. Found: 521.2715.

### EXAMPLE 155

### (2S,3S)-1-(4-Cyanobut-1-yl)-3-(2-methoxybenzylamino)-2-phenylpiperidine

The title compound was prepared by a procedure similar to that described in Example 93.
M.P. 89-81°C (dec).
¹H NMR (CDCl₃) δ 1.48 (m, 5H), 1.90 (m, 5H), 2.20 (t, 2H, J=9), 2.52 (m, 2H), 3.18 (m, 1H), 3.06 (d, 1H, J=3), 3.32 (d, 1H, J=12), 3.40 (s, 3H), 3.68 (d, 1H, J=12), 6.58 (d, 1H, J=9), 6.70 (t, 1H, J=6), 6.82 (d, 1H, J=6), 7.06 (t, 1H J=9), 7.24 (m, 5H). HRMS Calc'd for C₂₄H₃₁N₃O:377.2467. Found: 377.2449.

### EXAMPLE 156

### Cis-3-(5-Chloro-2-methoxybenzylamino)-1-[4-(4-fluorophenyl)-4-oxobut-1-yl]-2-phenylpiperidine

The title compound was prepared by a procedure similar to that described in Example 101.
¹H NMR (CDCl₃) δ 1.48 (m, 2H), 1.96 (m, 5H), 2.58 (m, 2H), 2.81 (m, 3H), 3.28 (m, 3H), 3.45 (s, 3H), 3.62 (d, 1H, J=15), 6.52 (d, 1H, J=9), 6.82 (d, 1H, J=3), 7.05 (m, 3H), 7.26 (m, 5H), 7.88 (m, 2H).

### EXAMPLE 157

### (2S,3S)-1-(4-Acetamidobut-1-yl)-3-(2-methoxybenzylamino)-2-phenylpiperidine

The title compound was prepared by a procedure similar to that described in Example 95.
¹H NMR (CDCl₃) δ 1.42 (m, 6H), 1.82 (m, 1H), 1.94 (s, 3H), 2.00 (m, 3H), 2.50 (m, 1H), 2.59 (m, 1H), 3.06 (m, 1H), 3.20 (m, 2H), 3.27 (d, 1H, J=3), 3.34 (d, 1H, J=15), 3.42 (s, 3H), 3.66 (d, 1H, J=15), 6.61 (d, 1H, J=9), 6.72 (t, 1H, J=6), 6.83 (d, 1H, J=6), 7.09 (t, 1H, J=9), 7.26 (m, 5H). Mass spectrum: m/z 409 (parent).

### EXAMPLE 158

### (2S,3S)-1-(4-Benzamidobut-1-yl)-3-(2-methoxybenzylamino)-2-phenylpiperidine

The title compound was prepared by a procedure similar to that described in Example 95.
M.p. 146-150°C (dec).
¹H NMR (CDCl₃) δ 1.46 (m, 6H), 1.72 (m, 1H), 1.98 (m, 3H), 2.52 (m, 2H), 3.16 (m, 1H), 3.25 (d, 1H, J=3), 3.30 (m, 3H), 3.38 (s, 3H), 3.68 (m, 1H), 6.24 (br s, 1H), 6.56 (d, 1H, J=9), 6.70 (t, 1H, J=6), 7.05 (t, 1H, J=9), 7.22 (m, 5H), 7.36 (m, 3H), 7.68 (d, 2H, J=6). HRMS Calc'd for C₃₀H₃₇N₃O₂:471.2885. Found: 471.2851.

### EXAMPLE 159

### Cis-2-(3,5-Dibromophenyl)-3-(2-methoxybenzylamino)piperidine

The title compound was prepared by a procedure similar to that described in Example 1.
M.p. > 240°C (HCl salt).
¹H NMR (CDCl₃) δ 7.48 (s, 1H), 7.31 (s, 2H), 7.14 (t, 1H, J=6 Hz), 6.94 (d, 1H, J=6 Hz), 6.79 (t, 1H, J=6 Hz), 6.49 (d, 1H, J=6 Hz), 3.74 (s, 1H), 3.68 (d, 1H, J=12 Hz), 3.54 (s, 3H), 3.34 (d, 1H, J=12 Hz), 3.20 (m, 1H), 2.70 (m, 2H), 2.07 (m, 1H), 1.82 (m, 7H), 1.54 (m, 1H), 1.46 (m, 1H). HRMS Calc'd for C₁₅H₂₂N₂OBr⁷⁹Br⁸¹:454.0078. Found: 454.0143.

## Claims (Claims for the following Contracting State(s): DE, GB, FR, IT, NL, SE, CH, BE, AT, LU, DK, LI)

1. A compound of the formula 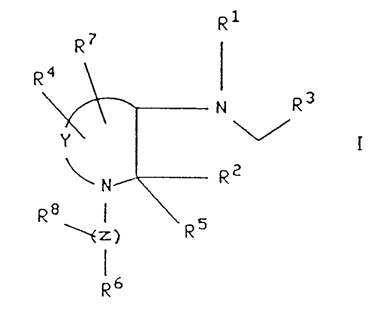 wherein Y is (CH₂)ₙ wherein n is an integer from 1 to 4, and wherein any one of the carbon-carbon single bonds in said (CH₂)ₙ may optionally be replaced by a carbon-carbon double bond, and wherein any one of the carbon atoms of said (CH₂)ₙ may optionally be substituted with R⁴, and wherein any one of the carbon atoms of said (CH₂)ₙ may optionally be substituted with R⁷;
Z is (CH₂)ₘ wherein m is an integer from 0 to 6, and wherein any one of the carbon-carbon single bonds of said (CH₂)ₘ may optionally be replaced by a carbon-carbon double bond or a carbon-carbon triple bond, and any one of the carbon atoms of said (CH₂)ₘ may optionally be substituted with R⁸;
R¹ is hydrogen or (C₁-C₈) alkyl optionally substituted with hydroxy, (C₁-C₄)alkoxy or fluoro;
R² is a radical selected from hydrogen, (C₁-C₆) straight or branched alkyl, (C₃-C₇) cycloalkyl wherein one of the CH₂ groups in said cycloalkyl may optionally be replaced by NH, oxygen or sulfur; aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; phenyl-(C₂-C₆) alkyl, benzhydryl and benzyl, wherein each of said aryl and heteroaryl groups and the phenyl moieties of said benzyl, phenyl (C₂-C₆) alkyl and benzhydryl may optionally be substituted with one or more substituents independently selected from halo, nitro, (C₁-C₆) alkyl, (C₁-C₆) alkoxy, trifluoromethyl, amino, (C₁-C₆)
alkylamino, 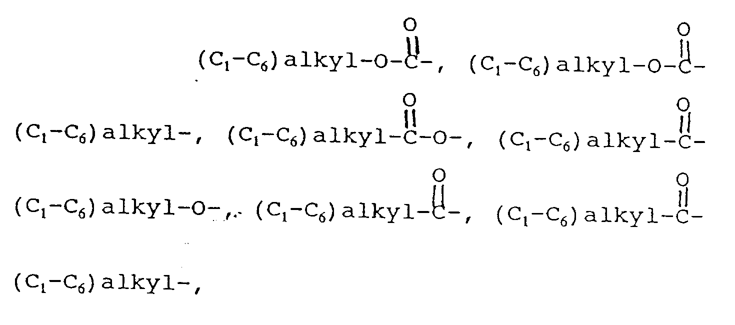 di-(C₁-C₆)alkylamino, 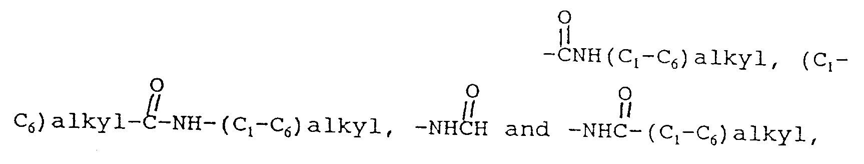 and wherein one of the phenyl moieties of said benzhydryl may optionally be replaced by naphthyl, thienyl, furyl or pyridyl;
R⁵ is hydrogen, phenyl or (C₁-C₆)alkyl;
or R² and R⁵, together with the carbon to which they are attached, form a saturated ring having from 3 to 7 carbon atoms wherein one of the CH₂ groups in said ring may optionally be replaced by oxygen, NH or sulfur;
R³ is aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; and cycloalkyl having 3 to 7 carbon atoms wherein one of the CH₂ groups in said cycloalkyl may optionally be replaced by NH, oxygen or sulfur; wherein each of said aryl and heteroaryl groups may optionally be substituted with one or more substituents, and said (C₃-C₇) cycloalkyl may optionally be substituted with one or two substituents, each of said substituents being independently selected from halo, nitro, (C₁-C₆) alkyl, (C₁-C₆) alkoxy, methyl, trifluoromethyl,
amino, (C₁-C₆) alkylamino, 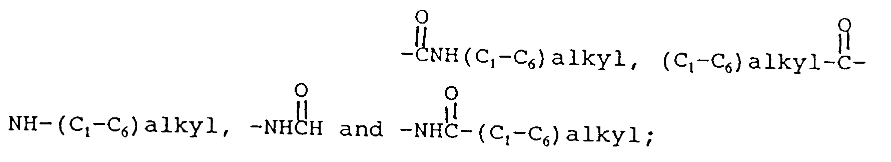 and R⁴ and R⁷ are each independently selected from hydrogen, hydroxy, halo, amino, oxo, cyano, methylene, hydroxymethyl, halomethyl, (C₁-C₆)alkylamino, di-(C₁-C₆)alkylamino, (C₁-C₆)alkoxy, 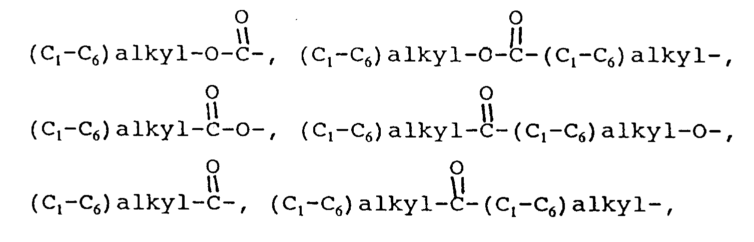 and the radicals set forth in the definition of R²,
R⁶ is 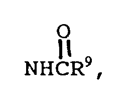 NHCH₂R⁹, SO₂R⁹ or one of the radicals set forth in any of the definitions of R², R⁴ and R⁷;
R⁸ is oximino (=NOH) or one of the radicals set forth in any of the definitions of R², R⁴ and R⁷;
R⁹ is (C₁-C₆)alkyl, hydrogen, phenyl, or phenyl (C₁-C₆)alkyl;
with the proviso that (a) when m is 0, R⁸ is absent, (b) when R⁴, R⁶, R⁷ or R⁸ is as defined in R², it cannot form, together with the carbon to which it is attached, a ring with R⁵, (c) when R⁴ and R⁷ are attached to the same carbon atom, then either each of R⁴ and R⁷ is independently selected from hydrogen, fluoro and (C₁-C₆) alkyl, or R⁴ and R⁷, together with the carbon to which they are attached, form (C₃-C₆) saturated carbocyclic ring that forms a spiro compound with the nitrogen-containing ring to which they are attached, and (d) one of R² and R⁵ must be other than hydrogen or unsubstituted (C₁-C₆)alkyl;
or a pharmaceutically acceptable acid addition salt thereof.

2. A compound as claimed in claim 1 wherein R¹ is H and n is 3 of the formula: 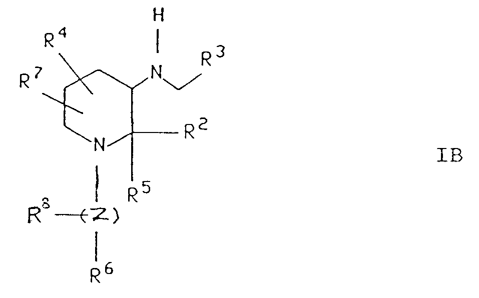 wherein R² to R⁸ and Z are as previously defined in claim 1.

3. A compound as claimed in claim 2 wherein R⁶ is H and m is 0 (Z absent) of formula: 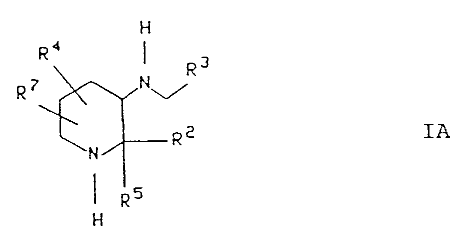 wherein R² to R⁷ are as previously defined in claim 1.

4. A compound as claimed in claim 1 wherein R⁶ is H, n is 3, and m is 0 (Z absent) of formula: 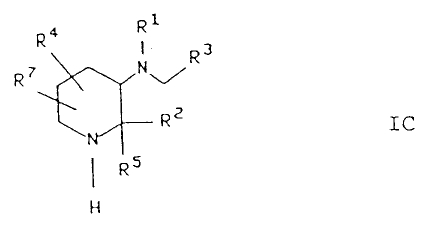 wherein R¹ to R⁵ and R⁷ are as previously defined in claim 1.

5. A compound according to claim 1 wherein R¹, R⁴, R⁵, R⁶ and R⁷ are each H, n is 3 or 4, m is 0, R² and R³ are each phenyl optionally substituted with one or more substituents chosen from chlorine, fluorine (C₁-C₆)alkyl, (C₁-C₆)alkoxy or trifluoromethyl.

6. A compound as claimed in claim 5 wherein R² is phenyl, and R³ is 2-methoxyphenyl wherein the phenyl moiety may optionally be further substituted with chlorine, fluorine, methyl, (C₁-C₆)alkoxy or trifluoromethyl.

7. The 2S, 3S enantioner of a compound of the formula (I) wherein R¹ and R⁶ are H and m is 0 of the formula: 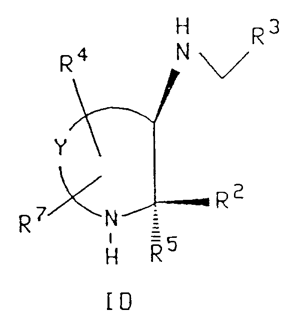 wherein R² to R⁵, R⁷and Y are as previously defined in claim 1.

8. A compound of formula IB as claimed in claim 2 wherein said compound is-:
(2S,3S)-1-(5-carboethoxypent-1-yl)-3-(2-methoxybenzylamino)-2-phenylpiperidine;
(2S,3S)-1-(6-hydroxy-hex-1-yl)-3-(2-methoxybenzylamino)-2-phenylpiperidine;
(2S,3S)-1-(4-hydroxy-4-phenylbut-1-yl)-3-(2-methoxybenzylamino)-2-phenylpiperidine;
(2S,3S)-1-(4-oxo-4-phenylbut-1-yl)-3-(2-methoxybenzylamino)-2-phenylpiperidine;
(2S,3S)-1-(5,6-dihydroxyhex-1-yl)-3-(2-methoxybenzylamino)-2-phenylpiperidine;
cis-3-(5-fluoro-2-methoxybenzylamino)-2-phenylpiperidine;
(2S,3S)-1-[4-(4-fluorophenyl)-4-oxobut-1-yl]-3-(2-methoxybenzylamino)-2-phenylpiperidine;
(2S,3S)-1-[4-[4-fluorophenyl)-4-hydroxybut-1-yl]-3-(2-methoxybenzylamino)-2-phenylpiperidine;
(2S,3S)-1-(4-benzamidobut-1-yl)-3-(2-methoxybenzylamino)-2-phenylpiperidine;
(2S,3S)-3-(2-methoxybenzylamino)-1-(5-N-methylcarboxamidopent-1-yl)-2-phenylpiperidine;
(2S,3S)-1-(4-cyanobut-1-yl)-3-(2-methoxybenzylamino)-2-phenylpiperidine;
(2S,3S)-1-[4-(2-naphthamido)but-1-yl]-3-(2-methoxybenzylamino)-2-phenylpiperidine;
(2S,3S)-1-(5-benzamidopent-1-yl)-3-(2-methoxybenzylamino)-2-phenylpiperidine;
(2S,3S)-1-(5-aminopent-1-yl)-3-(2-methoxybenzylamino)-2-phenylpiperidine;
cis-3-(2,5-dimethoxybenzylamino)-1-[4-(4-fluorophenyl)-4-oxobut-1-yl]-2-phenylpiperidine;
cis-3-(5-chloro-2-methoxybenzylamino)-1-(5,6-dihydroxyhex-1-yl)-2-phenylpiperidine; or
cis-1-(5,6-dihydroxyhex-1-yl)-3-(2,5-dimethoxybenzylamino)-2-phenylpiperidine

9. A compound of the formula IA as claimed in claim 3 wherein said compound is:
cis-3-(2-chlorobenzylamino)-2-phenylpiperidine;
cis-3-(2-trifluoromethylbenzylamino)-2-phenylpiperidine;
cis-3-(2-methoxybenzylamino)-2-(2-fluorophenyl)piperidine;
cis-3-(2-methoxybenzylamino)-2-(2-chlorophenyl)piperidine;
cis-3-(2-methoxybenzylamino)-2-(2-methylphenyl)piperidine;
cis-3-(2-methoxybenzylamino)-2-(3-methoxyphenyl)piperidine;
cis-3-(2-methoxybenzylamino)-2-(3-fluorophenyl)piperidine;
cis-3-(2-methoxybenzylamino)-2-(3-chlorophenyl)piperidine;
cis-3-(2-methoxybenzylamino)-2-phenylpiperidine;
cis-3-(2-methoxybenzylamino)-2-(3-methylphenyl)piperidine;
cis-3-(2-methoxybenzylamino)-2-(4-fluorophenyl)piperidine;
cis-3-(2-methoxybenzylamino)-2-(3-thienyl)- piperidine;
3-(2-methoxybenzylamino)-4-methyl-2-phenyl- piperidine;
3-(2-methoxybenzylamino)-5-methyl-2-phenyl- piperidine;
3-(2-methoxybenzylamino)-6-methyl-2-phenyl- piperidine;
(2S,3S)-3-(2-methoxybenzylamino)-2-phenylpiperidine;
cis-3-(2-methoxy-5-methylbenzylamino)-2-phenylpiperidine;
cis-3-(2-methoxynaphth-1-ylmethylamino)-2-phenylpiperidine;
(2S,3S)-3-(5-chloro-2-methoxybenzylamino)-2-phenylpiperidine;
(2S,3S)-3-(2,5-dimethoxybenzylamino)-2-phenylpiperidine;
cis-3-(3,5-difluoro-2-methoxybenzylamino)-2-phenylpiperidine;
cis-3-(4,5-difluoro-2-methoxybenzylamino)-2-phenylpiperidine;
cis-2-phenyl-3-[2-(prop-2-yloxy)benzylamino]piperidine;
cis-3-(2,5-dimethoxybenzyl)amino-2-(3-methoxyphenyl)piperidine hydrochloride;
cis-3-(5-chloro-2-methoxybenzyl)amino-2-(3-methoxyphenyl)piperidine dihydrochloride;
cis-3-(5-chloro-2-methoxybenzyl)amino-2-(3-chlorophenyl)piperidine dihydrochloride;
3-(2-methoxybenzylamino)-2,4-diphenylpiperidine; or
cis-3-(2-methoxybenzylamino)-2-phenylpyrrolidine.

10. A compound of the formula I as claimed in claim 1 wherein Y is (CH₂)ₙ and n is 4, and wherein R⁶ is H and m is 0 (Z absent) said compound being:
cis-3-(2-methoxybenzylamino)-2-phenylazacycloheptane;

11. A compound of the formula 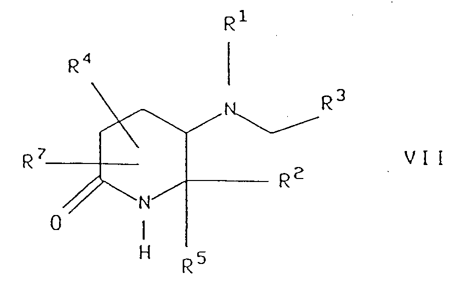 wherein R¹, R², R³, R⁴, R⁵ and R⁷ are as defined in claim 1.

12. A pharmaceutical composition comprising a compound of the formula I as claimed in any one of claims 1 to 10, or a pharmaceutically acceptable salt thereof, together with a pharmaceutically acceptable diluent or carrier.

13. A compound of the formula (I) as claimed in any one claims 1 to 10 for use in treating or preventing a condition in a mammal which is effected or facilitated by a decrease in substance P mediated neurotransmission including use in the treatment or prevention of inflammatory diseases, anxiety, colitis, depression or dysthymic disorders, psychosis, pain, allergies, chronic obstructive airways disease, hypersensitivity disorders, vasospastic diseases, fibrosing and collagen diseases, reflex sympathetic dystrophy, addiction disorders, stress related somatic disorders, peripheral neuropathy, neuralgia, neuropathological disorders, disorders related to immune enhancement or suppression and rheumatic diseases.

14. The use of a compound of the formula (I) as claimed in any one of claims 1 to 10 for the preparation of a medicament for use in the treatment or prevention of inflammatory diseases, anxiety, colitis, depression or dysthymic disorders, psychosis, pain, allergies, chronic obstructive airways disease, hypersensitivity disorders, vasospastic diseases, fibrosing and collagen diseases, reflex sympathetic dystrophy, addiction disorders, stress related somatic disorders, peripheral neuropathy, neuralgia, neuropathological disorders, disorders related to immune enhancement or suppression and rheumatic diseases.

## Claims (Claims for the following Contracting State(s): ES, GR)

1. A process for preparing a compound having the formula 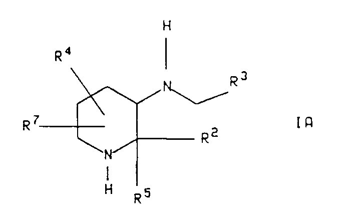 wherein R² is a radical selected from hydrogen, (C₁-C₆) straight or branched alkyl, (C₃-C₇) cycloalkyl wherein one of the CH₂ groups in said cycloalkyl may optionally be replaced by NH, oxygen or sulfur; aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; phenyl (C₂-C₆) alkyl, benzhydryl and benzyl, wherein each of said aryl and heteroaryl groups and the phenyl moieties of said benzyl, phenyl (C₂-C₆) alkyl and benzhydryl may optionally be substituted with one or more substituents independently selected from halo, nitro, (C₁-C₆) alkyl, (C₁-C₆) alkoxy, trifluoromethyl, amino, (C₁-C₆)
alkylamino, 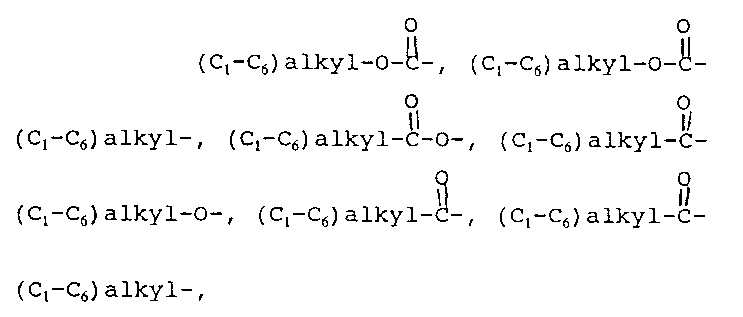 di-(C₁-C₆)alkylamino, 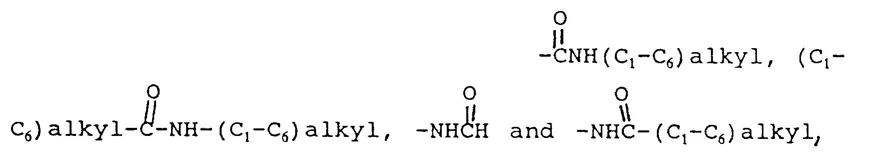 and wherein one of the phenyl moieties of said benzhydryl may optionally be replaced by naphthyl, thienyl, furyl or pyridyl;
R⁵ is hydrogen, phenyl or (C₁-C₆)alkyl;
or R² and R⁵, together with the carbon to which they are attached, form a saturated carbocyclic ring having from 3 to 7 carbon atoms wherein one of the CH₂ groups in said ring may optionally he replaced by oxygen, NH, or sulfur;
R³ is aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; and cycloalkyl having 3 to 7 carbon atoms wherein one of the CH₂ groups in said cycloalkyl may optionally be replaced by NH, oxygen or sulfur; wherein each of said aryl and heteroaryl groups may optionally be substituted with one or more substituents, and said (C₃-C₇) cycloalkyl may optionally be substituted with one or two substituents, each of said substituents being independently selected from halo, nitro, (C₁-C₆) alkyl, (C₁-C₆) alkoxy, methyl, trifluoromethyl, amino, (C₁-C₆) alkylamino, 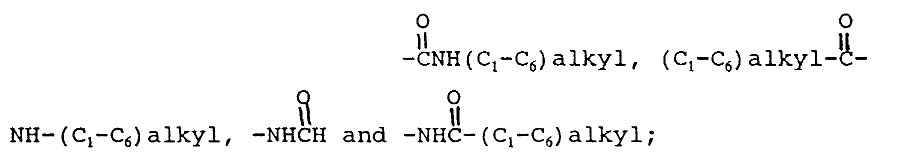 and
R⁴ and R⁷ are each independently selected from hydrogen, hydroxy, halo, amino, oxo, cyano, methylene, hydroxymethyl, halomethyl, (C₁-C₆)alkylamino, di-(C₁-C₆)alkylamino, (C₁-C₆)alkoxy, 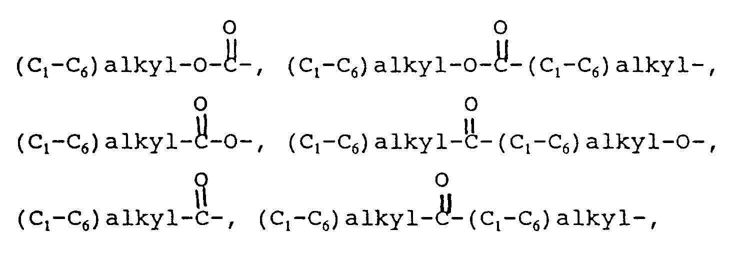 and the radicals set forth in the definition of R²;
with the proviso that (a) neither R⁴ nor R⁷ can form, together with the carbon to which it is attached, a ring with R⁵, (b) when R⁴ and R⁷ are attached to the same carbon atom, then either each of R⁴ and R⁷ is independently selected from hydrogen, fluoro and (C₁-C₆) alkyl, or R⁴ and R⁷, together with the carbon to which they are attached, form a (C₃-C₆) saturated carbocyclic ring that forms a spiro compound with the nitrogen-containing ring to which they are attached, (c) R² is not benzhydryl, and (d) neither R⁴ nor R⁷ is attached to the "6" position of the piperidine ring;
or a pharmaceutically acceptable acid addition salt thereof;
comprising: (a) reducing a compound of the formula 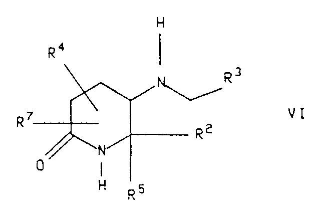 wherein R², R³, R⁴, R⁵ and R⁷ are defined as above; and (b) optionally reacting the compound of formula IA obtained thereby with a pharmaceutically acceptable acid.

2. A process according to claim 1, wherein said compound of formula VI is obtained by reacting a compound of the formula 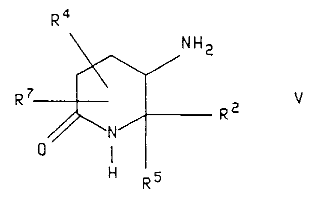 with sodium cyanoborohydride or sodium triacetoxyborohydride and a compound of the formula R³CHO wherein R³ is defined as in claim 1.

3. A process for preparing a compound having the formula 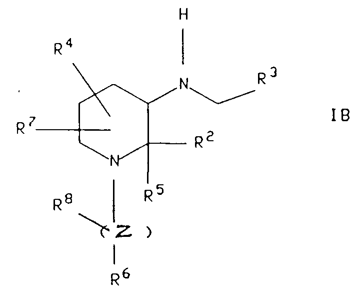 wherein R², R³, R⁴, R⁵ and R⁷ are defined as in claim 1;
Z is (CH₂)ₘ wherein m is an integer from 0 to 6, and wherein any one of the carbon-carbon single bonds of said (CH₂)ₘ may optionally be replaced by a carbon-carbon double bond or a carbon-carbon triple bond, and any one of the carbon atoms of said (CH₂)ₘ may optionally be substituted with R⁸;
R⁶ is 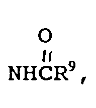 NHCH₂R⁹, SO₂R⁹ or one of the radicals set forth in any of the definitions of R², R⁴ and R⁷;
R⁸ is oximino (=NOH) or one of the radicals set forth in any of the definitions of R², R⁴ and R⁷;
R⁹ is (C₁-C₆)alkyl, hydrogen, phenyl, or phenyl (C₁-C₆)alkyl;
with the provisio that (a) when m is 0, R⁸ is absent, (b) when R⁴, R⁶, R⁷ or R⁸ is as defined in R², it cannot form, together with the carbon to which it is attached, a ring with R⁵, (c) when R⁴ and R⁷ are attached to the same carbon atom, then either each of R⁴ and R⁷ is independently selected from hydrogen and (C₁-C₆) alkyl, or R⁴ and R⁷, together with the carbon to which they are attached, form a (C₃-C₆) saturated carbocyclic ring that forms a spiro compound with the nitrogen-containing ring to which they are attached, (d) R² is not benzhydryl, and (e) neither R⁴ nor R⁷ is attached to the "6" position of the piperidine ring, and (d) one of R² and R⁵ must be other than hydrogen or unsubstituted (C₁-C₆)alkyl;
or a pharmaceutically acceptable acid addition salt thereof;
comprising: (a) reacting a compound of the formula IA, as defined in claim 1, with a compound of the formula R⁶-( Z ) -X, wherein R⁶ is defined as above, X is halo, Z is (CH₂)ₘ and one of the carbon-carbon single bonds said (CH₂)ₘ may optionally be replaced by a carbon-carbon double bond, and any one of the carbon atoms of said (CH₂)ₘ may optionally be substituted with R⁸; and (b) optionally reacting the compound of formula IB obtained thereby with a pharmaceutically acceptable acid.

4. A process for preparing a compound of the formula 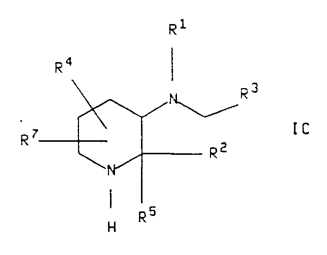 wherein R², R³, R⁴, R⁵ and R⁷ are defined as in claim 1; and R¹ is hydrogen or (C₁-C₈)alkyl optionally substituted with hydroxy, alkoxy or fluoro;
with the proviso that when R⁴ and R⁷ are attached to the same carbon atom, then either each of R⁴ and R⁷ is independently selected from hydrogen, fluoro and (C₁-C₆)alkyl, or R⁴ and R⁷, together with the carbon to which they are attached, form a (C₁-C₆) saturated carbocyclic ring that forms a spiro compound with the nitrogen containing ring to which they are attached, (b) R² is not benzhydryl, and (c) neither R⁴ nor R⁷ is attached to the "6" position of the piperidine ring;
or a pharmaceutically acceptable acid addition salt thereof;
comprising: (a) reducing a compound of the formula 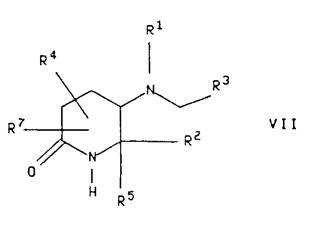 wherein R², R³, R⁴, R⁵ and R⁷ are defined as in claim 1 and R¹ is defined as above; and (b) optionally reacting the compound of formula IC obtained thereby with a pharmaceutically acceptable acid.

5. A process to claim 4, wherein said compound of formula VII is obtained by reacting a compound of the formula 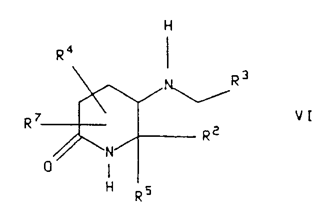 wherein R², R³, R⁴, R⁵ and R⁷ are defined as in claim 1, with a compound of the formula R¹X, wherein X is halo and R is defined as in claim 4.

6. A process for preparing a compound of the formula IB, as defined in claim 3, or a pharmaceutically acceptable acid addition salt thereof, comprising: (a) reducing a compound of the formula 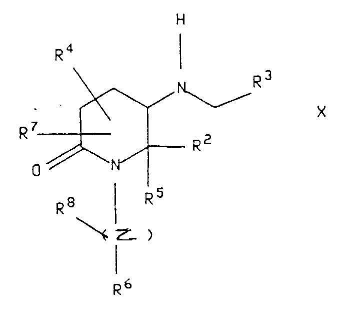 wherein R², R³, R⁴, R⁵ and R⁷ are defined as in claim 1 and R⁶, R⁸ and Z are defined as in claim 3, and (b) optionally reacting the compound of formula IB obtained thereby with a pharmaceutically acceptable acid.

7. A process for preparing a compound of the formula 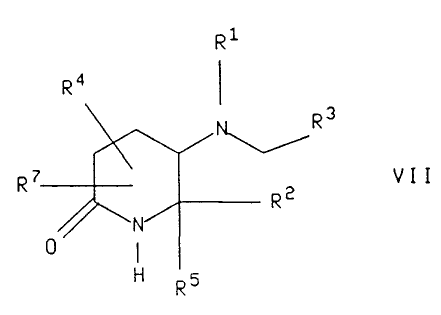 wherein R², R³, R⁴, R⁵ and R⁷ are defined as in claim 1 and R¹ is defined as in claim 4, comprising reacting a compound of the formula 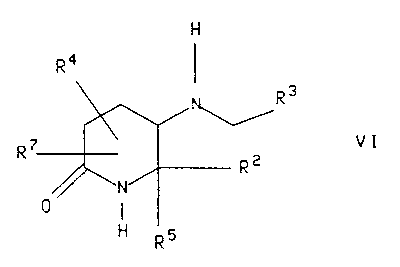 wherein R², R³, R⁴, R⁵ and R⁷ are defined as in claim 1, with a compound of the formula R¹X, wherein X is halo and R¹ is defined as in claim 4.

8. A process for preparing a compound having the formula 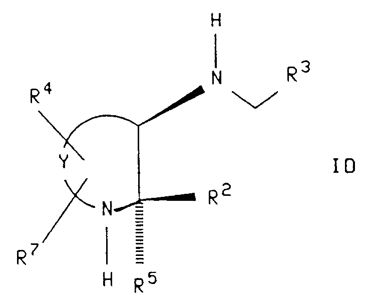 wherein R², R³, R⁴, R⁵ and R⁷ are defined as in claim 1; and Y is (CH₂)ₙ wherein n is an integer from 1 to 4, and wherein any one of the carbon-carbon single bonds in said (CH₂)ₙ may optionally be replaced by a carbon-carbon double bond or a carbon-carbon triple bond, and wherein any one of the carbon atoms of said (CH₂)ₙ may optionally be substituted with R⁴, and wherein any one of the carbon atoms of said (CH₂)ₙ may optionally be substituted with R⁷;
with the proviso that (a) neither R⁴, R⁶ nor R⁷ can form, together with the carbon to which it is attached, a ring with R⁵, and (b) when R⁴ and R⁷ are attached to the same carbon atom, then either each of R⁴ and R⁷ is independently selected from hydrogen, fluoro and (C₁-C₆)alkyl, or R⁴ and R⁷, together with the carbon to which they are attached, form a (C₃-C₆) saturated carbocyclic ring that forms a spiro compound with the nitrogen-containing ring to which they are attached;
or a pharmaceutically acceptable acid addition salt thereof;
comprising: (a) reacting a compound of the formula 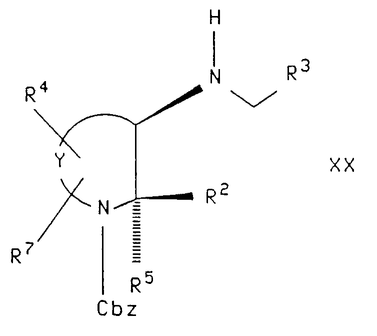 wherein R², R³, R⁴, R⁵ and R⁷ are defined as in claim 1, Y is defined as above and Cbz is carbobenzyloxy, with ammonium formate in the presence of palladium on charcoal; and (b) optionally reacting the compound of formula ID obtained thereby with a pharmaceutically acceptable acid.

9. A process according to claim 8, wherein said compound of the formula XX is obtained by reacting a compound of the formula 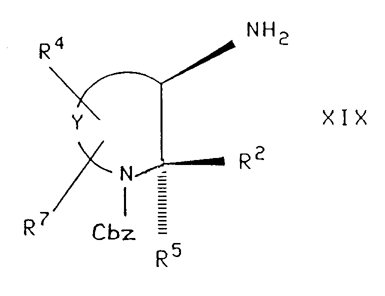 wherein R², R⁴,
R⁵ and R⁷ are defined as in claim 1 and Y is defined as in claim 8, with sodium cyanoborohydride or sodium triacetoxyborohydride and a compound of the formula R³CHO, wherein R³ is defined as in claim 1.

## Patentansprüche (Patentansprüche für folgende(n) Vertragsstaat(en): DE, GB, FR, IT, NL, SE, CH, BE, AT, LU, DK, LI)

1. Verbindung der Formel 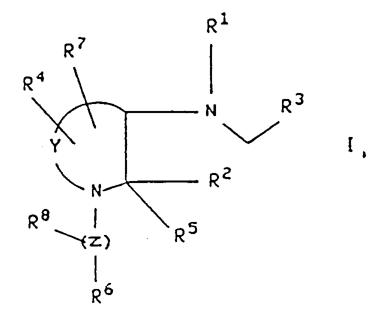 worin Y (CH₂)ₙ ist, worin n eine ganze Zahl von 1 bis 4 ist und worin irgendeine der Kohlenstoff-Kohlenstoff-Einfachbindungen in diesem (CH₂)ₙ fakultativ durch eine Kohlenstoff-Kohlenstoff-Doppelbindung ersetzt sein kann und worin irgendeines der Kohlenstoffatome in diesem (CH₂)ₙ fakultativ mit R⁴ substituiert sein kann und worin irgendeines der Kohlenstoffatome in diesem (CH₂)ₙ fakultativ mit R⁷ substituiert sein kann,
Z (CH₂)ₘ ist, worin m eine ganze Zahl von 0 bis 6 ist und worin irgendeine der Kohlenstoff-Kohlenstoff-Einfachbindungen in diesem (CH₂)ₘ fakultativ durch eine Kohlenstoff-Kohlenstoff-Doppelbindung oder eine Kohlenstoff-Kohlenstoff-Dreifachbindung ersetzt sein kann und irgendeines der Kohlenstoffatome in diesem (CH₂)ₘ fakultativ mit R⁸ substituiert sein kann;
R¹ Wasserstoff oder (C₁-C₈)Alkyl, fakultativ mit Hydroxy, (C₁-C₄)Alkoxy oder Fluor substituiert, ist,
R² ein Rest ist, ausgewählt aus Wasserstoff, geradem oder verzweigtem (C₁-C₆)Alkyl, (C₃-C₇)Cycloalkyl, worin eine der CH₂-Gruppen in diesem Cycloalkyl fakultativ durch NH, Sauerstoff oder Schwefel ersetzt sein kann; Aryl, ausgewählt aus Phenyl und Napthyl; Heteroaryl, ausgewählt aus Indanyl, Thienyl, Furyl, Pyridyl, Thiazolyl, Isothiazolyl, Oxazolyl, Isoxazolyl, Triazolyl, Tetrazolyl und Chinolyl; Phenyl-(C₂-C₆)-alkyl, Benzhydryl und Benzyl, worin jede der Aryl- und Heteroarylgruppen und die Phenylreste von diesem Benzyl, Phenyl(C₂-C₆)-alkyl und Benzhydryl fakultativ mit einem oder mehreren Substituenten substituiert sein kann, unabhängig ausgewählt aus Halogen, Nitro, (C₁-C₆)Alkyl, (C₁-C₆)Alkoxy, Trifluormethyl, Amino, (C₁-C₆)Alkylamino, 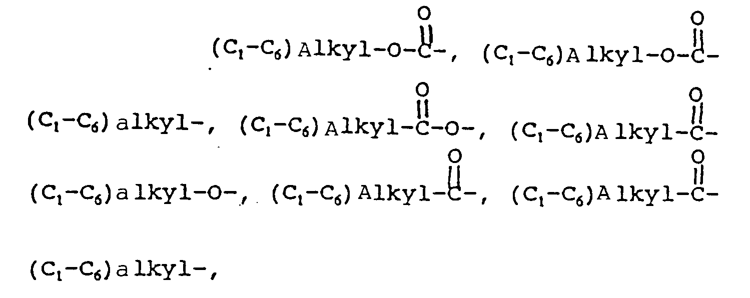 Di-(C₁-C₆)alkylamino, 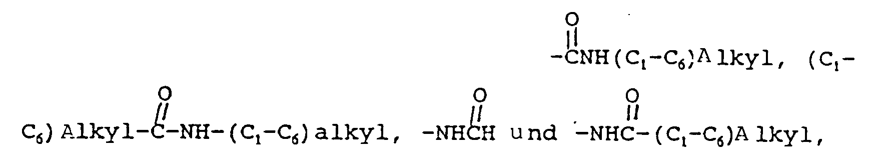 und worin einer der Phenylreste des Benzhydryls fakultativ durch Naphthyl, Thienyl, Furyl oder Pyridyl ersetzt sein kann;
R⁵ Wasserstoff, Phenyl oder (C₁-C₆)Alkyl ist;
oder R² und R⁵, zusammen mit dem Kohlenstoff, an den sie gebunden sind, einen gesättigten Ring mit 3 bis 7 Kohlenstoffatomen bilden, worin eine der CH₂-Gruppen in diesem Ring fakultativ durch Sauerstoff, NH oder Schwefel ersetzt sein kann;
R³ Aryl, ausgewählt aus Phenyl und Naphthyl; Heteroaryl, ausgewählt aus Indanyl, Thienyl, Furyl, Pyridyl, Thiazolyl, Isothiazolyl, Oxazolyl, Isoxazolyl, Triazolyl, Tetrazolyl und Chinolyl; und Cycloalkyl mit 3 bis 7 Kohlenstoffatomen ist, worin eine der CH₂-Gruppen in diesem Cycloalkyl fakultativ durch NH, Sauerstoff oder Schwefel ersetzt sein kann, worin jede der Aryl- und Heteroarylgruppen fakultativ mit einem oder mehreren Substituenten substituiert sein kann und das (C₃-C₇)Cycloalkyl fakultativ mit einem oder zwei Substituenten substituiert sein kann, wobei jeder dieser Substituenten unabhängig ausgewählt sein kann aus Halogen, Nitro, (C₁-C₆)Alkyl, (C₁-C₆)Alkoxy, Methyl, Trifluormethyl, Amino, (C₁-C₆)Alkylamino, 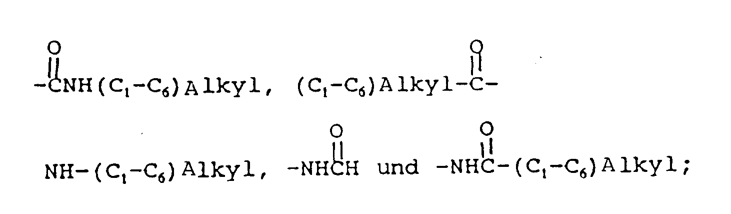 und
R⁴ und R⁷ jeweils unabhängig ausgewählt sind aus Wasserstoff, Hydroxy, Halogen, Amino, Oxo, Cyano, Methylen, Hydroxymethyl, Halogenmethyl, (C₁-C₆)Alkylamino, Di-(C₁-C₆)alkylamino, (C₁-C₆)Alkoxy, 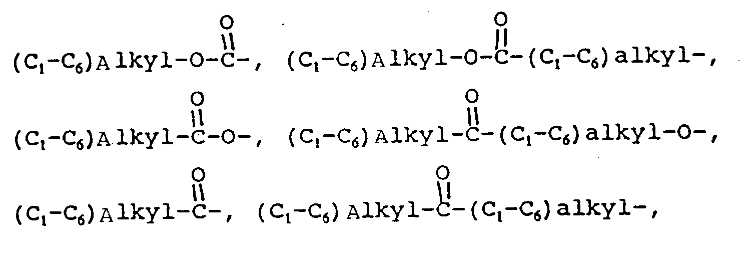 und den in der Definition von R² angegebenen Resten;
R⁶ 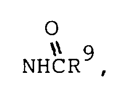 NHCH₂R⁹, SO₂R⁹ oder einer der in irgendeiner der Definitionen von R², R⁴ und R⁷ genannten Reste ist,
R⁸ Oximino (=NOH) oder einer der in irgendeiner der Definitionen von R², R⁴ und R⁷ genannten Reste ist;
R⁹ (C₁-C₆)Alkyl, Wasserstoff, Phenyl oder Phenyl(C₁-C₆)alkyl ist;
mit der Maßgabe, daß (a) wenn m 0 ist, R⁸ fehlt, (b) wenn R⁴, R⁶, R⁷ oder R⁸ wie in R² definiert ist, es, zusammen mit dem Kohlenstoff, an das es gebunden ist, mit R⁵ keinen Ring bilden kann, (c) wenn R⁴ und R⁷ an das gleiche Kohlenstoffatom gebunden sind, dann entweder jedes R⁴ und R⁷ unabhängig ausgewählt ist aus Wasserstoff, Fluor und (C₁-C₆)Alkyl, oder R⁴ und R⁷ bilden, zusammen mit dem Kohlenstoff, an den sie gebunden sind, einen (C₃-C₆)gesättigten carbocyclischen Ring, der mit dem stickstoffhaltigen Ring, an den sie gebunden sind, eine Spiroverbindung bildet, und (d) eines von R² und R⁵ von Wasserstoff oder unsubstituiertem (C₁-C₆)Alkyl verschieden sein muß;
oder ein pharmazeutisch annehmbares Säure-Additionssalz davon.

2. Verbindung nach Anspruch 1, worin R¹ H ist und n 3 ist, mit der Formel 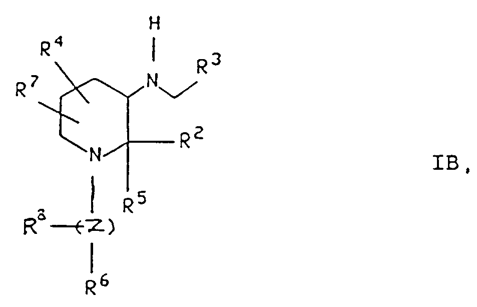 worin R² bis R⁸ und Z wie oben in Anspruch 1 definiert sind.

3. Verbindung nach Anspruch 2, worin R⁶ H ist und m 0 ist (Z fehlt), mit der Formel 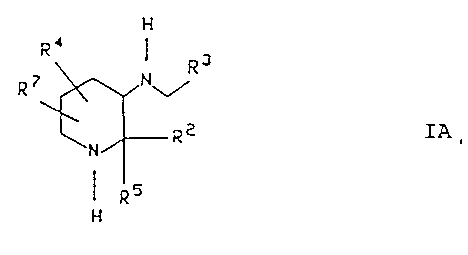 worin R² bis R⁷ wie oben in Anspruch 1 definiert sind.

4. Verbindung nach Anspruch 1, worin R⁶ H ist, n 3 ist und m 0 ist (Z fehlt), mit der Formel 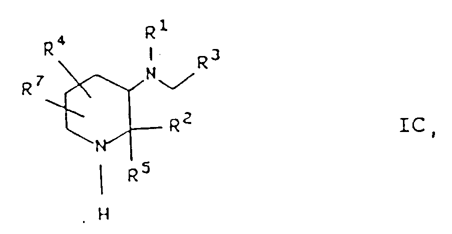 worin R¹ bis R⁵ und R⁷ wie oben in Anspruch 1 definiert sind.

5. Verbindung nach Anspruch 1, worin R¹, R⁴, R⁵, R⁶ und R⁷ jeweils H sind, n 3 oder 4 ist, m 0 ist, R² und R³ jeweils Phenyl sind, fakultativ substituiert mit einem oder mehreren Substituenten, ausgewählt aus Chlor, Fluor, (C₁-C₆)Alkyl, (C₁-C₆)Alkoxy oder Trifluormethyl.

6. Verbindung nach Anspruch 5, worin R² Phenyl ist und R³ 2-Methoxyphenyl ist, worin der Phenylrest fakultativ weiter mit Chlor, Fluor, Methyl, (C₁-C₆)Alkoxy oder Trifluormethyl substituiert sein kann.

7. Das 2S,3S-Enantiomer einer Verbindung der Formel (I), worin R¹ und R⁶ H sind und m 0 ist, mit der Formel 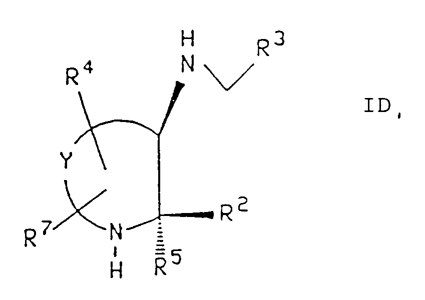 worin R² bis R⁵, R⁷ und Y wie oben in Anspruch 1 definiert sind.

8. Verbindung der Formel IB nach Anspruch 2, worin diese Verbindung ist:
(2S,3S)-1-(5-Carboethoxpent-1-yl)-3-(2-methoxybenzylamino)-2-phenylpiperidin,
(2S,3S)-1-(6-Hydroxyhex-1-yl)-3-(2-methoxybenzylamino)-2-phenylpiperidin,
(2S,3S)-1-(4-Hydroxy-4-phenylbut-1-yl)-3-(2-methoxybenzylamino)-2-phenylpiperidin,
(2S,3S)-1-(4-Oxo-4-phenylbut-1-yl)-3-(2-methoxybenylamino)-2-phenylpiperidin,
(2S,3S)-1-(5,6-Dihydroxyhex-1-yl)-3-(2-methoxybenzylamino)-2-phenylpiperidin,
cis-3-(5-Fluor-2-methoxybenzylamino)-2-phenylpiperidin,
(2S,3S)-1-[4-(4-Fluorphenyl)-4-oxobut-1-yl]-3-(2-methoxybenzylamino)-2-phenylpiperidin,
(2S,3S)-1-[4-(4-Fluorphenyl)-4-hydroxybut-1-yl]-3-(2-methoxybenzylamino)-2-phenylpiperidin,
(2S,3S)-1-(4-Benzamidobut-1-yl)-3-(2-methoxybenzylamino)-2-phenylpiperidin
(2S,3S)-3-(2-Methoxybenzylamino)-1-(5-N-methylcarboxamidopent-1-yl)-2-phenylpiperidin,
(2S,3S)-1-(4-Cyanobut-1-yl)-3-(2-methoxybenzylamino)-2-phenylpiperidin,
(2S,3S)-1-[4-(2-Naphthamido)but-1-yl]-3-(2-methoxybenzylamino)-2-phenylpiperidin,
(2S,3S)-1-(5-Benzamidopent-1-yl)-3-(2-methoxybenzylamino)-2-phenylpiperidin,
(2S,3S)-1-(5-Aminopent-1-yl)-3-(2-methoxybenzylamino)-2-phenylpiperidin,
cis-3-(2,5-Dimethoxybenzylamino)-1-[4-(4-fluorphenyl)-4-oxobut-1-yl]-2-phenylpiperidin,
cis-3-(5-Chlor-2-methoxybenzylamino)-1-(5,6-dihydroxyhex-1-yl)-2-phenylpiperidin oder
cis-1-(5,6-Dihydroxyhex-1-yl)-3-(2,5-dimethoxybenzylamino)-2-phenylpiperidin.

9. Verbindung der Formel IA nach Anspruch 3, worin die Verbindung ist:
cis-3-(2-Chlorbenzylamino)-2-phenylpiperidin,
cis-3-(2-Trifluormethylbenzylamino)-2-phenylpiperidin,
cis-3-(2-Methoxybenzylamino)-2-(2-fluorphenyl)-piperidin,
cis-3-(2-Methoxybenzylamino)-2-(2-chlorphenyl)-piperidin,
cis-3-(2-Methoxybenzylamino)-2-(2-methylphenyl)-piperidin,
cis-3-(2-Methoxybenzylamino)-2-(3-methoxyphenyl)-piperidin,
cis-3-(2-Methoxybenzylamino)-2-(3-fluorphenyl)-piperidin,
cis-3-(2-Methoxybenzylamino)-2-(3-chlorphenyl)-piperidin,
cis-3-(2-Methoxybenzylamino)-2-phenylpiperidin,
cis-3-(2-Methoxybenzylamino)-2-(3-methylphenyl)-piperidin,
cis-3-(2-Methoxybenzylamino)-2-(4-fluorphenyl)-piperidin,
cis-3-(2-Methoxybenzylamino)-2-(3-thienyl)-piperidin,
3-(2-Methoxybenzylamino)-4-methyl-2-phenylpiperidin,
3-(2-Methoxybenzylamino)-5-methyl-2-phenylpiperidin,
3-(2-Methoxybenzylamino)-6-methyl-2-phenylpiperidin,
(2S,3S)-3-(2-Methoxybenzylamino)-2-phenylpiperidin,
cis-3-(2-Methoxy-5-methylbenzylamino)-2-phenylpiperidin,
cis-3-(2-Methoxynaphth-1-ylmethylamino)-2-phenylpiperidin,
(2S,3S)-3-(5-Chlor-2-methoxybenzylamino)-2-phenylpiperidin,
(2S,3S)-3-(2,5-Dimethoxybenzylamino)-2-phenylpiperidin,
cis-3-(3,5-Difluor-2-methoxybenzylamino)-2-phenylpiperidin,
cis-3-(4,5-Difluor-2-methoxybenzylamino)-2-phenylpiperidin,
cis-2-Phenyl-3-[2-(prop-2-yloxy)benzylamino]piperidin,
cis-3-(2,5-Dimethoxybenzyl)amino-2-(3-methoxyphenyl)piperidin-hydrochlorid,
cis-3-(5-Chlor-2-methoxybenzyl)amino-2-(3-methoxyphenyl)piperidin-dihydrochlorid,
cis-3-(5-Chlor-2-methoxybenzyl)amino-2-(3-chlorphenyl)piperidin-dihydrochlorid,
3-(2-Methoxybenzylamino)-2,4-diphenylpiperidin oder
cis-3-(2-Methoxybenzylamino)-2-phenylpyrrolidin.

10. Verbindung der Formel I nach Anspruch 1, worin Y (CH₂)ₙ ist und n 4 ist und worin R⁶ H ist und m 0 ist (Z fehlt), wobei die Verbindung ist;
cis-3-(2-Methoxybenzylamino)-2-phenylazacycloheptan.

11. Verbindung der Formel 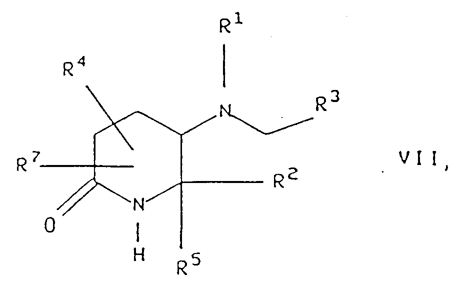 worin R¹, R², R³, R⁴, R⁵ und R⁷ wie in Anspruch 1 definiert sind.

12. Pharmazeutische Zusammensetzung, umfassend eine Verbindung der Formel I nach irgendeinem der Ansprüche 1 bis 10 oder ein pharmazeutisch annehmbares Salz davon zusammen mit einem pharmazeutisch annehmbaren Verdünnungsmittel oder Träger.

13. Verbindung der Formel (I) nach irgendeinem der Ansprüche 1 bis 10 zur Verwendung bei der Behandlung oder Prävention eines Zustandes bei einem Säugetier, der durch eine Abnahme einer durch Substanz P vermittelten Neurotransmission beeinflußt oder gelindert wird, einschließlich der Verwendung bei der Behandlung oder Prävention von entzündlichen Erkrankungen, Angst, Colitis, Depression oder dysthymischen Störungen, Psychose, Schmerz, Allergien, chronischen obstruktiven Erkrankungen der Luftwege, Hypersensibilitätsstörungen, vasospastischen Erkrankungen, fibrosierenden und Collagenerkrankungen, sympathetischer Reflexdystrophie, Addiktionsstörungen, stressbezogenen somatischen Störungen, peripherer Neuropathie, Neuralgie, neuropathologischen Störungen, mit einer Immunverstärkung oder -suppression im Zusammenhang stehenden Störungen und rheumatischen Erkrankungen.

14. Verwendung einer Verbindung der Formel (I) nach irgendeinem der Ansprüche 1 bis 10 zur Herstellung eines Arzneimittels zur Verwendung bei der Behandlung oder Prävention von entzündlichen Erkrankungen, Angst, Colitis, Depression oder dysthymischen Störungen, Psychose, Schmerz, Allergien, chronischen obstruktiven Erkrankungen der Luftwege, Hypersensibilitätsstörungen, vasospastischen Erkrankungen, fibrosierenden und Collagenerkrankungen, sympathetischer Reflexdystrophie, Addiktionsstörungen, stressbezogenen somatischen Störungen, peripherer Neuropathie, Neuralgie, neuropathologischen Störungen, mit einer Immunverstärkung oder -suppression im Zusammenhang stehenden Störungen und rheumatischen Erkrankungen.

## Patentansprüche (Patentansprüche für folgende(n) Vertragsstaat(en): ES, GR)

1. Verfahren zur Herstellung einer Verbindung der Formel 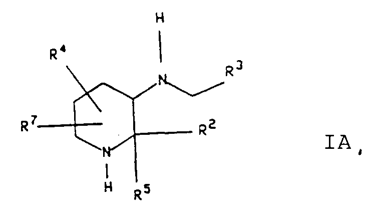 worin R² ein Rest ist, ausgewählt aus Wasserstoff, geradem oder verzweigtem (C₁-C₆)Alkyl, (C₃-C₇)Cycloalkyl, worin eine der CH₂-Gruppen in diesem Cycloalkyl fakultativ durch NH, Sauerstoff oder Schwefel ersetzt sein kann; Aryl, ausgewählt aus Phenyl und Napthyl; Heteroaryl, ausgewählt aus Indanyl, Thienyl, Furyl, Pyridyl, Thiazolyl, Isothiazolyl, Oxazolyl, Isoxazolyl, Triazolyl, Tetrazolyl und Chinolyl; Phenyl-(C₂-C₆)-Alkyl, Benzhydryl und Benzyl, worin jede dieser Aryl- und Heteroarylgruppen und die Phenylreste dieses Benzyls, Phenyl(C₂-C₆)Alkyls und Benzhydryls fakultativ mit einem oder mehreren Substituenten substituiert sein kann, unabhängig ausgewählt aus Halogen, Nitro, (C₁-C₆)Alkyl, (C₁-C₆)Alkoxy, Trifluormethyl, Amino, (C₁-C₆)Alkylamino 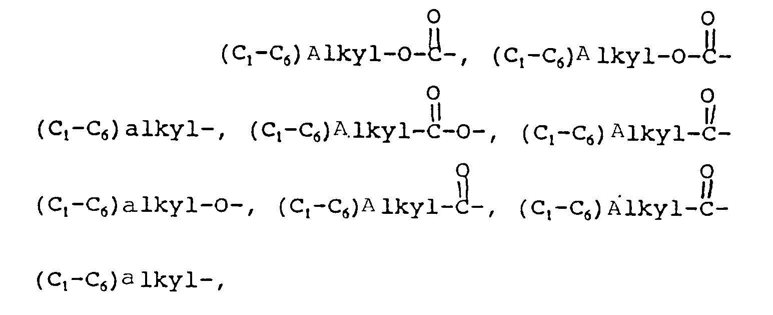 Di-(C₁-C₆)alkylamino, 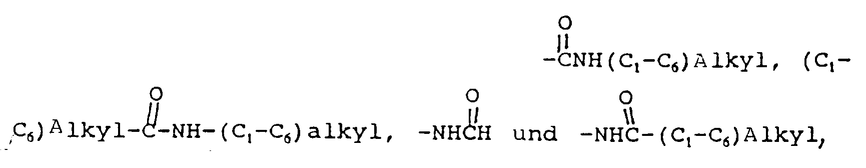 und worin einer der Phenylreste dieses Benzhydryls fakultativ durch Naphthyl, Thienyl, Furyl oder Pyridyl ersetzt sein kann;
R⁵ Wasserstoff, Phenyl oder (C₁-C₆)Alkyl ist;
oder R² und R⁵, zusammen mit dem Kohlenstoff, an den sie gebunden sind, einen gesättigten Ring mit 3 bis 7 Kohlenstoffatomen bilden, worin eine der CH₂-Gruppen in diesem Ring fakultativ durch Sauerstoff, NH oder Schwefel ersetzt sein kann;
R³ Aryl, ausgewählt aus Phenyl und Naphthyl; Heteroaryl, ausgewählt aus Indanyl, Thienyl, Furyl, Pyridin, Thiazolyl, Isothiazolyl, Oxazolyl, Isoxazolyl, Triazolyl, Tetrazolyl und Chinolyl; und Cycloalkyl mit 3 bis 7 Kohlenstoffatomen ist, worin eine der CH₂ Gruppen in diesem Cycloalkyl fakultativ durch NH, Sauerstoff oder Schwefel ersetzt sein kann, worin jede dieser Aryl- und Heteroarylgruppen fakultativ mit einem oder mehreren Substituenten substituiert sein kann und das (C₃-C₇)Cycloalkyl fakultativ mit einem oder zwei Substituenten substituiert sein kann, wobei jeder dieser Substituenten unabhängig ausgewählt sein kann aus Halogen, Nitro, (C₁-C₆)Alkyl, (C₁-C₆)Alkoxy, Methyl, Trifluormethyl, Amino, (C₁-C₆)Alkylamino 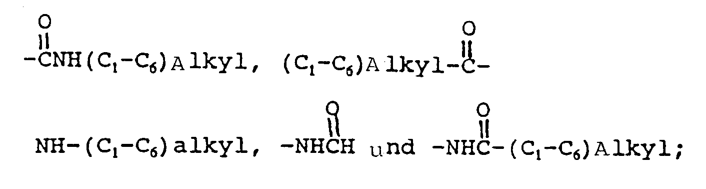 und
R⁴ und R⁷ jeweils unabhängig ausgewählt sind aus Wasserstoff, Hydroxy, Halogen, Amino, Oxo, Cyano, Methylen, Hydroxymethyl, Halogenmethyl, (C₁-C₆)Alkylamino, Di-(C₁-C₆)alkylamino, (C₁-C₆)Alkoxy, 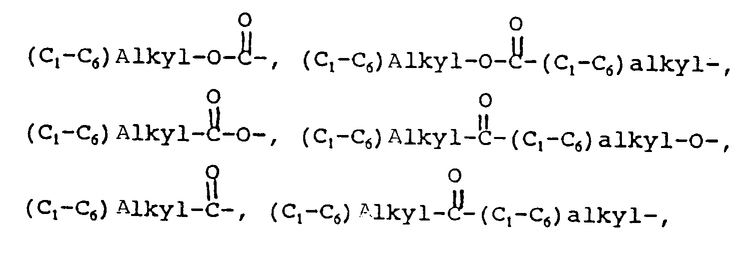 und den in der Definition von R² angegebenen Resten;
mit der Maßgabe, daß (a) weder R⁴ noch R⁷, zusammen mit dem Kohlenstoff, an den es gebunden ist, einen Ring mit R⁵ bilden kann, (b) wenn ⁴ und R⁷ an das gleiche Kohlenstoffatom gebunden sind, dann entweder jedes R⁴ und R⁷ unabhängig ausgewählt ist aus Wasserstoff, Fluor und (C₁-C₆)Alkyl, oder R⁴ und R⁷ bilden, zusammen mit dem Kohlenstoff, an den sie gebunden sind, einen (C₃-C₆)-gesättigten carbocyclischen Ring, der mit dem stickstoffhaltigen Ring, an den sie gebunden sind, eine Spiroverbindung bildet, (c) R² nicht Benzhydryl ist und (d) weder R⁴ noch R⁷ an die "6"-Stellung des Piperidinringes gebunden ist,
oder eines pharmazeutisch annehmbaren Säure-Additionssalzes davon,
umfassend: (a) Reduzieren einer Verbindung der Formel 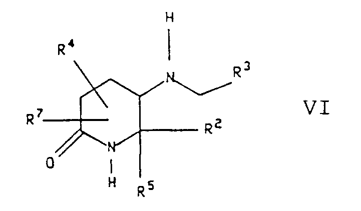 worin R², R³, R⁴, R⁵ und R⁷ wie oben definiert sind, und (b) fakultatives Umsetzen der so erhaltenen Verbindung der Formel IA mit einer pharmazeutisch annehmbaren Säure.

2. Verfahren nach Anspruch 1, in dem die Verbindung von Formel VI erhalten wird durch Umsetzen einer Verbindung der Formel 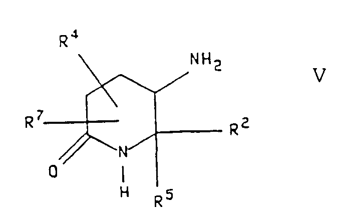 mit Natriumcyanoborhydrid oder Natriumtriacetoxyborhydrid und einer Verbindung der Formel R³CHO, worin R³ wie in Anspruch 1 definiert ist.

3. Verfahren zur Herstellung einer Verbindung der Formel 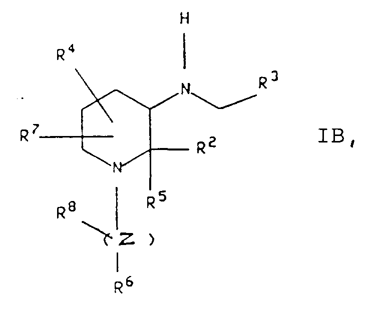 worin R², R³, R⁴, R⁵ und R⁷ wie in Anspruch 1 definiert sind,
Z (CH₂)ₘ ist, worin m eine ganze Zahl von 0 bis 6 ist und worin irgendeine der Kohlenstoff-Kohlenstoff-Einfachbindungen in diesem (CH₂)ₘ fakultativ durch eine Kohlenstoff-Kohlenstoff-Doppelbindung oder eine Kohlenstoff-Kohlenstoff-Dreifachbindung ersetzt sein kann und irgendeines der Kohlenstoffatome von diesem (CH₂)ₘ fakultativ mit R⁸ substituiert sein kann;
R⁶ 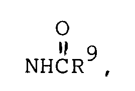 NHCH₂R⁹, SO₂R⁹ oder einer der in irgendeiner der Definitionen von R², R⁴, und R⁷ genannten Reste ist,
R⁸ Oximino (=NOH) oder einer der in irgendeiner der Definitionen von R², R⁴ und R⁷ genannten Reste ist;
R⁹ (C₁-C₆)Alkyl, Wasserstoff, Phenyl oder Phenyl(C₁-C₆)alkyl ist;
mit der Maßgabe, daß (a) wenn m 0 ist, R⁸ fehlt, (b) wenn R⁴, R⁶, R⁷ oder R⁸ wie in R² definiert ist, es, zusammen mit dem Kohlenstoff, an das es gebunden ist, mit R⁵ keinen Ring bilden kann, (c) wenn R⁴ und R⁷ an das gleiche Kohlenstoffatom gebunden sind, dann entweder jedes R⁴ und R⁷ unabhängig ausgewählt ist aus Wasserstoff und (C₁-C₆)Alkyl, oder R⁴ und R⁷ bilden, zusammen mit dem Kohlenstoff, an den sie gebunden sind, einen (C₃-C₆)gesättigten carbocyclischen Ring, der mit dem stickstoffhaltigen Ring, an den sie gebunden sind, eine Spiroverbindung bildet, (d) R² nicht Benzyhydryl ist und (e) weder R⁴ noch R⁷ an die "6"-Stellung des Piperidinringes gebunden ist, und (f) eines von R² und R⁵ von Wasserstoff oder unsubstituiertem (C₁-C₆)Alkyl verschieden sein muß;
oder eines pharmazeutisch annehmbaren Säure-Additionssalzes davon.
umfassend: (a) Umsetzen einer Verbindung der Formel IA gemäß Definition in Anspruch 1 mit einer Verbindung der Formel R⁶(Z)-X, worin R⁶ wie oben definiert ist, X Halogen ist, Z (CH₂)ₘ ist und eine der Kohlenstoff-Kohlenstoff-Einfachbindungen von diesem (CH₂)ₘ fakultativ durch eine Kohlenstoff-Kohlenstoff-Doppelbindung ersetzt sein kann und irgendeines der Kohlenstoffatome von diesem (CH₂)ₘ fakultativ mit R⁸ substituiert sein kann, und (b) fakultatives Umsetzen der so erhaltenen Verbindung der Formel IB mit einer pharmazeutisch annehmbaren Säure.

4. Verfahren zur Herstellung einer Verbindung der Formel 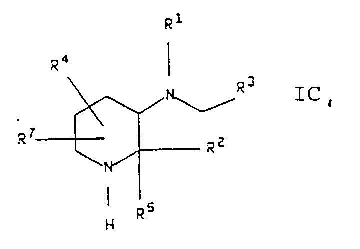 worin R², R³, R⁴, R⁵ und R⁷ wie in Anspruch 1 definiert sind und R¹ Wasserstoff oder (C₁-C₆)Alkyl, fakultativ mit Hydroxy, Alkoxy oder Fluor substituiert, ist,
mit der Maßgabe, daß, wenn R⁴ und R⁷ an das gleiche Kohlenstoffatom gebunden sind, dann entweder jedes R⁴ und R⁷ unabhängig ausgewählt ist aus Wasserstoff, Fluor und (C₁-C₆)Alkyl, oder R⁴ und R⁷ bilden, zusammen mit dem Kohlenstoff, an den sie gebunden sind, einen (C₁-C₆)gesättigten carbocyclischen Ring, der mit dem stickstoffhaltigen Ring, an den sie gebunden sind, eine Spiroverbindung bildet, (b) R² nicht Benzhydryl ist und (c) weder R⁴ noch R⁷ an die "6"-Stellung des Piperidinringes gebunden ist;
oder eines pharmazeutisch annehmbaren Säure-Additionssalzes davon,
umfassend: (a) Reduzieren einer Verbindung der Formel 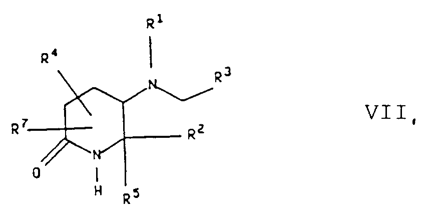 worin R², R³, R⁴, R⁵ und R⁷ wie in Anspruch 1 definiert sind und R¹ wie oben definiert ist, und (b) fakultatives Umsetzen der so erhaltenen Verbindung der Formel IC mit einer pharmazeutisch annehmbaren Säure.

5. Verfahren nach Anspruch 4, worin die Verbindung der Formel VII erhalten wird durch Umsetzen einer Verbindung der Formel 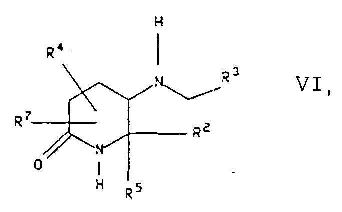 worin R², R³, R⁴, R⁵ und R⁷ wie in Anspruch 1 definiert sind, mit einer Verbindung der Formel R¹X, worin X Halogen ist und R¹ wie in Anspruch 4 definiert ist.

6. Verfahren zur Herstellung einer Verbindung der Formel IB gemäß Definition in Anspruch 3 oder eines pharmazeutisch annehmbaren Säure-Additionssalzes davon, umfassend: (a) Reduzieren einer Verbindung der Formel 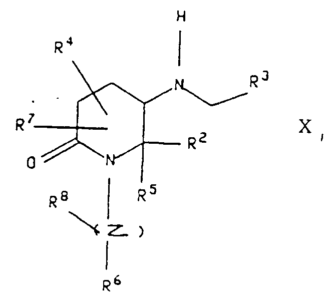 worin R², R³, R⁴, R⁵ und R⁷ wie in Anspruch 1 definiert sind und R⁶, R⁸ und Z wie in Anspruch 3 definiert sind, und (b) fakultatives Umsetzen der so erhaltenen Verbindung der Formel IB mit einer pharmazeutisch annehmbaren Säure.

7. Verfahren zur Herstellung einer Verbindung der Formel 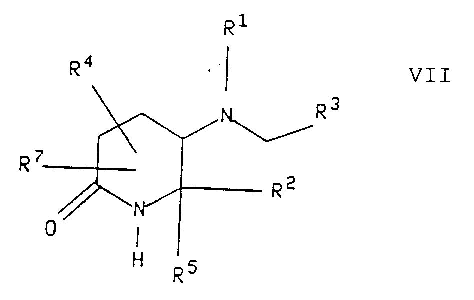 worin R², R³, R⁴, R⁵ und R⁷ wie in Anspruch 1 definiert sind und R¹ wie in Anspruch 4 definiert ist, umfassend das Umsetzen einer Verbindung der Formel 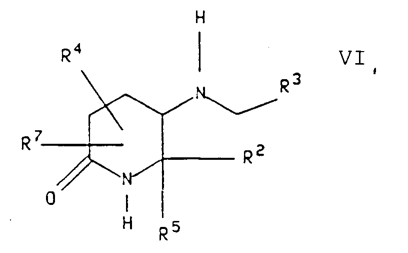 worin R², R³, R⁴, R⁵ und R⁷ wie in Anspruch 1 definiert sind, mit einer Verbindung der Formel R¹X, worin X Halogen ist und R¹ wie in Anspruch 4 definiert ist.

8. Verfahren zur Herstellung einer Verbindung der Formel 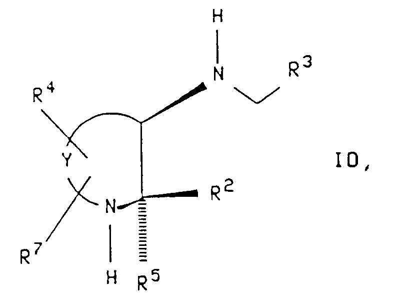 worin R², R³, R⁴, R⁵ und R⁷ wie in Anspruch 1 definiert sind und Y (CH₂)ₙ ist, worin n eine ganze Zahl von 1 bis 4 ist und irgendeine der Kohlenstoff-Kohlenstoff-Einfachbindungen in diesem (CH₂)ₙ fakultativ durch eine Kohlenstoff-Kohlenstoff-Doppelbindung ersetzt sein kann und worin irgendeines der Kohlenstoffatome in diesem (CH₂)ₙ fakultativ mit R⁴ substituiert sein kann und worin irgendeines der Kohlenstoffatome in diesem (CH₂)ₙ fakultativ mit R⁷ substituiert sein kann,
mit der Maßgabe, daß (a) weder R⁴, R⁶ noch R⁷, zusammen mit dem Kohlenstoff, an den es gebunden ist, einen Ring mit R⁵ bilden kann, (b) wenn R⁴ und R⁷ an das gleiche Kohlenstoffatom gebunden sind, dann entweder jedes R⁴ und R⁷ unabhängig ausgewählt ist aus Wasserstoff, Fluor und (C₁-C₆)Alkyl, oder R⁴ und R⁷ bilden, zusammen mit dem Kohlenstoff, an den sie gebunden sind, einen (C₃-C₆)gesättigten carbocyclischen Ring, der mit dem stickstoffhaltigen Ring, an den sie gebunden sind, eine Spiroverbindung bildet,
oder eines pharmazeutisch annehmbaren Säure-Additionssalzes davon,
umfassend: (a) Umsetzen einer Verbindung der Formel 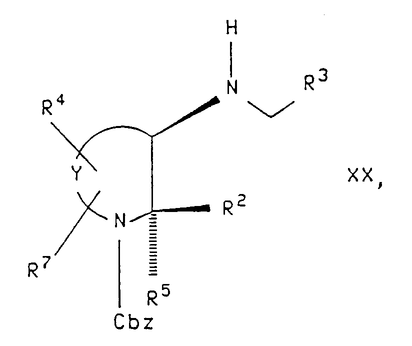 worin R², R³, R⁴, R⁵ und R⁷ wie in Anspruch 1 definiert sind, Y wie oben definiert ist und Cbz Carbobenzyloxy ist, mit Ammoniumformiat in Gegenwart von Palladium auf Tierkohle, und (b) fakultatives Umsetzen der so erhaltenen Verbindung der Formel ID mit einer pharmazeutisch annehmbaren Säure.

9. Verfahren nach Anspruch 8, bei dem die Verbindung der Formel XX erhalten wird durch Umsetzen einer Verbindung der Formel 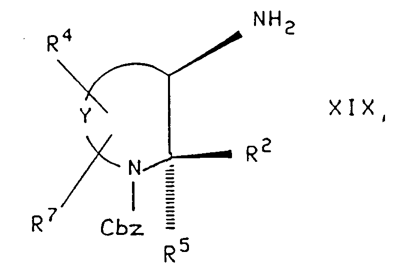 worin R², R⁴, R⁵ und R⁷ wie in Anspruch 1 definiert sind und Y wie in Anspruch 8 definiert ist, mit Natriumcyanoborhydrid oder Natriumtriacetoxyborhydrid und einer Verbindung der Formel R³CHO, worin R³ wie in Anspruch 1 definiert ist.

## Revendications (Revendications pour l'(les) Etat(s) contractant(s) suivant(s): DE, GB, FR, IT, NL, SE, CH, BE, AT, LU, DK, LI)

1. Composé de formule 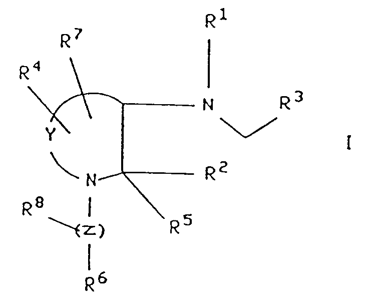 dans laquelle Y est un groupe (CH₂)ₙ où n est un nombre entier de 1 à 4, et dans laquelle l'une quelconque des liaisons simples carbone-à-carbone dans le groupe (CH₂)ₙ peut facultativement être remplacée par une double liaison carbone-à-carbone, et l'un quelconque des atomes de carbone du groupe (CH₂)ₙ peut facultativement être substitué avec R⁴, et l'un quelconque des atomes de carbone du groupe (CH₂)ₙ peut facultativement être substitué avec R⁷,
Z représente (CH₂)ₘ où m est un nombre entier de 0 à 6, et l'une quelconque des liaisons simples carbone-à-carbone du groupe (CH₂)ₘ peut facultativement être remplacée par une double liaison carbone-à-carbone ou une triple liaison carbone-à-carbone, et l'un quelconque des atomes de carbone du groupe (CH₂)ₘ peut facultativement être substitué par R⁸ ;
R¹ est de l'hydrogène ou un groupe alkyle en C₁ à C₈ facultativement substitué avec un radical hydroxy, alkoxy en C₁ à C₄ ou fluoro ;
R² est un radical choisi entre l'hydrogène, un radical alkyle en C₁ à C₆ à chaîne droite ou ramifiée, un radical cycloalkyle en C₃ à C₇ dans lequel l'un des groupes CH₂ peut facultativement être remplacé par un groupe NH, de l'oxygène ou du soufre ; un groupe aryle choisi entre phényle et naphtyle ; un groupe hétéroaryle choisi entre indanyle, thiényle, furyle, pyridyle, thiazolyle, isothiazolyle, oxazolyle, isoxazolyle, triazolyle, tétrazolyle et quinolyle ; un groupe phényl-(alkyle en C₂ à C₆), benzhydryle ou benzyle, où chacun des groupes aryle et hétéroaryle et les portions phényle du groupe benzyle, phényl-(alkyle en C₂ à C₆) et benzhydryle peut facultativement être substitué avec un ou plusieurs substituants choisis indépendamment entre des substituants halogéno, nitro, alkyle en C₁ à C₆, alkoxy en C₁ à C₆, trifluorométhyle, amino, (alkyle en C₁ à C₆)amino, 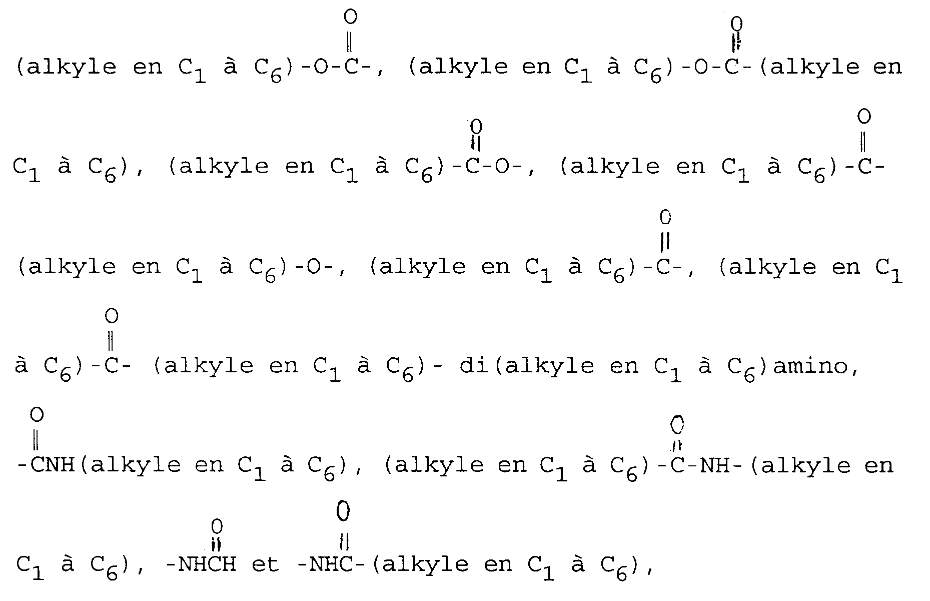 et dans laquelle l'une des portions phényle du groupe benzhydryle peut facultativement être remplacé par un groupe naphtyle, thiényle, furyle ou pyridyle ;
R⁵ est de l'hydrogène, un groupe phényle ou alkyle en C₁ à C₆ ;
ou bien R² et R⁵ forment, conjointement avec l'atome de carbone auquel ils sont attachés, un noyau saturé ayant 3 à 7 atomes de carbone, l'un des groupes CH₂ du noyau pouvant facultativement être remplacé par de l'oxygène, un radical NH ou du soufre ;
R³ est un groupe aryle choisi entre phényle et naphtyle ; un groupe hétéroaryle choisi entre indanyle, thiényle, furyle, pyridyle, thiazolyle, isothiazolyle, oxazolyle, isoxazolyle, triazolyle, tétrazolyle et quinolyle ; et un groupe cycloalkyle ayant 3 à 7 atomes de carbone dont l'un des groupes CH₂ peut facultativement être remplacé par NH, de l'oxygène ou du soufre ; chacun des groupes aryle et hétéroaryle peut facultativement être substitué avec un ou plusieurs substituants, le groupe cycloalkyle en C₃ à C₇ peut facultativement être substitué avec un ou deux substituants, chacun de ces substituants étant choisi indépendamment entre des substituants halogéno, nitro, alkyle en C₁ à C₆, alkoxy en C₁ à C₆, méthyle, trifluorométhyle, amino, (alkyle en C₁ à C₆)amino, 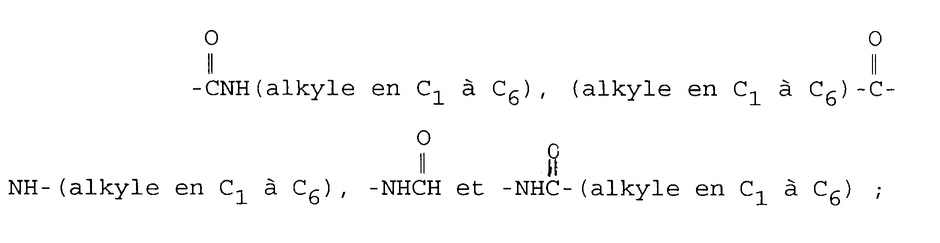 et
R⁴ et R⁷ sont tous deux choisis, indépendamment, entre l'hydrogène, des radicaux hydroxy, halogéno, amino, oxo, cyano, méthylène, hydroxyméthyle, halogénométhyle, (alkyle en C₁ à C₆)amino, di-(alkyle en C₁ à C₆)amino, alkoxy en 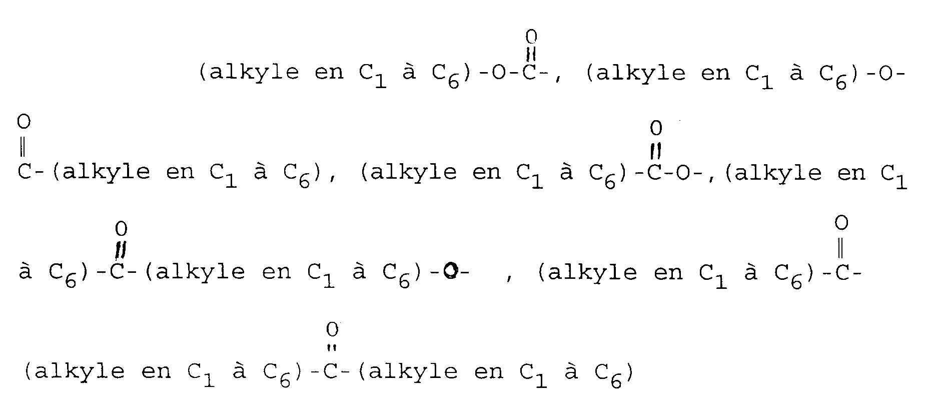 C₁ à C₆, et les radicaux indiqués dans la définition de R² ;
R⁶ est un groupe 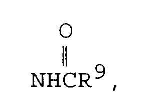 NHCH₂R⁹, SO₂R⁹ ou bien l'un des radicaux indiqués pour l'une quelconque des définitions de R², R⁴ et R⁷ ;
R⁸ est un groupe oximino (=NOH) ou l'un des radicaux indiqués dans l'une quelconque des définitions de R², R⁴ et R⁷ ;
R⁹ est un groupe alkyle en C₁ à C₆, de l'hydrogène, un groupe phényle ou phényl-(alkyle en C₁ à C₆) ;
sous réserve que (a) lorsque m est égal à 0, R⁸ soit absent, (b) lorsque R⁴, R⁶, R⁷ ou R⁸ est tel que défini dans R², il ne puisse pas former, conjointement avec le carbone auquel il est attaché, un noyau avec R⁵, (c) lorsque R⁴ et R⁷ sont attachés au même atome de carbone, chacun d'eux soit indépendamment choisi entre de l'hydrogène, un radical fluoro et alkyle en C₁ à C₆, ou bien R⁴ et R⁷, conjointement avec l'atome de carbone auquel ils sont attachés, forment un noyau carbocyclique saturé en C₃ à C₆ qui donne un composé spiro avec le noyau contenant de l'azote auquel ils sont attachés et (d) l'un de R² et R⁵ doive représenter autre chose que de l'hydrogène ou un groupe alkyle en C₁ à C₆ non substitué ;
ou un sel d'addition d'acide pharmaceutiquement acceptable de ce composé.

2. Composé suivant la revendication 1, dans lequel R¹ représente H et n est égal à 3, de formule : 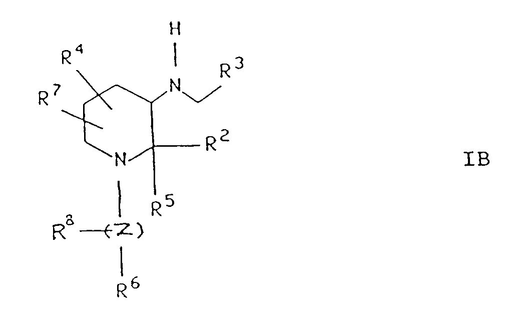 dans laquelle R² à R⁸ et Z sont tels que précédemment définis dans la revendication 1.

3. Composé suivant la revendication 2, dans lequel R⁶ représente H et m est égal à 0 (Z absent), de formule : 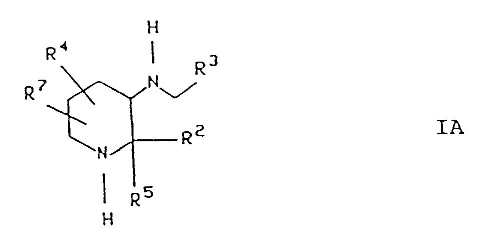 dans laquelle R² à R⁷ sont tels que précédemment définis dans la revendication 1.

4. Composé suivant la revendication 1, dans lequel R⁶ représente H, n est égal à 3 et m est égal à 0 (Z absent, de formule : 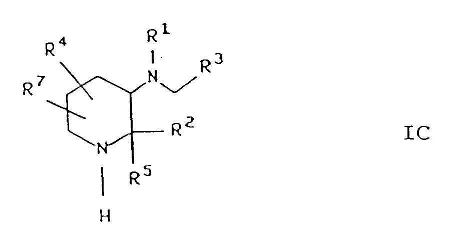 dans laquelle R¹ à R⁵ et R⁷ sont tels que précédemment définis dans la revendication 1.

5. Composé suivant la revendication 1, dans lequel R¹, R⁴, R⁵, R⁶ et R⁷ représentent chacun H, n a la valeur 3 ou 4, m est égal à 0, R² et R³ sont chacun un groupe phényle facultativement substitué avec un ou plusieurs substituants choisis entre du chlore, du fluor, un groupe alkyle en C₁ à C₆, alkoxy en C₁ à C₆ et trifluorométhyle.

6. Composé suivant la revendication 5, dans lequel R² est un groupe phényle et R³ est un groupe 2-méthoxyphényle dont la portion phényle peut facultativement être encore substituée avec du chlore, du fluor, un radical méthyle, alkoxy en C₁ à C₆ ou trifluorométhyle.

7. L'énantiomère 2S, 3S d'un composé de formule (I) dans laquelle R¹ et R⁶ représentent H et m est égal à 0, de formule : 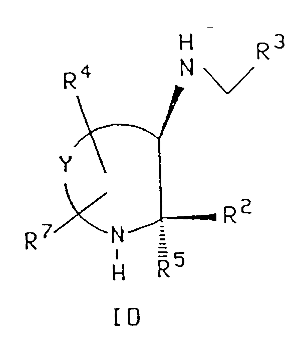 dans laquelle R² à R⁵, R⁷ et Y sont tels que précédemment définis dans la revendication 1.

8. Composé de formule IB suivant la revendication 2, qui est :
la (2S,3S)-1-(5-carbéthoxypent-1-yl)-3-(2-méthoxybenzylamino)-2-phénylpipéridine ;
la (2S,3S)-1-(6-hydroxy-hex-1-yl)-3(2-méthoxybenzylamino)-2-phénylpipéridine ;
la (2S,3S)-1-(4-hydroxy-4-phénylbut-1-yl)-3-(2-méthoxybenzylamino)-2-phénylpipéridine ;
la (2S,3S)-1-(4-oxo-4-phénylbut-1-yl)-3-(2-méthoxybenzylamino)-2-phénylpipéridine ;
la (2S,3S)-1-(5,6-dihydroxyhex-1-yl)-3-(2-méthoxybenzylamino)-2-phénylpipéridine ;
la cis-3-(5-fluoro-2-méthoxybenzylamino)-2-phénylpipéridine ;
la(2S,3S)-1-[4-(4-fluorophényl)-4-oxobut-1-yl]-3-(2-méthoxybenzylamino)-2-phénylpipéridine ;
la(2S,3S)-1-[4-(4-fluorophényl)-4-hydroxybut-1-yl]-3-(2-méthoxybenzylamino)-2-phénylpipéridine ;
la(2S,3S)-1-(4-benzamidobut-1-yl)-3-(2-méthoxybenzylamino)-2-phénylpipéridine ;
la (2S,3S)-3-(2-méthoxybenzylamino)-1-(5-N-méthylcarboxamidopent-1-yl)-2-phénylpipéridine ;
la (2S,3S)-1-(4-cyanobut-1-yl)-3-(2-méthoxybenzylamino)-2-phénylpipéridine ;
la (2S,3S)-1-[4-(2-naphtamido)but-1-yl]-3-(2-méthoxybenzylamino)-2-phénylpipéridine ;
la (2S,3S)-1-(5-benzamidopent-1-yl)-3-(2-méthoxybenzylamino)-2-phénylpipéridine ;
la (2S,3S)-1-(5-aminopent-1-yl)-3-(2-méthoxybenzylamino)-2-phénylpipéridine ;
la cis-3-(2,5-diméthoxybenzylamino)-1-[4-(4-fluorophényl)-4-oxobut-1-yl]-2-phénylpipéridine ;
la cis-3-(5-chloro-2-méthoxybenzylamino)-1-(5,6-dihydroxyhex-1-yl)-2-phénylpipéridine ; ou
la cis-1-(5,6-dihydroxyhex-1-yl)-3-(2,5-diméthoxybenzylamino)-2-phénylpipéridine.

9. Composé de formule IA suivant la revendication 3, qui est :
la cis-3-(2-chlorobenzylamino)-2-phénylpipéridine ;
la cis-3-(2-trifluorométhylbenzylamino)-2-phénylpipéridine ;
la cis-3-(2-méthoxybenzylamino)-2-(2-fluorophényl)pipéridine ;
la cis-3-(2-méthoxybenzylamino)-2-(2-chlorophényl)pipéridine ;
la cis-3-(2-méthoxybenzylamino)-2-(2-méthylphényl)-pipéridine ;
la cis-3-(2-méthoxybenzylamino)-2-(3-méthoxyphényl)pipéridine ;
la cis-3-(2-méthoxybenzylamino)-2-(3-fluorophényl)pipéridine ;
la cis-3-(2-méthoxybenzylamino)-2-(3-chlorophényl)pipéridine ;
la cis-3-(2-méthoxybenzylamino)-2-phénylpipéridine ;
la cis-3-(2-méthoxybenzylamino)-2-(3-méthylphényl)pipéridine ;
la cis-3-(2-méthoxybenzylamino)-2-(4-fluorophényl)pipéridine ;
la cis-3-(2-méthoxybenzylamino)-2-(3-thiényl)pipéridine ;
la 3-(2-méthoxybenzylamino)-4-méthyl-2-phénylpipéridine ;
la 3-(2-méthoxybenzylamino)-5-méthyl-2-phénylpipéridine ;
la 3-(2-méthoxybenzylamino)-6-méthyl-2-phénylpipéridine ;
la (2S,3S)-3-(2-méthoxybenzylamino)-2-phénylpipéridine ;
la cis-3-(2-méthoxy-5-méthylbenzylamino)-2-phénylpipéridine ;
la cis-3-(2-méthoxynapht-1-ylméthylamino)-2-phénylpipéridine ;
la (2S,3S)-3-(5-chloro-2-méthoxybenzylamino)-2-phénylpipéridine ;
la (2S,3S)-3-(2,5-diméthoxybenzylamino)-2-phénylpipéridine ;
la cis-3-(3,5-difluoro-2-méthoxybenzylamino)-2-phénylpipéridine ;
la cis-3-(4,5-difluoro-2-méthoxybenzylamino)-3-phénylpipéridine ;
la cis-2-phényl-3-[2-(prop-2-yloxy)benzylamino]pipéridine ;
le chlorhydrate de cis-3-(2,5-diméthoxybenzyl)amino-2-(3-méthoxyphényl)pipéridine ;
le dichlorhydrate de cis-3-(5-chloro-2-méthoxybenzyl)amino-2-(3-méthoxyphényl)pipéridine ;
le dichlorhydrate de cis-3-(5-chloro-2-méthoxybenzyl)amino-2-(3-chlorophényl)pipéridine ;
la 3-(2-méthoxybenzylamino)-2,4-diphénylpipéridine ; ou
la cis-3-(2-méthoxybenzylamino)-2-phénylpyrrolidinone.

10. Composé de formule I suivant la revendication 1, dans laquelle Y est un groupe (CH₂)ₙ et n est égal à 4, et dans lequel R⁶ représente H et m est égal à 0 (Z absent), ledit composé étant le cis-3-(2-méthoxybenzylamino)-2-phénylazacycloheptane.

11. Composé de formule 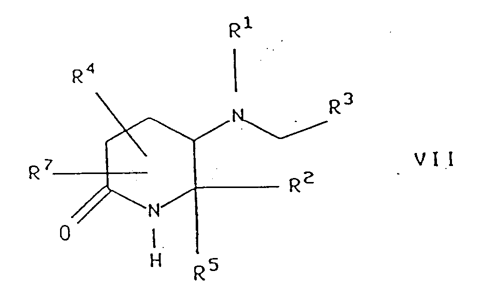 dans laquelle R¹, R², R³, R⁴, R⁵ et R⁷ sont tels que définis dans la revendication 1.

12. Composition pharmaceutique comprenant un composé de formule I suivant l'une quelconque des revendications 1 à 10 ou un sel pharmaceutiquement acceptable de ce composé, conjointement avec un diluant ou support acceptable du point de vue pharmaceutique.

13. Composé de formule (I) suivant l'une quelconque des revendications 1 à 10, destiné à être utilisé dans le traitement curatif ou préventif d'un état chez un mammifère qui est influencé ou facilité par une diminution de la neurotransmission sous la médiation de la substance P, comprenant son utilisation dans le traitement curatif ou préventif de maladies inflammatoires, de l'anxiété, de la colite, de troubes dépressifs ou dysthymiques, de psychoses, de douleurs, d'allergies, de bronchopneumopathie chronique obstructive,de troubles dus à l'hypersensibilité, de maladies angiospastiques, de troubles par formation d'un tissu fibreux et de troubles du collagène, de dystrophie sympathique réflexe, de troubles dues à la toxicomanie, de troubles somatiques en rapport avec le stress, de névopathie périphérique, de névralgie, de troubles neuropathologiques, de troubles en rapport avec la facilitation immunologique ou l'immunosuppression et de troubles rhumatismaux.

14. Utilisation d'un composé de formule (I) suivant l'une quelconque des revendications 1 à 10 pour la préparation d'un médicament destiné à être utilisé dans le traitement curatif ou préventif de maladies inflammatoires, de l'anxiété, de la colite, de troubes dépressifs ou dysthymiques, de psychoses, de douleurs, d'allergies, de bronchopneumopathie chronique obstructive,de troubles dus à l'hypersensibilité, de maladies angiospastiques, de troubles par formation d'un tissu fibreux et de troubles du collagène, de dystrophie sympathique réflexe, de troubles dus à la toxicomanie, de troubles somatiques en rapport avec le stress, de névopathie périphérique, de névralgie, de troubles neuropathologiques, de troubles en rapport avec la facilitation immunologique ou l'immunosuppression et de troubles rhumatismaux.

## Revendications (Revendications pour l'(les) Etat(s) contractant(s) suivant(s): ES, GR)

1. Procédé de production d'un composé répondant à la formule 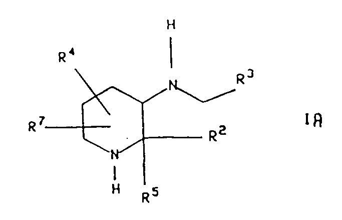 dans laquelle R² est un radical choisi entre l'hydrogène, un radical alkyle en C₁ à C₆ à chaîne droite ou ramifiée, un radical cycloalkyle en C₃ à C₇ dans lequel l'un des groupes CH₂ peut facultativement être remplacé par un groupe NH, de l'oxygène ou du soufre ; un groupe aryle choisi entre phényle et naphtyle ; un groupe hétéroaryle choisi entre indanyle, thiényle, furyle, pyridyle, thiazolyle, isothiazolyle, oxazolyle, isoxazolyle, triazolyle, tétrazolyle et quinolyle ; un groupe phényl-(alkyle en C₂ à C₆), benzhydryle ou benzyle, où chacun des groupes aryle et hétéroaryle et les portions phényle du groupe benzyle, phényl-(alkyle en C₂ à C₆) et benzhydryle peut facultativement être substitué avec un ou plusieurs substituants choisis indépendamment entre des substituants halogéno, nitro, alkyle en C₁ à C₆, alkoxy en C₁ à C₆, trifluorométhyle, amino, (alkyle en C₁ à C₆)amino, alkyle en 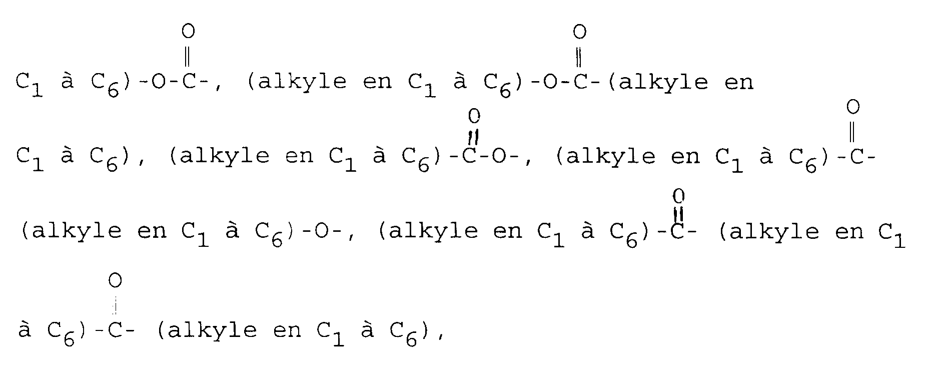 di(alkyle en C₁ à C₆)amino, 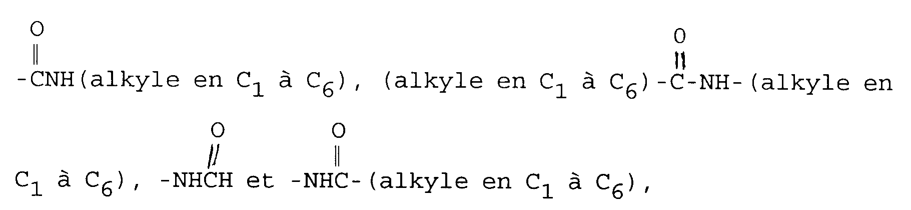 et dans laquelle l'une des portions phényle du groupe benzhydryle peut facultativement être remplacée par un groupe naphtyle, thiényle, furyle ou pyridyle ;
R⁵ est de l'hydrogène, un groupe phényle ou alkyle en C₁ à C₆ ;
ou bien R² et R⁵ forment, conjointement avec l'atome de carbone auquel ils sont attachés, un noyau carbocyclique saturé ayant 3 à 7 atomes de carbone, l'un des groupes CH₂ du noyau pouvant facultativement être remplacé par de l'oxygène, un radical NH ou du soufre ;
R³ est un groupe aryle choisi entre phényle et naphtyle ; un groupe hétéroaryle choisi entre indanyle, thiényle, furyle, pyridyle, thiazolyle, isothiazolyle, oxazolyle, isoxazolyle, triazolyle, tétrazolyle et quinolyle ; et un groupe cycloalkyle ayant 3 à 7 atomes de carbone dont l'un des groupes CH₂ peut facultativement être remplacé par NH, de l'oxygène ou du soufre ; chacun des groupes aryle et hétéroaryle peut facultativement être substitué avec un ou plusieurs substituants, et le groupe cycloalkyle en C₃ à C₇ peut facultativement être substitué avec un ou deux substituants, chacun de ces substituants étant choisi indépendamment entre des substituants halogéno, nitro, alkyle en C₁ à C₆, alkoxy en C₁ à C₆, méthyle, trifluorométhyle, amino, (alkyle en C₁ à C₆)amino, 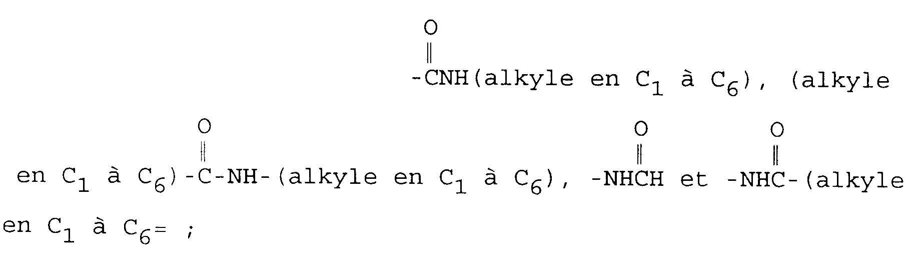 et
R⁴ et R⁷ sont tous deux choisis, indépendamment, entre l'hydrogène, des radicaux hydroxy, halogéno, amino, oxo, cyano, méthylène, hydroxyméthyle, halogénométhyle, (alkyle en C₁ à C₆)amino, di-(alkyle en C₁ à C₆)amino, alkoxy en C₁ à C₆, 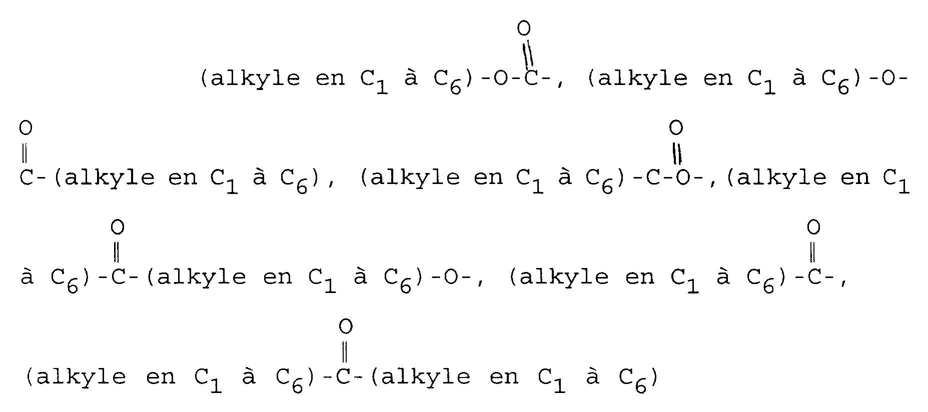 et les radicaux indiqués dans la définition de R² ;
sous réserve que (a) ni l'un ni l'autre de R⁴ et R⁷ ne puisse former, conjointement avec le carbone auquel il est attaché, un noyau avec R⁵, (b) lorsque R⁴ et R⁷ sont attachés au même atome de carbone, chacun d'eux soit indépendamment choisi entre de l'hydrogène, un radical fluoro et alkyle en C₁ à C₆, ou bien R⁴ et R⁷, conjointement avec l'atome de carbone auquel ils sont attachés, forment un noyau carbocyclique saturé en C₃ à C₆ qui donne un composé spiro avec le noyau contenant de l'azote auquel ils sont attachés, (c) R² ne représente pas un groupe benzhydryle et (d) ni l'un ni l'autre de R⁴ et R⁷ ne soit attaché en position "6 du noyau de pipéridine ;
ou d'un sel d'addition d'acide pharmaceutiquement acceptable de ce composé ;
comprenant : (a) la réduction d'un composé de formule 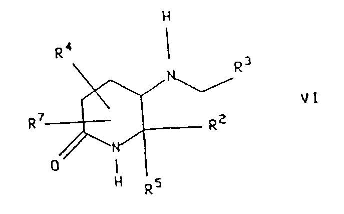 dans laquelle R², R³, R⁴, R⁵ et R⁷ sont tels que définis ci-dessus ; et (b) la réaction facultative du composé de formule IA ainsi obtenu avec un acide pharmaceutiquement acceptable.

2. Procédé suivant la revendication 1, dans lequel le composé de formule VI est obtenu par réaction d'un composé de formule 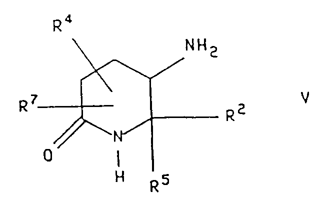 avec le cyanoborohydrure de sodium ou le triacétoxyborohydrure de sodium et un composé de formule R³CHO dans laquelle R³ est tel que défini dans la revendication 1.

3. Procédé de production d'un composé de formule 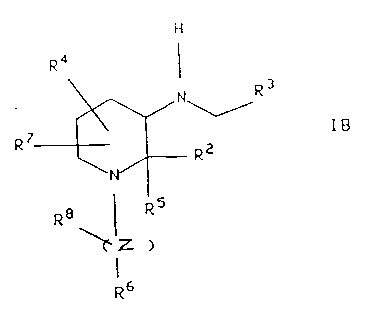 dans laquelle R², R³, R⁴, R⁵ et R⁷ sont tels que définis dans la revendication 1 ;
Z est un groupe (CH₂)ₘ dans laquelle m est un nombre entier de 0 à 6, et l'une quelconque des simples liaisons carbone-à-carbone du groupe (CH₂)ₘ peut facultativement être remplacé par une double liaison carbone-à-carbone ou une triple liaison carbone-à-carbone, et l'un quelconque des atomes de carbone du groupe (CH₂)ₘ peut facultativement être substitué par R⁸ ;
R⁶ est un groupe 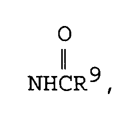 NHCN₂R⁹, SO₂R⁹ ou l'un des radicaux indiqués dans l'une quelconque des définitions de R², R⁴ et R⁷ ;
R⁸ est un groupe oximino (=NOH) ou l'un des radicaux indiqués dans l'une quelconque des définitions de R², R⁴ et R⁷ ;
R⁹ est un groupe alkyle en C₁ à C₆, de l'hydrogène, un groupe phényle ou phényl-(alkyle en C₁ à C₆) ;
sous réserve que (a) lorsque m est égal à 0, R⁸ soit absent, (b) lorsque R⁴, R⁶, R⁷ ou R⁸ est tel que défini dans R², il ne puisse pas former, conjointement avec le carbone auquel il est attaché, un noyau avec R⁵ ; (c) lorsque R⁴ et R⁷ sont attachés au même atome de carbone, chacun d'eux soit alors indépendamment choisi entre l'hydrogène et un groupe alkyle en C₁ à C₆, ou bien R⁴ et R⁷, conjointement avec l'atome de carbone auquel ils sont attachés, forment un noyau carbocylique saturé en C₃ à C₆ qui forme un composé spiro avec le noyau contenant de l'azote auquel ils sont attachés, (d) R² ne soit pas un groupe benzhydryle et (e) ni l'un ni l'autre de R⁴ et R⁷ ne soit attaché dans la position "6" du noyau de pipéridine et (d) l'un de R² et R⁵ doive représenter autre chose que de l'hydrogène ou un groupe alkyle en C₁ à C₆ non substitué ;
ou d'un sel d'addition d'acide pharmaceutiquement acceptable de ce composé ;
comprenant : (a) la réaction d'un composé de formule IA, tel que défini dans la revendication 1, avec un composé de formule R⁶-(Z)-X où R⁶ est tel que défini ci-dessus, X est un radical halogéno et Z est un groupe (CH₂)ₘ, et l'une des liaisons simples carbone-à-carbone du groupe (CH₂)ₘ peut facultativement être remplacée par une double liaison carbone-à-carbone, et l'un quelconque des atomes de carbone du groupe (CH₂)ₘ peut facultativement être substitué avec R⁸ ; et (b) la réaction facultative du composé de formule IB ainsi obtenu avec un acide pharmaceutiquement acceptable.

4. Procédé de production d'un composé de formule 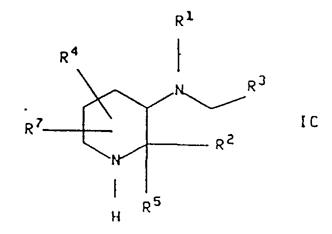 dans laquelle R², R³, R⁴, R⁵ et R⁷ sont tels que définis dans la revendication 1 ; et R¹ est de l'hydrogène ou un groupe alkyle en C₁ à C₈ facultativement substitué avec un radical hydroxy, alkoxy ou fluoro ;
sous réserve que lorsque R⁴ et R⁷ sont attachés au même atome de carbone, chacun d'eux soit alors indépendamment choisi entre l'hydrogène, un radical fluoro et un radical alkyle en C₁ à C₆, ou bien R⁴ et R⁷, conjointement avec l'atome de carbone auquel ils sont attachés, forment un noyau carbocyclique saturé en C₁ à C₆ qui forme un composé spiro avec le noyau contenant de l'azote auquel ils sont attachés, (b) R² n'est pas un groupe benzhydryle et (c) ni l'un ni l'autre de R⁴ et R⁷ n'est attaché à la position "6" du noyau pipéridine ;
ou d'un sel d'addition d'acide pharmaceutiquement acceptable de ce composé ;
comprenant (a) la réduction d'un composé de formule 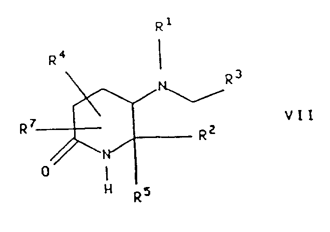 dans laquelle R², R³, R⁴, R⁵ et R⁷ sont tels que définis dans la revendication 1 et R¹ est tel que défini ci-dessus ; et (b) la réaction facultative du composé de formule IC ainsi obtenu avec un sel pharmaceutiquement acceptable.

5. Procédé suivant la revendication 4, dans lequel le composé de formule VII est obtenu par réaction d'un composé de formule 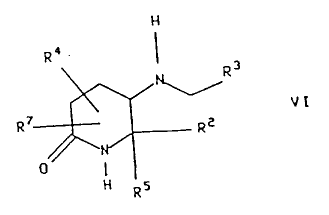 dans laquelle R², R³, R⁴, R⁵ et R⁷ sont tels que définis dans la revendication 1, avec un composé de formule R¹X dans laquelle X est un radical halogéno et R est tel que défini dans la revendication 4.

6. Procédé de production d'un composé de formule IB suivant la revendication 3 ou d'un sel d'addition d'acide pharmaceutiquement acceptable de ce composé, comprenant : (a) la réduction d'un composé de formule 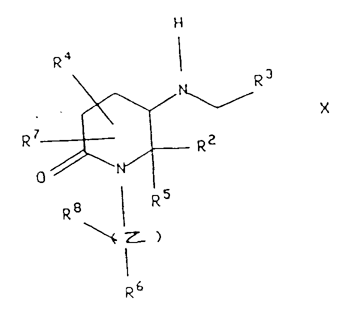 dans laquelle R², R³, R⁴, R⁵ et R⁷ sont tels que définis dans la revendication 1 et R⁶, R⁸ et Z sont tels que définis dans la revendication 3 et (b) la réaction facultative du composé de formule IB ainsi obtenu avec un sel pharmaceutiquement acceptable.

7. Procédé de production d'un composé de formule 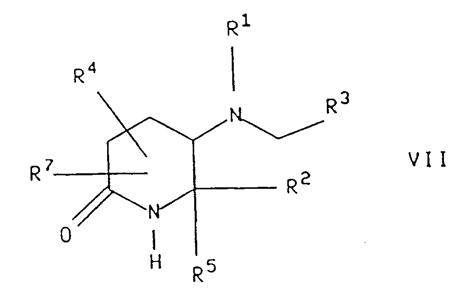 dans laquelle R², R³, R⁴, R⁵ et R⁷ sont tels que définis dans la revendication 1 et R¹ est tel que défini dans la revendication 4, comprenant la réaction d'un composé de formule 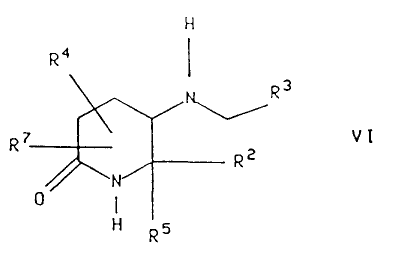 dans laquelle R², R³, R⁴, R⁵ et R⁷ sont tels que définis dans la revendication 1, avec un composé de formule R¹X dans laquelle X est un radical halogéno et R¹ est tel que défini dans la revendication 4.

8. Procédé de production d'un composé de formule 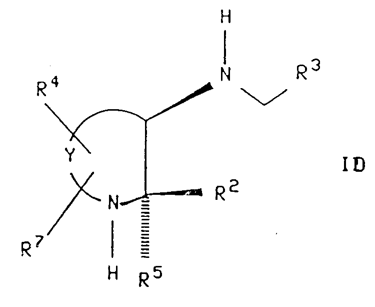 dans laquelle R², R³, R⁴, R⁵ et R⁷ sont tels que définis dans la revendication 1 ; et Y est un groupe (CH₂)ₙ dans lequel n est un nombre entier de 1 à 4 et l'une quelconque des liaisons simples carbone-à-carbone (CH₂)ₙ peut facultativement être remplacée par une double liaison carbone-à-carbone ou une triple liaison carbone-à-carbone, et l'un quelconque des atomes de carbone du groupe (CH₂)ₙ peut facultativement être substitué avec R⁴, et l'un quelconque des atomes de carbone du groupe (CH₂)ₙ peut facultativement être substitué avec R⁷,
sous réserve que (a) aucun de R⁴, R⁶ ou R⁷ ne puisse former, conjointement avec l'atome de carbone auquel il est attaché, un noyau avec R⁵, et (b) lorsque R⁴ et R⁷ sont attachés au même atome de carbone, chacun de R⁴ et R⁷ soit alors indépendamment choisi entre de l'hydrogène, un radical fluoro et alkyle en C₁ à C₆, ou bien R⁴ et R⁷, conjointement avec l'atome de carbone auquel ils sont attachés, forment un noyau carbocyclique saturé en C₃ à C₆ qui forme un composé spiro avec le noyau contenant de l'azote auquel ils sont attachés ;
ou d'un sel d'addition d'acide pharmaceutiquement acceptable de ce composé ;
comprenant : (a) la réaction d'un composé de formule 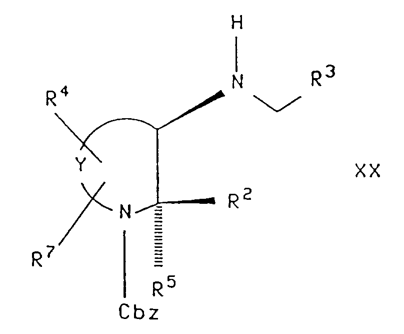 dans laquelle R², R³, R⁴, R⁵ et R⁷ sont tels que définis dans la revendication 1, Y est tel que défini ci-dessus et Cbz est un groupe carbobenzyloxy, avec le formiate d'ammonium en présence de palladium fixé sur du charbon ; et (b) la réaction facultative du composé de formule ID ainsi obtenu avec un acide pharmaceutiquement acceptable.

9. Procédé suivant la revendication 8, dans lequel le composé de formule XX est obtenu par réaction d'un composé de formule 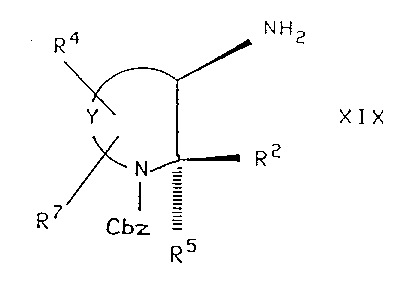 dans laquelle R², R⁴, R⁵ et R⁷ sont tels que définis dans la revendication 1 et Y est tel que défini dans la revendication 8, avec le cyanoborohydrure de sodium ou le triacétoxyborohydrure de sodium et un composé de formule R³CHO dans laquelle R³ est tel que défini dans la revendication 1.
